(19) Europäisches Patentamt
European Patent Office
Office européen des brevets

(11) **EP 2 307 191 B1**

(12) **EUROPÄISCHE PATENTSCHRIFT**

(45) Veröffentlichungstag und Bekanntmachung des Hinweises auf die Patenterteilung:
**08.08.2018   Patentblatt 2018/32**

(21) Anmeldenummer: **09772420.7**

(22) Anmeldetag: **30.06.2009**

(51) Int Cl.:
**B30B 15/02** *(2006.01)*      **B30B 11/00** *(2006.01)*

(86) Internationale Anmeldenummer:
**PCT/EP2009/058154**

(87) Internationale Veröffentlichungsnummer:
**WO 2010/000720 (07.01.2010 Gazette 2010/01)**

(54) **VERFAHREN ZUR HERSTELLUNG EINES OXIDISCHEN GEOMETRISCHEN FORMKÖRPERS**

METHOD FOR PRODUCING A GEOMETRIC OXIDIC MOLDED BODY

PROCÉDÉ DE FABRICATION D'UN CORPS MOULÉ GÉOMÉTRIQUE CONTENANT DES OXYDES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **02.07.2008   DE 102008040094**
**02.07.2008   DE 102008040093**
**02.07.2008   US 77601 P**
**02.07.2008   US 77638 P**

(43) Veröffentlichungstag der Anmeldung:
**13.04.2011   Patentblatt 2011/15**

(73) Patentinhaber: **BASF SE**
**67056 Ludwigshafen am Rhein (DE)**

(72) Erfinder:
• **EGER, Knut**
**67117 Limburgerhof (DE)**
• **FAUST, Jens Uwe**
**67346 Speyer (DE)**
• **BORCHERT, Holger**
**67591 Offstein (DE)**
• **STREIBERT, Ralf**
**67126 Hochdorf-Assenheim (DE)**
• **MÜLLER-ENGEL, Klaus Joachim**
**76297 Stutensee (DE)**
• **RAICHLE, Andreas**
**01109 Dresden (DE)**

(74) Vertreter: **BASF IP Association**
**BASF SE**
**G-FLP-C006**
**67056 Ludwigshafen (DE)**

(56) Entgegenhaltungen:
EP-A2- 0 184 790      BE-A1- 894 920
DE-A1- 10 019 831     DE-A1-102005 037 678
GB-A- 1 350 634       JP-A- 8 333 166
US-A1- 2005 263 926

**EP 2 307 191 B1**

**Beschreibung**

Vorliegende Erfindung betrifft ein Verfahren zur Herstellung eines oxidischen geometrischen Formkörpers, umfassend das mechanische Verdichten eines in den Füllraum einer Matrize eingebrachten pulverförmigen Haufwerks, aus Bestandteilen, die wenigstens eine Metallverbindung, die durch thermische Behandlung bei einer Temperatur $\geq 100$ °C in eine Metalloxid überführbar ist, oder wenigstens ein Metalloxid, oder wenigstens ein Metalloxid und wenigstens eine solche Metallverbindung mit der Maßgabe umfassen, dass wenigstens ein Bestandteil das pulverförmigen Haufwerks ein Nitratsalz, ein Ammoniumsalz oder Ammoniumnitrat ist, zu einem geometrischen Vorläuferformkörper, bei dem sich der Füllraum in einer mit einer vertikalen Bohrachse B von oben nach unten durch das Matrizenmaterial hindurchgeführten Matrizenbohrung befindet und wenigstens durch

- die Innenwand der Matrizenbohrung,
- die obere Stirnfläche eines von unten entlang der Bohrachse B in die Matrizenbohrung hub- und senkbeweglich eingeführten unteren Stempels, auf der das in den Füllraum eingebrachte pulverförmige Haufwerk aufliegt, und
- die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels, dessen untere Stirnfläche das in den Füllraum eingebrachte pulverförmige Haufwerk von oben berührt, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt und nach beendeter Verdichtung der obere Stempel vom gebildeten geometrischen Vorläuferformkörper abgehoben und der geometrische Vorläuferformkörper durch Anheben des unteren Stempels aus der Matrizenbohrung entfernt wird, sowie ein sich daran anschließendes Verfahren der thermischen Behandlung des geometrischen Vorläuferformkörpers bei einer Temperatur $\geq 100$ °C, bei dem sich wenigstens eine Teilmenge seiner Bestandteile unter Ausbildung wenigstens einer gasförmigen Verbindung zersetzt und/oder chemisch umsetzt und der oxidische geometrische Formkörper sich ausbildet.

Verfahren zur Herstellung eines oxidischen geometrischen Formkörpers unter Anwendung von zu der in der Präambel dieser Schrift ausgeführten Verfahrensweise ähnlichen Verfahrensweisen sind bekannt (vgl. z. B. EP-A 184 790, US 2005/0263926 sowie die JP-A 10/29097).

Sie werden üblicherweise angewendet, um aus pulverförmigen Gemischen (Haufwerken) von Metalloxiden und/oder von solchen Metallverbindungen (z. B. Salzen) die durch Erhitzen (thermisches Behandeln) in Metalloxide überführbar sind (wenigstens durch thermisches Behandeln in Anwesenheit von gasförmigem molekularem Sauerstoff und/oder von gasförmigen Sauerstoff freisetzenden Komponenten) geometrische (z. B. kreiszylindrische oder kreisringzylindrische (kurz auch "ringförmige") Vorläuferformkörper zu erzeugen, die nach darauffolgend durchgeführter thermischer Behandlung (in der Regel bei Temperaturen $\geq 100$ °C) als Katalysatoren (in diesem Fall spricht man von Vollkatalysatoren) oder als Trägerformkörper (kurz auch nur "Trägerkörper") für katalytische Aktivmassen (z. B. für (z. B. ringförmige) Schalenkatalysatoren (enthalten die katalytische Aktivmasse auf die äußere Oberfläche des Trägerformkörpers aufgebracht) oder für (z. B. ringförmige) Tränkkatalysatoren (die katalytisch aktive Masse wird (z. B. durch Tränken) in das Innere des Trägerformkörpers eingebracht) verwendet werden können. Der Begriff "oxidischer Formkörper" bringt dabei zum Ausdruck, dass der Formkörper wenigstens ein Metalloxid, häufig wenigstens ein Multimetalloxid (es enthält neben Sauerstoff wenigstens zwei voneinander verschieden Metalle; Halbmetalle wie Phosphor, Antimon, Arsen und Silizium sollen in dieser Schrift auch den Metallen zugerechnet werden) enthält.

Anstelle der Bezeichnung Tränkkatalysatoren wird häufig auch die Bezeichnung Trägerkatalysatoren verwendet. Bei den katalytischen Aktivmassen handelt es sich dabei häufig um Multimetalloxide. Verwendung finden solchermaßen hergestellte geometrische Katalysatorformkörper beispielsweise zur (gegebenenfalls mit inerten Formkörpern verdünnten) Beschickung des Innenraums der Reaktionsrohre eines Rohrbündelreaktors mit einem Katalysatorfestbett. Als inerte Formkörper zum Verdünnen kommen auch geometrische Trägerkörper (z. B. ringförmige) in Betracht. Ein solches Katalysatorfestbett eignet sich unter anderem zur Durchführung von heterogen katalysierten Gasphasenreaktionen (z. B. Partialoxidationen organischer Verbindungen). Das entsprechende Reaktionsgasgemisch strömt durch das Katalysatorfestbett und während der Verweilzeit an der Katalysatoroberfläche erfolgt die gewünschte Reaktion.

Nachteilig an durch mechanisches Verdichten eines pulverförmigen Haufwerk erzeugten geometrischen Formkörpern ist ganz generell, dass der Zusammenhalt des Pulverkorns im resultierenden geometrischen Formkörper im wesentlichen nicht durch intramolekulare chemische Bindungen, sondern durch bleibende interpartikuläre Bindungen bewerkstelligt wird. Zwar resultiert aus Partikelverformungen und Bruchvorgängen beim Verdichtungsvorgang in der Regel eine Zunahme der interpartikulären Gesamtkontaktfläche, dennoch ist der Betrag der durch das Verdichten erzeugten interpartikulären Bindungskräfte ein vergleichsweise beschränkter.

Gemäß eingehender Untersuchungen der Anmelderin ist der vorgenannte Sachverhalt insbesondere im Zusammenhang mit nach Verfahren gemäß der Präambel dieser Schrift hergestellten oxidischen geometrischen Formkörpern sowie deren geometrischen Vorläuferformkörpern von Relevanz. Da der im Rahmen der Verdichtung des in den Füllraum der Matrize eingebrachten pulverförmigen Haufwerks ausgeübte Pressdruck innerhalb des pulverförmigen Haufwerks im wesentlichen räumlich allseitig wirkt, ist es nicht vermeidbar, dass bei der Erzeugung des geometrischen Vorläuferformkörpers dessen Mantelfläche gegen die Innenwand der Matrizenbohrung gepresst wird. Letzteres bedingt, dass die Mantelfläche des geometrischen Vorläuferformkörpers in gewissem Umfang auf der Innenwand der Matrizenbohrung haftet. Beim Entfernen des wie beschrieben ausgebildeten geometrischen Vorläuferformkörpers aus der Matrizenbohrung durch Anheben des unteren Stempels, muss daher die diesbezügliche Haftreibung überwunden werden. Ist selbige ausgeprägt, kann es bei ihrer Überwindung im geometrischen Vorläuferformkörper zur Ausbildung von optisch kaum wahrnehmbaren Rissen kommen. Bei einer nachfolgenden thermischen Behandlung solcher geometrischer Vorläuferformkörper, im Rahmen derer im geometrischen Vorläuferformkörper zusätzlich Gase freigesetzt werden (normalerweise enthält das komprimierte Material sich bei der thermischen Behandlung unter Ausbildung von gasförmigen Substanzen zersetzende und/oder chemisch umsetzende Bestandteile (z. B. Porenbildner)), nimmt eine im geometrischen Vorläuferformkörper bereits vorhandene Rissbildung in der Regel ganz offensichtlich noch zu und entwickelt sich gegebenenfalls bis zum Bruch. Teilweise entwickelt sich aus einer vorhandenen Rissbildung (die, wie bereits gesagt, häufig kaum sichtbar ist) auch erst beim Einfüllen in die z. B. Reaktionsrohre und/oder im Verlauf der Durchführung der katalytischen Gasphasenreaktion der unerwünschte Bruch. In vielen Fällen wird die thermische Behandlung der geometrischen Vorläuferformkörper auch erst im Reaktor (z. B. im Reaktionsrohr) befindlich vorgenommen (z. B. durch Durchleiten entsprechend erhitzter Gase durch die bereits beschickten Reaktionsrohre). Im Katalysatorbett vorhandene Bruchstücke bedingen jedoch eine Verdichtung desselben und verursachen beim das selbige durchströmenden Reaktionsgasgemisch letztlich eine Erhöhung des beim Durchströmen erlittenen Druckverlustes.

Eine Gegenmaßnahme, die zur Minderung des vorstehend beschriebenen Erscheinungsbildes ergriffen werden kann, besteht z. B. darin, vorab der Einbringung von oxidischen geometrischen Formkörpern im Rahmen ihrer Herstellung entstandenen Bruch abzusieben (vgl. z. B. US-B 7,147,011 und die Deutsche Anmeldung mit dem Aktenzeichen 102007028332.8). Im Rahmen einer solchen Siebung zerbrechen in der Regel auch jene oxidischen geometrischen Formkörper, die zuvor lediglich ausgeprägte Rissbildung gezeigt haben, so dass die Bruchbildung beim Befüllen der Reaktionsrohre mit dem Siebrückstand in der Regel nur noch minimal ist.
Nachteilig an einer solchen Verfahrensweise ist jedoch, dass die Rohstoffkosten für eine großtechnische Katalysatorherstellung nicht unerheblich sind, weshalb der beim Sieben anfallende Siebdurchgang einen nicht unerheblichen materiellen Verlust bedeutet.

Eine weitere Gegenmaßnahme, die im relevanten Stand der Technik zur Minderung des beschriebenen Erscheinungsbildes bereits ergriffen wird, besteht darin, in das zum geometrischen Vorläuferformkörper zu verdichtende pulverförmige Haufwerk feinteiliges Gleitmittel (z. B. Bornitrid und/oder Graphit; vgl. DE-A 102005037678 und die Deutsche Anmeldung mit dem Aktenzeichen 102007003778.5 sowie den in diesen Schriften zitierten Stand der Technik) zuzugeben, um auf diese Weise die Haftreibung zwischen der Mantelfläche des geometrischen Vorläuferformkörpers und der Innenwand der Matrizenbohrung zu verringern. Nachteilig an einer solchen Verfahrensweise ist jedoch der zusätzliche Bedarf eines Hilfsmittels, das sich darüber hinaus gegebenenfalls nachteilig auf die resultierende katalytische Wirksamkeit auswirken kann.

Als weitere mögliche Gegenmaßnahme wird man eine Matrize verwenden, deren Matrizenbohrung eine möglichst glatte Oberfläche aufweist. Insbesondere dann, wenn wenigstens ein Bestandteil des mechanisch zu verdichtenden pulverförmigen Haufwerks ein Nitratsalz (z. B. kann wenigstens eine Metallverbindung ein Metallnitrat oder ein Metallnitrathydrat sein), ein Ammoniumsalz (z. B. kann wenigstens eine Metallverbindung ein Ammoniumsalz sein (z. B. Ammoniumheptamolybdattetrahydrat); selbstverständlich können auch dem pulverförmigen Haufwerk zugesetzte Porenbildner Ammoniumsalze sein wie z. B. $NH_4HCO_3$, $NH_4OH$, $(NH_4)_2CO_3$, $NH_4HSO_4$, $(NH_4)_2SO_4$, $NH_4CHO_2$, $NH_4CH_3CO_2$ und Ammoniumoxalat) oder Ammoniumnitrat ist, haben sich die vorgenannten Maßnahmen jedoch nicht in vollem Umfang als befriedigend erwiesen. Vielmehr haben sorgfältige Untersuchungen gezeigt, dass im Rahmen der beschriebenen zu lösenden Problemstellung dem die Matrizenbohrung berührenden Matrizenmaterial eine entscheidende Rolle zukommt, und dies vor allem bei Berücksichtigung des Sachverhalts, dass eine befriedigende Verfahrensweise je Matrize auch über längere Produktionszeiträume Produktivitäten an oxidischen geometrischen Formkörpern von bis zu 20 000 Stück je Stunde und mehr gewährleisten sollte.

Die GB 1350634 A offenbart eine Matrize zum Pressen von Ferritpulver im Magnetfeld mit einem wirksamen Teil aus Hartmetall, das zu 80 - 91,5% aus WC und zu 8 bis 15% aus Ni besteht.

Die BE 894920 empfiehlt Werkzeug aus ähnlichem Material für den Tiefziehvorgang.

Demgemäß wird mit der vorliegenden Patentanmeldung ein Verfahren zur Herstellung eines oxidischen geometrischen Formkörpers, umfassend das mechanische Verdichten eines in den Füllraum einer Matrize eingebrachten pulverförmigen Haufwerks, aus Bestandteilen, die wenigstens eine Metallverbindung, die durch thermische Behandlung bei einer Temperatur $\geq$ 100 °C in ein Metalloxid überführbar ist, oder wenigstens ein Metalloxid, oder wenigstens ein Metalloxid und wenigstens eine solche Metallverbindung mit der Maßgabe umfassen, dass wenigstens ein Bestandteil des pulverförmigen Haufwerks ein Nitratsalz, ein Ammoniumsalz oder Ammoniumnitrat ist, zu einem geometrischen Vorläuferformkörper, bei dem sich der Füllraum in einer mit einer vertikalen Bohrachse B von oben nach unten durch das Matrizenmaterial hindurchgeführten Matrizenbohrung befindet und wenigstens durch

- die Innenwand der Matrizenbohrung,
- die obere Stirnfläche eines von unten entlang der Bohrachse B in die Matrizenbohrung hub- und senkbeweglich eingeführten unteren Stempels, auf der das in den Füllraum eingebrachte pulverförmige Haufwerk aufliegt, und
- die längs der Bohrachse B in einem axialen Ausgangsabstand A oberhalb der oberen Stirnfläche des unteren Stempels befindliche untere Stirnfläche eines entlang der Bohrachse B hub- und senkbeweglich angebrachten oberen Stempels, dessen unter Stirnfläche das in den Füllraum eingebrachte pulverförmige Haufwerk von oben berührt, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt und nach beendeter Verdichtung der obere Stempel vom gebildeten geometrischen Vorläuferformkörper abgehoben und der geometrische Vorläuferformkörper durch Anheben des unteren Stempels aus der Matrizenbohrung entfernt wird,
sowie ein sich daran anschließendes Verfahren der thermischen Behandlung des geometrischen Vorläuferformkörpers bei einer Temperatur $\geq 100\,°C$, bei dem sich wenigstens eine Teilmenge seiner Bestandteile unter Ausbildung wenigstens einer gasförmigen Verbindung zersetzt und/oder chemisch umsetzt und der oxidische geometrische Formkörper sich ausbildet, zur Verfügung gestellt, das
dadurch gekennzeichnet ist, dass das die Matrizenbohrung berührende Matrizenmaterial ein Hartmetall ist, das zu $\geq 80$ Gew.-% aus dem Hartstoff Wolframcarbid und zu wenigstens 5 Gew.-% aus dem metallischen Bindemittel Nickel besteht.

Unter einem Hartmetall soll in dieser Schrift ein gesinterter Verbundwerkstoff aus $\geq 80$ Gew.-% Wolframcarbid als Hartstoff und $\geq 5$ Gew.-% Nickel als zähem metallischem Bindemittel verstanden werden. D.h., ein gesinterter Verbundwerkstoff, der wenigstens 80 Gew.-% Wolframcarbid als Hartstoff und wenigstens 5 Gew.-% Nickel als zähem metallischem Bindemittel enthält.

Als weitere Hartstoffe kann das erfindungsgemäß einzusetzende Hartmetall zusätzlich andere Metallcarbide, Metallnitride und Metallboride enthalten, wobei das Metall jeweils bevorzugt wenigstens ein Metall aus der Gruppe bestehend aus W, Ti, Zr, Hf, V, Nb, Ta, Mo und Cr ist. Als weitere metallische Bindemittel kann das Hartmetall neben Nickel noch Fe, Co und/oder Cr enthalten. Erfindungsgemäß vorteilhaft ist der auf das metallische Bindemittel Ni im Hartmetall entfallende Gewichtsanteil dabei größer, als der auf jedes andere im Hartmetall enthaltene metallische Bindemittel entfallende Gewichtsanteil.

Als weitere Hartstoffe kommen für das erfindungsgemäß einzusetzende Hartmetall vor allem Hartstoffe aus der Gruppe bestehend aus Titancarbid (TiC), Tantalcarbid (TaC), Niobcarbid (NbC), Vanadiumcarbid (VC), Chromcarbid ($Cr_3C_2$) und Mischmetallcarbide (z. B. Tantal-Niob-Carbid (TaNbC)), die wenigstens zwei der in den vorgenannten Metallcarbiden enthaltenen Metalle enthalten, in Betracht, die kornwachstumshemmend wirken.

Als zähe metallische Bindemittel kommen erfindungsgemäß bevorzugt ausschließlich Nickel oder Nickel und Chrom in den erfindungsgemäß einzusetzenden Hartstoffen zur Anwendung.

In der Regel beträgt der auf die metallischen Bindemittel entfallende Gewichtsanteil an den erfindungsgemäß einzusetzenden Hartmetallen bis zu 20 Gew.-%, oft bis zu 15 Gew.-% und vielfach bis zu 10 Gew.-%.

Die Herstellung erfindungsgemäß geeigneter gesinterter Hartmetalle erfolgt vorzugsweise so, dass man die gewünschte Menge an hochschmelzenden Hartstoffen (bezogen auf das Gewicht des resultierenden Hartmetalls $\geq 80$ Gew.-% WC) und die gewünschte Menge an tiefer schmelzendem Metallpulver (zähem metallischem Bindemittel; bezogen auf das Gewicht des resultierenden Hartmetalls $\geq 5$ Gew.-% Nickel) mischt (vorzugsweise homogen) und (z. B. im elektrischen Ofen) auf Temperaturen unterhalb des Schmelzpunktes der hochschmelzenden Hartstoffe erhitzt, wobei die Temperatur und Zeitdauer der Erhitzung so gewählt werden, dass der Hartstoffanteil zu einem Skelett (der Hartphase) zusammensintert, in welches der Metallanteil als Bindephase (Bindemittel) eingelagert ist. Die Korngröße (insbesondere des Hartstoffanteils) im Hartmetallpulver (Ausgangspulver) kann dabei z. B. 0,2 $\mu m$ bis 15 $\mu m$, vorteilhaft 0,5 bis 3 $\mu m$, besonders vorteilhaft 1 bis 1,5 $\mu m$ betragen.
Die Herstellung von gesinterten Hartmetallen ist z. B. in den Schriften AT-PS 358833, EP-A 1364732, AT-PS 362943 sowie in der Studienarbeit "Ermüdungsverhalten des Hartmetalls G55 Co bei Raumtemperatur", von Frank Hebner aus Erlangen, 7. September 2003, an der Friedrich-Alexander-Universität Erlangen-Nürnberg, Institut für Werkstoffwissenschaften, Lehrstuhl I - allgemeine Werkstoffeigenschaften, Prof. Dr. H. Mughrabi und dem in diesen Schriften zitierten Stand der Technik beschrieben. In der Regel weisen erfindungsgemäß einzusetzende Hartmetalle eine Rockwellhärte von nicht unter 80, eine Vickers Härte $\geq 1500$ und eine Biegefestigkeit $\geq 2000$ N|mm$^2$ auf.

Erfindungsgemäß bevorzugt besteht das erfindungsgemäß einzusetzende Hartmetall zu $\geq 85$ Gew.-%, beson-

ders bevorzugt zu ≥ 90 Gew.-% aus Wolframcarbid (WC) und zu ≥ 5 Gew.-% aus Nickel, besonders bevorzugt zu ≥ 7 Gew.-% aus Nickel.

Noch besser ist für das erfindungsgemäße Verfahren der Einsatz eines Hartmetalls, das, bezogen auf sein Gewicht, zu 90 bis 95 Gew.-% aus Wolframcarbid (WC), zu ≥ 0 bis 1 Gew.-% aus wenigstens einem Metallcarbid aus der Gruppe bestehend aus Titancarbid (TiC), Tantalcarbid (TaC), Niobcarbid (NbC), Vanadiumcarbid (VC), Chromcarbid ($Cr_3C_2$) und Mischmetallcarbiden (z. B. Tantal-Niob-Carbid (TaNbC)), die wenigstens zwei der in den vorgenannten Metallcarbiden enthaltenen Metalle enthalten, sowie zu bis zu 10 Gew.-% aus Ni, Fe, Co und/oder Cr (vorzugsweise Ni und Cr), mit der Maßgabe, dass der Gewichtsanteil des Ni am Hartmetall ≥ 5 Gew.-% beträgt, besteht.

Ganz besonders bevorzugt wird beim erfindungsgemäßen Verfahren als Hartmetall ein solches eingesetzt, das aus

| | |
|---|---|
| 90 bis 95 Gew.-% | WC, |
| ≥ 0 bis 1 Gew.-% | TiC und/oder TaNbC; und |
| 5 bis 10 Gew.-% | Ni oder Ni und Cr |

mit der Maßgabe besteht, dass der Gewichtsanteil des Ni am Hartmetall ≥ 5 Gew.-% beträgt.

Unter den vorgenannten Hartmetallen sind ferner jene günstig, die aus

| | | |
|---|---|---|
| | 89,2 bis 94,8 Gew.-% | WC, |
| | 0,2 bis 0,8 Gew.-% | TiC und TaNbC, |
| sowie | 5 bis 10 Gew.-% | Ni |

bestehen.

Zur letztgenannten Gruppe von Hartmetallen gehört unter anderem das erfindungsgemäß besonders günstig verwendbare Hartmetall G 10-Ni der Hartmetall® Gesellschaft in D-70572 Stuttgart.

Alle vorstehenden Ausführungen zum Hartmetall sind insbesondere für Korngrößen (Korndurchmesser) im Hartmetallpulver (insbesondere seines Hartstoffanteils) von 0,5 μm bis 2 μm, bevorzugt von 1 bis 1,5 μm zutreffend.

Grundsätzlich kann die Matrize beim erfindungsgemäßen Verfahren ausschließlich aus einem der vorstehend beschriebenen Hartmetalle bestehen.

Nicht zuletzt aus Wirtschaftlichkeitsgründen ist die Matrize jedoch vorteilhaft aus einem Werkstoffverbund gefertigt. Dieser (das Matrizenmaterial) besteht nur auf seiner die

Matrizenbohrung berührenden Seite aus dem erfindungsgemäß geforderten Hartmetall und auf seiner von der Matrizenbohrung abgewandten Seite vorteilhaft aus einem Werkzeugstahl. Vorzugsweise hat der Werkzeugstahl die folgende Elementzusammensetzung WS:

| | |
|---|---|
| 1,50 bis 1,80 Gew.-% | C, |
| 0,10 bis 0,40 Gew.-% | Si, |
| 0,10 bis 0,50 Gew.-% | Mn, |
| ≥ 0 bis 0,05 Gew.-% | P, |
| ≥ 0 bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| ≥ 0 bis 0,60 Gew.-% | W, und |
| ≥ 0 bis 0,10 Gew.-% | eines oder mehrere seltene Erdmetalle, und im übrigen Fe und herstellungsbedingte Verunreinigungen, wobei die Prozentangaben jeweils auf das Gesamtgewicht bezogen sind. |

Besonders bevorzugte Werkzeugstähle WS sind die DIN-Werkstoffe 1.2601 und 1.2379. D. h., die Matrize besteht erfindungsgemäß zweckmäßig aus einem die Matrizenbohrung aufweisenden Kern aus Hartmetall und einer den Kern der Matrize umfassenden Matrizeneinfassung aus einem Werkzeugstahl (vorzugsweise aus einem solchen der Elementzusammensetzung WS). Eine Wanddicke des die Matrizenbohrung berührenden Hartmetalls von wenigen Millimetern (z. B. 1 bis 10 mm, vielfach 2 bis 8 mm, oder 2 bis 6 mm, oder 2 bis 4 mm) ist dabei in der Regel ausreichend. Die Wanddicke der Matrizeneinfassung aus Werkzeugstahl wird normalerweise bei wenigen Zentimetern (z. B. 0,5 bis 3 cm, oder 1 bis 2 cm) liegen.

Der Mittenrauhwert Ra (nach DIN 4768) der Innenwand der Matrizenbohrung sollte beim erfindungsgemäßen Verfahren vorzugsweise nicht mehr als 0,2 $\mu$m und besonders bevorzugt nicht mehr als 0,1 $\mu$m und noch besser nicht mehr als 0,05 $\mu$m betragen. Der Mittenrauhwert ist dabei der arithmetische Mittelwert der absoluten Beträge der Abstände des Rauheitsprofils von der mittleren Linie innerhalb der Messstrecke. Entsprechende geringe Rauheiten sind durch Polieren zu erreichen. Die Herstellung einer erfindungsgemäß zu verwendenden Matrize mit einem Kern aus Hartmetall (z. B. G10-Ni) und einer Matrizeneinfassung aus Werkzeugstahl (z. B. aus dem DIN-Werkstoff 1.2379) ist in einfacher Weise z. B. durch sogenanntes Einschrumpfen möglich. Es wird dabei zunächst die Matrizeneinfassung aus dem Werkzeugstahl hergestellt. Diese wird dann erhitzt, wobei sie sich ausdehnt. In die ausgedehnte Matrizeneinfassung kann der Hartmetallkern eingefügt werden. Beim Abkühlen zieht sich die Matrizeneinfassung wieder zusammen und bildet mit dem Hartmetallkern einen quasi nahtlosen Verbund.

Wie bereits ausgeführt, umfasst das erfindungsgemäße Verfahren die Herstellung von geometrischen Vorläuferformkörpern aus pulverförmigem Haufwerk, das bereits (bei Normalbedingungen (25 °C, 1 atm) in der Regel festes) wenigstens ein Metalloxid enthält, und/oder wenigstens eine solche Metallverbindung (z. B. ein Metallsalz), die durch thermische Behandlung bei einer Temperatur ≥ 100 °C in ein (bei Normalbedingungen in der Regel festes) Metalloxid überführbar ist (wenigstens durch thermisches Behandeln in Anwesenheit von gasförmigem molekularem Sauerstoff und/oder von gasförmigen Sauerstoff freisetzenden Komponenten; prinzipiell kann die Sauerstoffquelle z. B. in Form eines Peroxids auch Bestandteil des pulverförmigen Haufwerks und damit des erfindungsgemäß resultierenden geometrischen Vorläuferformkörpers sein). Das feste Metalloxid kann dabei ein solches sein, das neben Sauerstoff nur ein oder aber auch mehr als ein (z. B. zwei oder drei) Metallelemente enthält. Ebenso kommen als Metallverbindungen grundsätzlich solche in Betracht, die nur ein, oder aber auch mehr als ein (z. B. zwei oder drei) Metallelemente enthalten.

Ferner muss wenigstens ein Bestandteil des erfindungsgemäß zu verdichtenden pulverförmigen Haufwerks ein Nitratsalz, ein Ammoniumsalz oder Ammoniumnitrat sein. Grundsätzlich kann ein solches Nitratsalz beispielsweise eine der vorgenannten Metallverbindungen sein. D. h., das pulverförmige Haufwerk kann als solches Nitratsalz (der Begriff soll in dieser Schrift auch deren Hydrate subsumieren) z. B. wenigstens ein Metallnitrat aus der Gruppe bestehend aus Kobaltnitrat, Eisennitrat, Wismutnitrat, Nickelnitrat, Cäsiumnitrat, Kupfernitrat, Calciumnitrat, Magnesiumnitrat und den Hydraten dieser Nitrate, enthalten. Selbstverständlich subsumiert der Begriff Nitratsalz aber auch die Salpetersäure, als das protische Salz des Nitratanions. Grundsätzlich kann auch das Ammoniumsalz eine der vorgenannten Metallverbindungen sein (beispielhaft genannt seien das Ammoniummetavanadat sowie das Ammoniumheptamolybdattetrahydrat).

Selbstverständlich kann das erfindungsgemäß zu verdichtende pulverförmige Haufwerk als Ammoniumsalz auch solche Ammoniumsalze zugesetzt enthalten, die kein Metallatom/-ion (keinen metallischen Konstituenten) enthalten, und im weiteren Verlauf der erfindungsgemäßen Herstellung eines oxidischen geometrischen Formkörpers im wesentlichen als Porenbildner fungieren. Als solche porenbildende Ammoniumsalze seien beispielhaft genannt $NH_4HCO_3$, $NH_4OH$, $(NH_4)_2CO_3$, $NH_4HSO_4$, $(NH_4)_2SO_4$, $NH_4CHO_2$, $NH_4CH_3CO_2$ und Ammoniumoxalat sowie die Hydrate der vorgenannten Verbindungen. Selbstverständlich kann das erfindungsgemäß zu verdichtende pulverförmige Haufwerk auch Ammoniumnitrat (oder dessen Hydrat) zugesetzt enthalten, das sowohl ein Nitratsalz als auch ein Ammoniumsalz ist.

Als weitere Voraussetzung ist es für das erfindungsgemäße Verfahren wesentlich, dass das erfindungsgemäß zu verdichtende pulverförmige Haufwerk, und mit diesem die resultierenden geometrischen Vorläuferformkörper, Substanzen (Bestandteile) enthalten, die sich unter den bei der thermischen Behandlung der geometrischen Vorläuferformkörper angewandten Bedingungen (Behandlungstemperatur ≥ 100 °C) wenigstens teilweise unter Ausbildung von (unter den Bedingungen der thermischen Behandlung der geometrischen Vorläuferformkörper) gasförmigen Verbindungen zersetzen und/oder chemisch umsetzen (z. B. unter Ausbildung von Ammoniak, Wasserdampf, $CO_2$, CO und/oder Stickstoffoxiden). In der Regel beträgt der mit der thermischen Behandlung der geometrischen Vorläuferformkörper einhergehende und auf deren Ausgangsgewicht bezogene Gewichtsverlust (infolge des vorgenannten Ausgasens) 0,5 bis 40 Gew.-%, häufig 0,8 bis 35 Gew.-% oder 2 bis 30 Gew.-%.

Eine Ausbildung (Freisetzung) von gasförmigen Verbindungen im Rahmen einer erfindungsgemäßen thermischen Behandlung von erfindungsgemäß erzeugten geometrischen Vorläuferformkörpern ist normalerweise z. B. dann gegeben, wenn die Bestandteile des erfindungsgemäß zu verdichtenden pulverförmigen Haufwerks wenigstens teilweise organischer Natur sind bzw. Hydroxidionen, Carbonationen, Hydrogencarbonationen, Ammoniumionen, Hydrogenphosphationen, Hydrogensulfationen und/oder Nitrationen enthalten, die sich bei der erfindungsgemäßen thermischen Behandlung in der Regel wenigstens teilweise zersetzen. Dabei können die Hydroxidionen, Carbonationen, Hydrogencarbonationen, Ammoniumionen, Hydrogenphosphationen und/oder Nitrationen grundsätzlich bereits Bestandteil der nicht Oxid seienden Metallverbindungen in dem erfindungsgemäß zu verdichtenden pulverförmigen Haufwerk sein. Sie können dem erfindungsgemäß zu verdichtenden pulverförmigen Haufwerk aber auch zusätzlich (oder nur) als Bestandteil von bei der nachfolgenden thermischen Behandlung der geometrischen Vorläuferformkörper Poren bildenden und keine Metallverbindungen seienden Substanzen zugegeben werden.

Selbstverständlich können beim erfindungsgemäßen Verfahren dem erfindungsgemäß zu verdichtenden pulverförmigen Haufwerk in erfindungsgemäß vorteilhafter Weise als Hilfsmittel auch Gleitmittel zugesetzt werden, die die Haftreibung zwischen dem erfindungsgemäß hergestellten geometrischen Vorläuferformkörper und der Innenwand der Matrizenbohrung zusätzlich zur erfindungsgemäßen Maßnahme der Nutzung eines spezifischen Hartmetalls als die Matrizenbohrung berührendes Matrizenmaterial weiter herabsetzen. Als solche Gleit- bzw. Schmiermittel können z. B. Graphit, Ruß, Polyethylenglykol, Stearinsäure, Salz der Stearinsäure, Stärke, Polyacrylsäure, Mineralöl, Pflanzöl, Wasser, Bornitrid, Bortrifluorid, Glycerin, feinteiliges Teflonpulver und/oder Celluloseether verwendet werden. Es sei an dieser Stelle jedoch nochmals festgehalten, dass das erfindungsgemäße Verfahren nicht zuletzt dadurch besticht, dass es die Minimierung des Bedarfs derartiger Hilfsmittel ermöglicht. Vorgenannte Gleitmittel können sich im Rahmen der erfindungsgemäßen thermischen Behandlung der geometrischen Vorläuferformkörper gegebenenfalls auch unter Bildung gasförmiger Substanzen zersetzen bzw. chemisch umsetzen.

Als weitere Formgebungshilfsmittel kann das erfindungsgemäß zu verdichtende pulverförmige Haufwerk sogenannte Verstärkungsmittel zugesetzt enthalten, die den Zusammenhalt im resultierenden Komprimat fördern. Solche Verstärkungsmittel können beispielsweise Mikrofasern aus Glas, Asbest, Siliciumcarbid und/oder Kaliumtitanat sein.

Bezogen auf die Gesamtmenge des erfindungsgemäß zu verdichtenden pulverförmigen Haufwerks wird die Gesamtmenge an Formgebungshilfsmitteln in der Regel nicht mehr als 30 Gew.-%, meist nicht mehr als 20 Gew.-% und vielfach nicht mehr als 10 Gew.-% betragen.

Im Rahmen der erfindungsgemäßen thermischen Behandlung der geometrischen Vorläuferformkörper kommt es zur Ausbildung der erfindungsgemäß erwünschten oxidischen geometrischen Formkörper. Erfindungsgemäß erfolgt diese thermische Behandlung bei Temperaturen ≥ 100 °C. Häufig erfolgt die erfindungsgemäße thermische Behandlung bei Temperaturen ≥ 150 °C, oder ≥ 200 °C (z. B. 300 bis 800 °C, oder 350 bis 750 °C, oder 300 bis 700 °C). Insbesondere im Fall einer Herstellung von oxidischen geometrischen Trägerformkörpern kann die thermische Behandlung eine solche bei Temperaturen ≥ 600 °C, oder ≥ 1000 °C einbeziehen. 1500 °C werden in den meisten Fällen nicht überschritten.

Grundsätzlich kann die erfindungsgemäße thermische Behandlung von erfindungsgemäß hergestellten geometrischen Vorläuferformkörpern (z. B. kreiszylindrischen, ringförmigen, kreiszylinderähnlichen oder ringähnlichen) sowohl unter Vakuum, unter inerter Atmosphäre (z. B. $N_2$, Edelgase, Wasserdampf, $CO_2$ etc.) unter reduzierender Atmosphäre (z.B. $H_2$ oder $NH_3$) oder unter oxidierender Atmosphäre erfolgen. In der Regel werden oxidierende Atmosphären molekularen Sauerstoff enthalten. Typische oxidierende Atmosphären sind Gemische aus Inertgas ($N_2$, Edelgase, Wasserdampf, $CO_2$ etc.) und molekularem Sauerstoff. Üblicherweise wird der Gehalt an molekularem Sauerstoff dabei wenigstens 0,1 Vol.-%, häufig wenigstens 0,2 Vol.-%, vielfach wenigstens 0,5 Vol.-%, oft wenigstens 1 Vol.-%, oder wenigstens 10 Vol.-%, oder wenigstens 20 Vol.-% betragen. Selbstverständlich kann der Gehalt an molekularem Sauerstoff in solchen Gemischen aber auch 30 Vol.-%, oder 40 Vol.-%, oder 50 Vol.-%, oder mehr betragen. Selbstverständlich kommt als oxidierende Atmosphäre für eine solche thermische Behandlung aber auch reiner molekularer Sauerstoff in Betracht. Häufig wird eine oxidierende thermische Behandlung unter Luft erfolgen. Generell kann die thermische Behandlung unter stehender oder unter fließender Gasatmosphäre (z.B. im Luftstrom) erfolgen. Vorteilhaft wird in der Regel eine fließende Gasatmosphäre angewendet.

Der Begriff der Atmosphäre (bzw. der Gasatmosphäre) in welcher die thermische Behandlung erfolgt, ist dabei in dieser Schrift so zu verstehen, dass er sich aus den erfindungsgemäß hergestellten geometrischen Vorläuferformkörpern im Rahmen der thermischen Behandlung aufgrund von Zersetzungsvorgängen und/oder chemischen Reaktionsvorgängen sich entwickelnde Gase nicht umfasst. Selbstverständlich kann die Gasatmosphäre, in welcher die thermische Behandlung erfolgt, aber auch ausschließlich oder teilweise aus diesen Gasen bestehen. Auch können sowohl die Behandlungstemperatur als auch die Behandlungsatmosphäre über die Dauer der thermischen Behandlung zeitlich konstant oder zeitlich variabel gestaltet sein. Für den Fall, dass als Ergebnis der anschließenden thermischen Behandlung von erfindungsgemäß erhältlichen geometrischen Vorläuferformkörpern oxidische geometrische Vollkatalysatoren angestrebt werden, deren Aktivmasse wenigstens ein Multimetalloxid ist, erfolgt die thermische Behandlung häufig bei Temperaturen von 150 bis 650°C, vielfach 200 bis 600°C, oft 250 bis 550°C und vielfach 300 bis 500°C. Der Begriff "Multimetalloxid" meint in dieser Schrift kein einfaches Gemisch verschiedener Metalloxide, sondern eine komplexe Polyoxyverbindung die neben Sauerstoff wenigstens zwei voneinander verschiedene Metalle (metallische Konstituenten) enthält.

Die erfindungsgemäße thermische Behandlung der geometrischen Vorläuferformkörper kann beim erfindungsgemäßen Verfahren grundsätzlich sowohl in einer dafür ausgestalteten speziellen Vorrichtung (z. B. in einem Bandcalcinierer) als auch erst in dem Reaktor, in welchem sie zur Anwendung kommen sollen (z. B. in den Reaktionsrohren eines Rohrbündelreaktors) vorgenommen werden. Im letzteren Fall wird man zweckmäßiger Weise heiße Gase durch die Reaktionsrohre leiten.

Üblicherweise wird das pulverförmige Haufwerk beim erfindungsgemäßen Verfahren anfasstrocken eingesetzt. Es kann jedoch bis zu 10 % seines Gesamtgewichts Substanzen zugesetzt enthalten, die bei Normalbedingungen (25°C, 1 atm) flüssig sind. Das erfindungsgemäße Verfahren ist aber auch dann anwendbar, wenn das pulverförmig Haufwerk überhaupt keine solchen flüssigen Substanzen mehr enthält. Selbstverständlich kann das pulverförmige Haufwerk auch feste Solvate (z.B. Hydrate) enthalten, die solche flüssigen Substanzen in chemisch und/oder physikalisch gebundener Form aufweisen. Derartige Solvate zersetzen sich bei der erfindungsgemäßen thermischen Behandlung der geometrischen Vorläuferformkörper in der Regel ebenfalls unter gasförmiger Freisetzung der Solvatphase. Erfindungsgemäß vorteilhaft liegt die Restfeuchte des erfindungsgemäß zu verdichtenden Haufwerks bei < 10 Gew.-%, in Abwesenheit von Kristallwasser (bzw. Kristallsolvatphase) in der Regel bei < 5 Gew.-%.

Die Partikeldurchmesser des erfindungsgemäß zu verdichtenden feinteiligen pulverförmigen Haufwerks werden (zugesetzte Formgebungs- und Porosierungsmittel ausgenommen) für wenigstens 90 % des Gewichts des pulverförmigen Haufwerks (zugesetzte Formgebungs- und Porosierungsmittel nicht miteinbezogen) anwendungstechnisch zweckmäßig im Bereich von 10 bis 2000 $\mu$m, vielfach im Bereich von 20 bis 1800 $\mu$m, oder von 30 bis 1700 $\mu$m, oder von 40 bis 1600 $\mu$m, oder von 50 bis 1500 $\mu$m liegen. Besonders häufig werden vorgenannte Partikeldurchmesser im Bereich von 100 bis 1500 $\mu$m oder 150 bis 1500 $\mu$m liegen.

Im Übrigen gelten für diese Schrift die nachfolgenden Definitionen.

Der Begriff "untere (obere) Stirnfläche" eines Stempels meint in dieser Schrift die Oberfläche der Stirn des Stempels an seinem unteren (oberen) Ende. Ist der Stempel z. B. ein Kreisringzylinder, ist sowohl seine untere Stirnfläche als auch seine obere Stirnfläche ein Kreisring. Ist der Stempel dagegen ein Kreiszylinder, ist sowohl seine unter Stirnfläche als auch seine obere Stirnfläche ein Kreis.

Der Begriff "Kreiszylinder" meint in dieser Schrift stets einen "geraden Kreiszylinder". Verbindet man die Endpunkte paralleler Radien zweier in parallelen Ebenen liegender gleich großer Kreise miteinander durch Strecken, so entsteht ein Kreiszylinder. Die Verbindungsstrecken heißen Mantellinien des Zylinders. Stehen sie senkrecht auf den parallelen Kreisebenen, so heißt der Zylinder "gerade" oder Rotationszylinder. Die Verbindungsstrecke der Kreismittelpunkte ist die Symmetrieachse des geraden Kreiszylinders (häufig auch einfach "Kreiszylinderachse" oder "Achse des Kreiszylinders" genannt). Die Gesamtheit aller Mantellinien bildet die Mantelfläche des Zylinders.

In analoger Weise meint der Begriff "Kegelstumpf" in dieser Schrift einen anderen speziellen Rotationskörper. Dieser (der Kegelstumpf) entsteht dadurch, dass man von einem geraden Kreiskegel parallel zur Grundfläche einen kleineren geraden Kegel abschneidet. Die größere der beiden durch das Abschneiden erzeugten parallelen Kreisflächen wird in dieser Schrift auch als Grundfläche und die kleinere als Deckfläche bezeichnet. Der Abstand von Grund- und Deckfläche wird als Höhe des Kegelstumpfes bezeichnet. Die Dritte der einen Kegelstumpf begrenzenden Flächen wird als Mantelfläche desselben bezeichnet. Die Verbindungslinie der Mittelpunkte von Grund- und Deckfläche bildet die Symmetrieachse des Kegelstumpfs (häufig auch einfach "Achse des Kegelstumpfs" genannt). Unter einem Kegel versteht man einen Körper, der durch einen Kreis (Grundkreis oder Basiskreis) und einen Punkt außerhalb der Ebene des Kreises (Spitze des Kegels) festgelegt ist und dadurch entsteht, dass man die Punkte auf der Umrisslinie des Kreises mit dem einen Punkt außerhalb der Ebene des Kreises verbindet. Steht die Verbindungslinie der Spitze des Kegels mit dem Mittelpunkt des Basiskreises des Kegels senkrecht zur Basisebene, so liegt ein gerader Kreiskegel oder Drehkegel vor.

Der Begriff "Kreisring" meint in dieser Schrift die Fläche zwischen zwei konzentrischen Kreisen, d.h., zwischen zwei Kreisen mit gemeinsamem Mittelpunkt.

Verbindet man von zwei in parallelen Ebenen liegenden kongruenten (deckungsgleichen) Kreisringen (dem Grundkreisring und dem Deckkreisring) die jeweiligen Endpunkte paralleler Radien auf den beiden äußeren Kreisen sowie die jeweiligen Endpunkte paralleler Radien auf den beiden inneren Kreisen durch Strecken, so entsteht ein Kreisringzylinder. Die Verbindungsstrecken der Endpunkte auf den beiden inneren Kreisen heißen innere Mantellinien des Kreisringzylinders (ihre Gesamtheit bildet den inneren Mantel des Kreisringzylinders) und die Verbindungsstrecken der Endpunkte auf den beiden äußeren Kreisen heißen äußere Mantellinien des Kreisringzylinders (ihre Gesamtheit bildet den äußeren Mantel des Kreisringzylinders). Stehen die Mantellinien senkrecht auf den beiden Kreisringen, so heißt der Kreisringzylinder gerade oder nicht schief. Der Begriff "Kreisringzylinder" soll in dieser Schrift stets für den geraden Kreisringzylinder stehen. Die Verbindungsstrecke der Kreisringmittelpunkte heißt Achse des Kreisringzylinders.

Der Begriff der "Bohrung" soll in dieser Schrift nicht so verstanden werden, dass die entsprechende Öffnung mit Hilfe von Bohrern durch Bohren erzeugt worden sein muss. Vielmehr kann die Öffnung auch auf andere Weise (z. B. mit Hilfe eines Lasers, einer Fräse oder eines Schneidbrenners) erzeugt worden sein. Die Symmetrie der Öffnung soll jedoch so sein, wie wenn sie durch Bohren mit Hilfe eines Bohrers (oder mehrerer Bohrer) erzeugt worden wäre (selbstverständlich kann sie auch so erzeugt worden sein).

Als Mantelfläche wird die Oberfläche eines geometrischen Formkörpers ohne Boden (Grundfläche) und Deckel (Deckfläche) bezeichnet.

Das Merkmal "dass die Mantelfläche eines Kreiszylinders auf der Innenwand einer Bohrung gleitet" (oder umgekehrt) ist in dieser Schrift so zu verstehen, dass, soweit nichts anderes gesagt wird, die der Mantelfläche entsprechende Außenwand des Kreiszylinders über den Bereich der Gleitstrecke (d. h., über den Gleitbereich) auf der Innenwand der Bohrung gleichmäßig, aber gasdurchlässig sowie axial beweglich aufliegt.

Das erfindungsgemäße Verdichtungsverfahren ist insbesondere im Fall der Herstellung solcher geometrischer Vorläuferformkörper von Interesse, bei denen der Endabstand E 2 bis 10 mm, oder 2 bis 8 mm, oder 3 bis 8 mm, oder 3 bis 7 mm beträgt (eine eventuelle Krümmung der Stirnfläche soll bei der Bestimmung der Abstände A und E keine Berücksichtigung finden; d. h., gemeint ist immer der Abstand der oberen/unteren Umrisslinie der Mantelfläche der Stempel).

In einer einfachen Ausgestaltung der erfindungsgemäßen Verfahrensweise entspricht die geometrische Form der Innenwand der Matrizenbohrung derjenigen der Mantelfläche eines Kreiszylinders KZ* und sowohl die geometrische Form des unteren Stempels als auch die geometrische Form des oberen Stempels entsprechen derjenigen eines Kreiszylinders I*.

Sowohl die obere Stirnfläche des unteren Stempels als auch die untere Stirnfläche des oberen Stempels sind im vorbeschriebenen Fall vorzugsweise eine plane Kreisfläche, auf der die Bohrachse B der Matrizenbohrung senkrecht steht. Die Bohrachse B und die Symmetrieachsen der Kreiszylinder I* liegen auf einer geraden Linie. Der Außendurchmesser der beiden Stempel ist im vorstehenden Fall anwendungstechnisch zweckmäßig marginal kleiner als der Innendurchmesser der Matrizenbohrung, so dass beide Stempel mit ihrer Außenwand auf der Innenwand der Matrizenbohrung axial gleitend in die Matrizenbohrung eingeführt werden können. Darüber hinaus bilden die so zwischen der unteren (bzw. oberen) Umrisslinie des oberen Stempels (bzw. unteren Stempels) und der Innenwand der Matrizenbohrung im Zustand des Ausgangsabstands A sowie im Zustand des Endabstands E bestehenden Ringspalte Auslässe für die beim Verdichtungsvorgang (Kompressionsvorgang) durch Verringerung des Matrizenfüllraums komprimierte Gasphase (normalerweise Luft oder Stickstoff). Um einen möglichst gleichmäßigen Ringspalt zu gewährleisten, kann z. B. wie in der DE-A 197 14 430 bezüglich der Herstellung von kreiszylinderförmigen Formkörpern durch Tablettieren von pulverförmigem Haufwerk beschrieben vorgegangen werden. Die vorstehend beschriebenen Ringspalte sind aber auch ursächlich dafür, dass am resultierenden Pressling sowohl im Bereich der Grundfläche als auch im Bereich der Deckfläche in geringem Umfang ein Grat entstehen kann. Die Verdichtung des pulverförmigen Haufwerks innerhalb eines Grats ist weniger stark ausgeprägt als im Bulk des resultierenden Komprimats.

Die Entfernung des Grats von ansonsten kreiszylinderförmigen Vorläuferformkörper ist daher im weiteren Verlauf der Bearbeitung desselben vergleichsweise leicht möglich. In der Regel brechen die Grate z. B. im Rahmen einer analog zur Beschreibung der Deutschen Anmeldung mit dem Aktenzeichen 102007028332.8 durchzuführenden Bruchsiebung von alleine ab und werden abgetrennt. Im Übrigen muss sich die Breite der vorbeschriebenen Ringspalte unter anderem an der Körnung des erfindungsgemäß zu verdichtenden pulverförmigen Haufwerks orientieren. D. h., die Breite der Ringspalte sollte in der Regel so limitiert sein, dass sie nicht größer ist als das zweifache (besser nicht größer ist als das Einfache) der im zu verdichtenden pulverförmigen Haufwerk am häufigsten vertretenen Längstausdehnung des Pulverkorns (die Längstausdehnung eines Pulverkorns ist die längste direkte geradlinige Verbindung zweier auf der Oberfläche des Pulverkorns befindlicher Punkte; besteht das pulverförmige Haufwerk aus durch Agglomeration von Primärkorn erzeugtem Sekundärkorn, ist es normalerweise zweckmäßig, zur Bemessung der noch tolerierbaren Ringspaltbreite die Längstausdehnung des Primärkorns heranzuziehen). Häufig betragen vorgenannte Ringspaltbreiten beim erfindungsgemäßen Verfahren wenige Hundertstel Millimeter. Die Matrize selbst weist üblicherweise eine plane obere Stirnfläche auf. Anwendungstechnisch zweckmäßig ist auch die untere Stirnfläche der Matrize plan gestaltet und die Bohrachse B verläuft zu beiden Ebenen senkrecht. Vorzugsweise weist die Matrize (sieht man von der Matrizenbohrung ab) die Form eines Kreiszylinders mit einer planen oberen und einer planen unteren Stirnfläche auf. In der äußeren Wand des Kreiszylinders verläuft auf halber Höhe vorteilhaft ein horizontal verlaufender Ring bzw. eine runde Vertiefung. Sie dient der Fixierung der Matrize in der Matrizenscheibe mittels einer oder mehreren Befestigungsschrauben.

Grundsätzlich kann beim erfindungsgemäßen Verfahren die Verringerung des Ausgangsabstands A auf den Endabstand E so erfolgen, dass beide (der untere und der obere) Stempel aktiv aufeinander zu bewegt werden. Selbstverständlich kann aber auch so vorgegangen werden, dass der unter Stempel seine Position beibehält und nur der obere Stempel bewegt (nach unten abgesenkt) wird.

In der Regel ist es für eine möglichst gleichmäßige Oberflächenhärte des beim erfindungsgemäßen Verfahren resultierenden Komprimats (geometrischen Vorläuferformkörpers) ganz generell vorteilhaft, wenn bei der Verrringerung des Ausgangsabstands A auf den Endabstand E Ober- und Unterstempel gemeinsam aufeinander zu bewegt werden (der obere Stempel wird abgesenkt, der untere Stempel wird angehoben). In diesem Fall wird der erforderliche Kompressionsdruck auf das pulverförmige Haufwerk vom oberen und vom unteren Stempel gleichmäßig ausgeübt, was eine einheitlichere Seitendruckfestigkeit des resultierenden Komprimats über dessen Höhe bedingt. Auch resultiert dabei ein geometrischer Vorläuferformkörper mit einer homogeneren Massendichte über seine gesamte Dimension. Letzteres bedingt nach der thermischen Behandlung ein homogeneres Porengefüge und auf letzterem basierend schlussendlich eine verbesserte Katalysatorperformance. Zum Einstellen des Ausgangsabstands A befindet sich der untere Stempel (sprachlich vereinfacht in dieser Schrift auch "Unterstempel") üblicherweise in der Matrizenbohrung, während der Oberstempel diese freigibt, damit das pulverförmige Haufwerk von oben eingeführt werden kann. Anschließend wird der Oberstempel abgesenkt, bis seine untere Stirnfläche das in der Matrizenbohrung auf der oberen Stirnfläche des unteren Stempels aufliegende pulverförmige Haufwerk berührt. Von dem so eingestellten Ausgangsabstand A der beiden Stirnflächen ausgehend wird anschließend, wie bereits beschrieben, der axiale Endabstand E der beiden Stirnflächen eingestellt. Die im Endabstand E bei vorstehend beschriebener Verfahrensweise von beiden Stempeln (bzw. wenigstens vom Oberstempel) in typischer Weise ausgeübten Pressdrucke liegen üblicherweise im Bereich von 50 bis 5000 kg/cm². Bei Bedarf kann auch unter Anwendung eines Vordrucks zunächst auf einen vorläufigen Endabstand verdichtet werden. Die diesbezüglich angewendeten Vordrucke betragen typisch 5 - 500 kg/cm³. Die zur anschließenden Endverdichtung auf den zugehörigen endgültigen Endabstand angewandten Haupt(verdichtungs)drucke sind normalerweise größer als die angewendeten Vordrucke.

Nach beendeter Verdichtung wird der obere Stempel vom gebildeten kreiszylinderförmigen Vorläuferformkörper abgehoben und der kreiszylindrische Vorläuferformkörper durch Anheben des unteren Stempels aus der Matrizenbohrung entfernt. Um die dabei zwischen der Mantelfläche des kreiszylindrischen Vorläuferformkörpers und der Innenwand der Matrizenbohrung auftretende Rollreibung zu verringern ist es anwendungstechnisch zweckmäßig, die Matrizenbohrung nicht so zu gestalten, dass die geometrische Form der Innenwand der Matrizenbohrung derjenigen der Mantelfläche eines Kreiszylinders KZ* sondern wenigstens im oberen Teil der Matrizenbohrung derjenigen der Mantelfläche eines sich von unten nach oben geringfügig erweiternden Kegelstumpfes entspricht, dessen Querschnittsfläche an seinem unteren Ende der Querschnittsfläche des Kreiszylinders KZ* entspricht (im Unterschied zum Kreiszylinder ist die Querschnittsfläche eines Kegelstumpfs über die Höhe des Kegelstumpfs nicht konstant, sondern nimmt von der Deckfläche zur Grundfläche hin zu). Die beschriebene Geometrie von oberem und unterem Stempel wird dabei beibehalten. Der Durchmesser des oberen und des unteren Stempels beträgt im vorstehend beschriebenen Fall einer Herstellung von kreiszylindrischen Vorläuferformkörpern in vielen Fällen ebenfalls 2 bis 10 mm, oder 2 bis 8 mm, oder 4 bis 8 mm, oder 5 bis 7 mm.

In entsprechender Weise wie kreiszylindrische Vorläuferformkörper können auch kreisringzylindrische oder kurz "ringförmige" Vorläuferformkörper hergestellt werden.
Aus Gründen einer möglichst verschwindenden Rollreibung zwischen der Mantelfläche von erzeugtem Komprimat und der Innenwand der Matrizenbohrung, wird auch in diesem Fall anwendungstechnisch vorteilhaft eine Matrizenbohrung eingesetzt, deren Innenwand wenigstens im oberen Teil der Matrizenbohrung die geometrische Form der Mantelfläche eines sich von unten nach oben geringfügig erweiternden Kegelstumpfes aufweist. Im Folgenden soll daher nur diese Fallgestaltung detailliert ausgeführt werden (die Herstellung ringförmiger Vorläuferformkörper erfolgt analog, der Längsabschnitt II der Matrizenbohrung ist dann jedoch ein weitergeführter Längsabschnitt I). Die resultierenden geometrischen Vorläuferformkörper sollen in dieser Schrift als "ringähnliche" Vorläuferformkörper bezeichnet werden. Die Herstellung "ringförmiger" Vorläuferformkörper ist in völlig entsprechender Weise möglich. Der Unterschied besteht letztlich nur darin, dass die Matrizenbohrung dann von oben nach unten keine Vorweite aufweist.

Im Fall einer Herstellung von ringähnlichen Vorläuferformkörpern wird der sich in einer mit einer vertikalen Bohrachse B von oben nach unten durch das Matrizenmaterial hindurchgeführten Matrizenbohrung befindliche Füllraum anwendungstechnisch geschickt zusätzlich zur Innenwand der Matrizenbohrung, der oberen Stirnfläche des unteren Stempels sowie der unteren Stirnfläche des oberen Stempels durch die Mantelfläche eines aus der geometrischen Mitte der oberen Stirnfläche des unteren Stempels heraus entlang der Bohrachse B in der Matrizenbohrung von unten nach oben geführten Mittelstiftes MF, der wenigstens bis zur geometrischen Mitte der unteren Stirnfläche des oberen Stempels hinaufreicht, begrenzt, und, anwendungstechnisch zweckmäßig

- entspricht die geometrische Form der (äußeren) Mantelfläche des unteren Stempels derjenigen der Mantelfläche eines Kreiszylinders I,

- entspricht die geometrische Form der (äußeren) Mantelfläche des oberen Stempels derjenigen der Mantelfläche eines Kreiszylinders II,

- ist in der geometrischen Mitte der oberen Stirnfläche des unteren Stempels eine von oben nach unten durch den unteren Stempel hindurchgeführte Mittelbohrung MB$^U$ ausgebildet;

- ragt im Ausgangsabstand A der beiden Stirnflächen der Mittelstift MF von unten durch die Mittelbohrung MB$^U$ hindurch bis wenigstens zur geometrischen Mitte der unteren Stirnfläche des oberen Stempels hinauf;

- weist der Mittelstift MF von unten nach oben die geometrische Form eines Kreiszylinders Z mit einer kreiszylindrischen Mantelfläche MZ auf;

- ist die Länge der Umrisslinie des Kreiszylinders Z kleiner als die Länge der Umrisslinie des Kreiszylinders I sowie kleiner als die Länge der Umrisslinie des Kreiszylinders II;

- sind die Position des Mittelstiftes MF und die Position der Matrize einschließlich der Matrizenbohrung längs der Bohrachse B während des Verfahrens relativ zueinander fixiert;

- ist in der geometrischen Mitte der unteren Stirnfläche des oberen Stempels eine in den oberen Stempel hineinführende und mit wenigstens einem Auslass aus dem oberen Stempel (gasdurchlässig) in Verbindung stehende Mittelbohrung $MB^O$ ausgebildet, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann;

- liegen die Symmetrieachsen der Matrizenbohrung, des Kreiszylinders I, des Kreiszylinders II, der Mittelbohrung MB°, des Mittelstiftes MF und der Mittelbohrung $MB^U$ auf einer gemeinsamen durch die Matrizenbohrung vertikal verlaufenden geraden Linie L;

- weist die Matrizenbohrung längs ihrer Bohrachse einen Längsabschnitt I auf, auf dessen Länge I die geometrische Form der Innenwand der Matrizenbohrung derjenigen der Mantelfläche eines Kreiszylinders KZ entspricht, und an dessen oberem Ende sich unmittelbar ein nach oben gerichtete Längsabschnitt II der Matrizenbohrung anschließt, der die Länge II aufweist;

- sind die Ausmaße des Längsabschnitts I der Matrizenbohrung und des Kreiszylinders I so beschaffen, dass der untere Stempel während des Verfahrens (der Verringerung des Ausgangsabstands A auf den Endabstand E) stets jeweils wenigstens auf einer Teillänge (vorzugsweise auf einer Teillänge von wenigstens 10 %, oder von wenigstens 20 %, oder von wenigstens 30 % (in der Regel jedoch ≤ 90 %, oder ≤ 80 %) der Länge I) des Längsabschnitts I mit seiner (äußeren) Mantelfläche auf der Innenwand der Matrizenbohrung gleitend in die Matrizenbohrung geführt ist;

- sind die Ausmaße der Mittelbohrung $MB^U$ und des Kreiszylinders Z so beschaffen, dass der untere Stempel während des Verfahrens (der Verringerung des Ausgangsabstands A auf den Endabstand E) stets wenigstens im Bereich des Eingangs seiner Mittelbohrung $MB^U$ in seine obere Stirnfläche mit der Innenwand der Mittelbohrung $MB^U$ auf der kreiszylindrischen Mantelfläche MZ des Mittelstiftes MF gleitend in die Matrizenbohrung geführt ist;

- entspricht die geometrische Form der Innenwand der Matrizenbohrung auf der Länge II des Längsabschnitts II von unten nach oben derjenigen der Mantelfläche eines sich von unten nach oben erweiternden Kegelstumpfes KS, dessen Querschnittsfläche an seinem unteren Ende der Querschnittsfläche des Kreiszylinders KZ an dessen oberem Ende entspricht, mit der Maßgabe, dass beim Erreichen des Endabstands E die untere Stirnfläche des oberen Stempels sich im Längsabschnitt II und die obere Stirnfläche des unteren Stempels sich nicht unterhalb des Längsabschnitts I befindet, so dass sich der durch das mechanische Verdichten des pulverförmigen Haufwerks zwischen den beiden Stirnflächen ausgebildete ringähnliche Vorläuferformkörper beim Erreichen des Endabstands E wenigstens teilweise im Längsabschnitt II befindet. D. h., beim Erreichen des Endabstands E zwischen der oberen Stirnfläche des unteren Stempels und der unteren Stirnfläche des oberen Stempels befindet sich wenigstens ein Teil der Abstandsstrecke zwischen den beiden Stirnfläche im Längsabschnitt II.

Vorteilhaft befinden sich bei der beschriebenen Herstellung von ringähnlichen Vorläuferformkörpern beim Erreichen des Endabstands E zwischen der oberen Stirnfläche des unteren Stempels und der unteren Stirnfläche des oberen Stempels wenigstens 20 % oder wenigstens 30 %, vorzugsweise wenigstens 40 % oder wenigstens 50 %, besonders bevorzugt wenigstens 60 % oder wenigstens 70 % und ganz besonders bevorzugt wenigstens 80 % bzw. wenigstens 90 % der Abstandsstrecke (oder 100 % der Abstandsstrecke, d. h., die gesamte Abstandsstrecke zwischen der oberen Stirnfläche des unteren Stempels und der unteren Stirnfläche des oberen Stempels bei Erreichen des Endabstands E) zwischen den beiden Stirnflächen im Längsabschnitt II der Matrizenbohrung. Am vorteilhaftesten ist das beschriebene Verfahren dann, wenn sich beim Erreichen des Endabstands E sowohl die unter Stirnfläche des oberen Stempels als auch die obere Stirnfläche des unteren Stempels im Längsabschnitt II der Matrizenbohrung befinden, so dass sich der gesamte durch das mechanische Verdichten des pulverförmigen Haufwerks zwischen den beiden Stirnflächen ausgebildete ringähnliche Vorläuferformkörper beim Erreichen des Endabstands E im Längsabschnitt II befindet. Dabei erweist es sich im vorgenannten Fall als günstig, wenn sich bereits im Zustand des Ausgangsabstands A sowohl die untere Stirnfläche des oberen Stempels als auch die obere Stirnfläche des unteren Stempels im Längsabschnitt II befinden.

Die Umrisslinie des Kreiszylinders II ist beim erfindungsgemäßen Verfahren anwendungstechnisch zweckmäßig normalerweise länger oder gleich lang wie die Umrisslinie des Kreiszylinders I. In der Regel sind die beiden vorgenannten Umrisslinien gleich lang.

Darüber hinaus liegen die obere Stirnfläche des unteren Stempels und die untere Stirnfläche des oberen Stempels vorteilhaft in zueinander parallelen Ebenen, auf denen die Bohrachse B senkrecht steht.

Das beschriebene Verfahren eignet sich insbesondere zur Herstellung solcher ringähnlicher Vorläuferformkörper, bei denen der Endabstand E (eine eventuelle Krümmung der Stirnflächen soll bei der Bestimmung der Abstände A und E keine Berücksichtigung finden; d. h., gemeint ist immer der Abstand der oberen/unteren Umrisslinie der zylindrischen Mantelfläche der Stempel) 2 bis 10 mm, oder 2 bis 8 mm, oder 3 bis 8 mm, oder 3 bis 7 mm beträgt. Sie alle sollen in dieser Schrift im Speziellen als ringähnliche Vorläuferformkörper F bezeichnet werden. Häufig beträgt dabei der Quotient Q aus der Länge der Umrisslinie des Kreiszylinders Z (Zähler) und der Länge der Umrisslinie des Kreiszylinders I (Nenner) 0,3 bis 0,7 oder 0,4 bis 0,6.

D. h., die Differenz, gebildet durch Substraktion des Radius der Umrisslinie des Kreiszylinders Z vom Radius der Umrisslinie des Kreiszylinders I, beträgt im Fall von ringähnlichen Formkörpern F vielfach 1 bis 3 mm, oder 1 bis 2 mm, oder 1,5 bis 2 mm, oder 1 bis 1,5 mm. Der Durchmesser der Umrisslinie des Kreiszylinders I beträgt im Fall von ringähnlichen Formkörpern F in vielen Fällen ebenfalls 2 bis 10 mm, oder 2 bis 8 mm, oder 4 bis 8 mm, oder 5 bis 7 mm.

Wie bereits gesagt, ist die Querschnittsfläche eines Kegelstumpfs im Unterschied zum Kreiszylinder über die Höhe des Kegelstumpfes nicht konstant, sondern nimmt von der Deckfläche zur Grundfläche hin zu.

Dies trifft selbstredend auch auf den Kegelstumpf KS zu, der auf der Länge des Längsabschnitts II in die Matrizenbohrung einbeschrieben werden kann und dessen Querschnittsfläche von unten nach oben zunimmt ("auf den Kopf gestellter Kegelstumpf").

Ist H die Höhe des Kegelstumpfes KS, ist es vorteilhaft, wenn die Aufweitung des Kegelstumpfes KS von unten (von der Deckfläche) nach oben (zur Grundfläche) so beschaffen ist, das zwischen dem Durchmesser DD der Deckfläche, dem Durchmesser DG der Grundfläche und der Höhe H des Kegelstumpfs KS die nachfolgende Beziehung erfüllt ist:

$$0,003 \bullet H \leq DG - DD \leq 0,050 \bullet H \qquad (I).$$

Vorzugsweise gilt beim erfindungsgemäßen Verfahren

$$0,005 \bullet H \leq DG - DD \leq 0,025 \bullet H \qquad (II).$$

Besonders bevorzugt gilt beim erfindungsgemäßen Verfahren

$$0,007 \bullet H \leq DG - DD \leq 0,015 \bullet H \qquad (III).$$

Das Vorgenannte trifft insbesondere im Fall einer erfindungsgemäßen Herstellung von ringähnlichen Vorläuferformkörpern F zu.

Normalerweise weist bei der erfindungsgemäßen Herstellung ringähnlicher Vorläuferformkörper sowohl die (für das pulverförmige Haufwerk zugängliche) obere Stirnfläche des unteren Stempels als auch die (für das pulverförmige Haufwerk zugängliche) untere Stirnfläche des oberen Stempels die geometrische Form der Stirnfläche eines Kreisringzylinders auf. D.h., beide Stirnflächen sind normalerweise Kreisringe, die vorzugsweise kongruent sind. Aus verschiedenen Gründen (vgl. z. B. EP-A 184 790) kann es jedoch zweckmäßig sein, eine oder beide der vorgenannten Stirnflächen (die beiden äußeren und die beiden inneren Kreise bleiben dabei vorzugsweise kongruent) z.B. konkav zu gestalten (d.h., der Kreisring ist ins Stempelinnere einwärts gewölbt). In diesem Fall weist die entsprechende Stirn des zugehörigen Stempels die geometrische Form einer kreisförmigen Rille (36) (= eine kreisförmige Vertiefung; bei einer Herstellung von ringähnlichen Vorläuferformkörpern F liegt die Rillentiefe in der Regel bei ≤ 2 mm) auf. Die daraus jeweils resultierende Stirnfläche des erfindungsgemäß hergestellten ringähnlichen Vorläuferformkörpers ist dann in entsprechender Weise nicht plan, sondern nach außen (konvex) gewölbt. Eine derartige Ausgestaltung erweist sich insbesondere im Fall von erfindungsgemäß hergestellten Trägerformkörpern als vorteilhaft. Durch die gekrümmten Stirnflächen kommt es bei der Herstellung daraus resultierender Träger- oder Schalenkatalysatoren in einem geringeren Umfang zur Ausbildung von unerwünschten Zwillingen oder Drillingen der resultierenden Katalysatorformkörper. Der Radius einer solchen Krümmung beträgt in der Regel das 0,4- bis 5-fache des Außendurchmessers des Kreiszylinders I. Im übrigen gilt das in der EP-A 184 790 bezüglich der Vorteilhaftigkeit gekrümmter Stirnflächen von Hohlzylindern Gesagte in entsprechender Weise.

Grundsätzlich kann das Profil der oberen Stirn des unteren Stempels und/oder das Profil der unteren Stirn des oberen Stempels der erfindungsgemäßen Herstellung ringähnlicher Vorläuferformkörper aber auch in jedweder anderen für Tabletten (insbesondere pharmazeutische) bekannten Weise gestaltet sein. Beispielsweise können eine oder beide der oben genannten Stirnflächen auch konvex gestaltet werden. Auch kann eine der beiden Stirnflächen konkav und die andere konvex gestaltet werden. Im Fall einer Herstellung von ringähnlichen Vollkatalysatoren sind beide Stirnflächen jedoch vorzugsweise plan gestaltet.

Der Außendurchmesser des unteren Stempels ist bei der erfindungsgemäßen Herstellung ringähnlicher Vorläuferformkörper üblicherweise marginal kleiner als der Innendurchmesser der Matrizenbohrung im Längsabschnitt I, so dass der untere Stempel mit seiner Außenwand auf der Innenwand des Längsabschnitts I der Matrizenbohrung axial gleitend in die Matrizenbohrung eingeführt werden kann. Da sich beim Erreichen des Endabstandes E darüber hinaus nicht nur die untere Stirnfläche des oberen Stempels sondern vorzugsweise auch die obere Stirnfläche des unteren Stempels im Längsabschnitt II der Matrizenbohrung befindet, ist der Außendurchmesser des unteren Stempels somit regelmäßig kleiner als der Innendurchmesser der Matrizenbohrung auf der Höhe der oberen Stirnfläche des unteren Stempels beim Erreichen des Endabstands E. In entsprechender Weise ist der Außendurchmesser des oberen Stempels anwendungstechnisch zweckmäßig normalerweise etwas kleiner als der Innendurchmesser der Matrizenbohrung auf der Höhe der unteren Stirnfläche des oberen Stempels beim Erreichen des Endabstands E. Auf vorgenannte Art und Weise wird gewährleistet, dass sich sowohl der untere Stempel als auch der obere Stempel im verfahrenstechnisch erforderlichen Rahmen vergleichsweise frei in den relevanten Längsabschnitten der Matrizenbohrung bewegen können. Darüber hinaus bilden die so zwischen der unteren (bzw. oberen) Umrisslinie des oberen Stempels (bzw. unteren Stempels) und der Innenwand der Matrizenbohrung im Zustand des Ausgangsabstands A sowie im Zustand des Endabstands E bestehenden Ringspalte Auslässe für die beim Verdichtungsvorgang (Kompressionsvorgang) durch Verringerung des Matrizenfüllraums komprimierte Gasphase (normalerweise Luft oder Stickstoff). Um einen möglichst gleichmäßigen Ringspalt zu gewährleisten, kann z. B. wie in der DE-A 197 14 430 bezüglich der Herstellung von kreiszylinderförmigen Formkörpern durch Tablettieren von pulverförmigem Haufwerk beschrieben vorgegangen werden. Das Gleiten des unteren Stempels auf der Innenwand der Matrizenbohrung des Längsabschnittes I erweist sich in diesem Zusammenhang als ein wesentlicher Vorteil der beschriebenen Verfahrensweise.

Die vorstehend beschriebenen Ringspalte sind aber (wie bereits im Fall von kreiszylindrischen Vorläuferformkörpern beschrieben) auch ursächlich dafür, dass am erfindungsgemäß hergestellten ringähnlichen Pressling sowohl im Bereich der Grundfläche als auch im Bereich der Deckfläche in geringem Umfang ein Grat entstehen kann. Die Verdichtung des pulverförmigen Haufwerks innerhalb eines Grats ist weniger stark ausgeprägt als im Bulk des erfindungsgemäß erzeugten Komprimats. Die Entfernung des Grats vom ringähnlichen Vorläuferformkörper ist daher im weiteren Verlauf der Bearbeitung desselben vergleichsweise leicht möglich. In der Regel brechen die Grate z. B. im Rahmen einer wie in der Deutschen Anmeldung mit dem Aktenzeichen 102007028332.8 beschrieben durchzuführenden Bruchsiebung von alleine ab und werden abgetrennt.

Im Übrigen muss sich (wie bereits gesagt) die Breite der vorbeschriebenen Ringspalte unter anderem an der Körnung des erfindungsgemäß zu verdichtenden pulverförmigen Haufwerks orientieren. D. h., die Breite der Ringspalte sollte in der Regel so limitiert sein, dass sie nicht größer ist als das Zweifache (besser nicht größer ist als das Einfache) der im zu verdichtenden pulverförmigen Haufwerk am häufigsten vertretenen Längstausdehnung des Pulverkorns (die Längstausdehnung eines Pulverkorn ist die längste direkte geradlinige Verbindung zweier auf der Oberfläche des Pulverkorns befindlicher Punkte; besteht das pulverförmige Haufwerk aus durch Agglomeration von Primärkorn erzeugtem Sekundärkorn, ist es in der Regel zweckmäßig, zur Bemessung der noch tolerierbaren Ringspaltbreite die Längstausdehnung des Primärkorns heranzuziehen).

Im Rahmen der erfindungsgemäßen Herstellung von ringähnlichen Vorläuferformkörpern F betragen die vorgenannten Ringspaltbreiten in der Regel wenige (normalerweise weniger als zehn, meist weniger als fünf) Hundertstel Millimeter, und dies auch dann, wenn sich beim Erreichen des Endabstands E beide Stirnflächen im Längsabschnitt II befinden. Vorzugsweise ist auch in diesem Fall die Umrisslinie des Kreiszylinders II gleich lang wie die Umrisslinie des Kreiszylinders I.

Prinzipiell kann die Matrizenbohrung bei der erfindungsgemäßen Herstellung ringähnlicher Vorläuferformkörper nur aus den Längsabschnitten I (31) und II (32) bestehen (nur die Längsabschnitte I und II aufweisen).

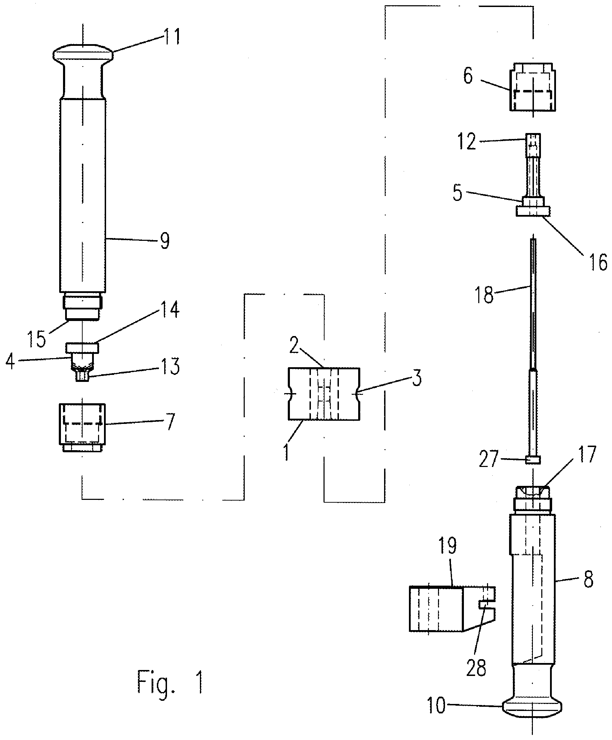

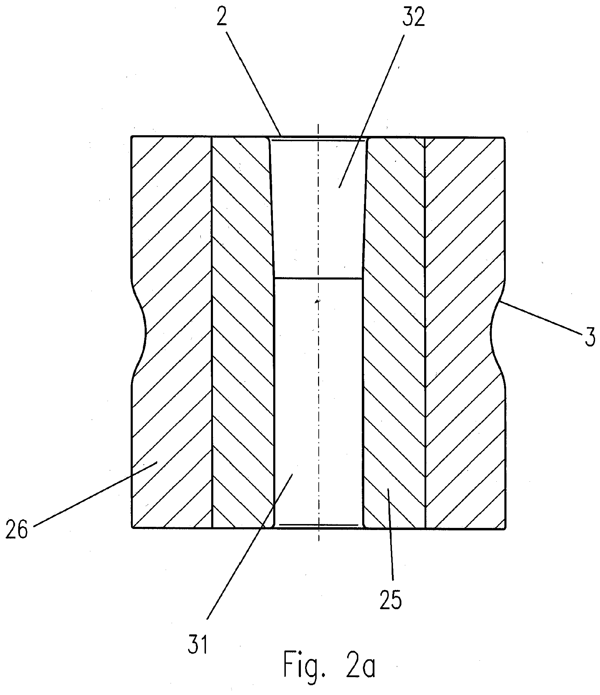

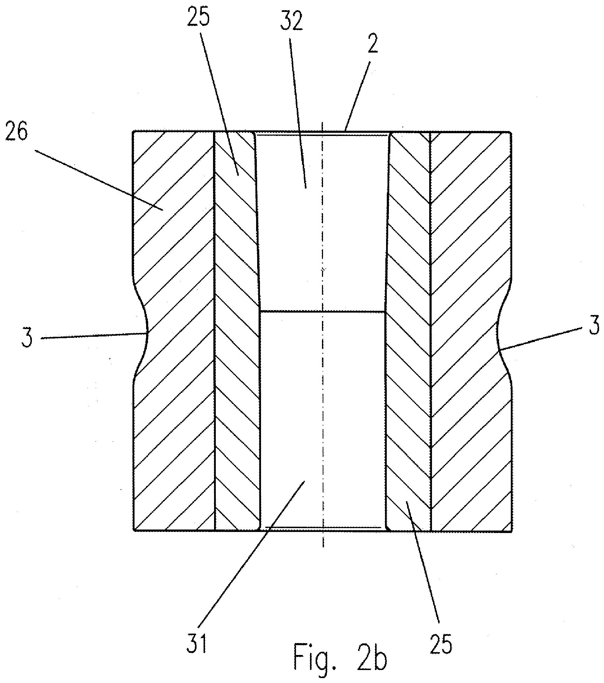

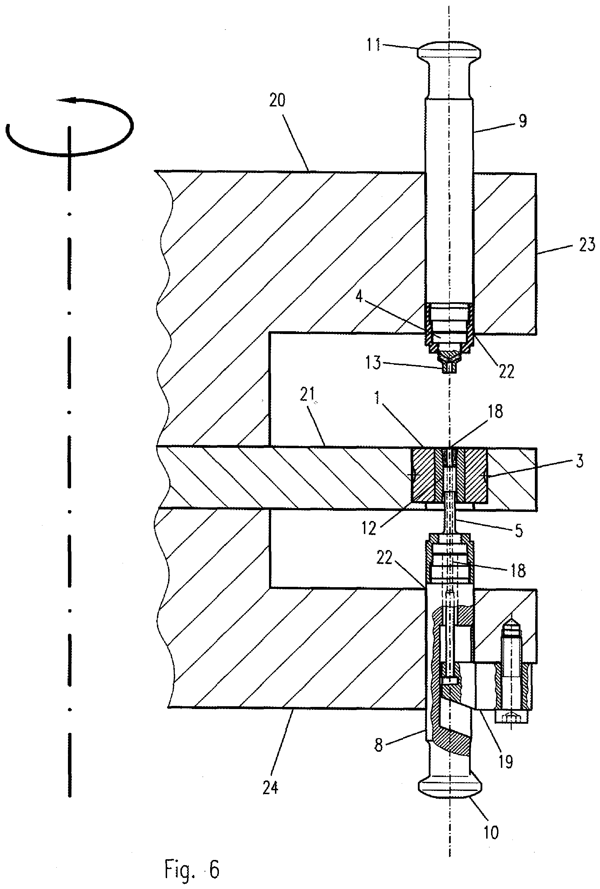

Matrizen mit derartigen Matrizenbohrungen sollen in dieser Schrift als "Matrizen mit einfachem Kegelstumpf" bezeichnet werden. Einen Längsabschnitt durch Matrizen dieser Art zeigen beispielhaft die Figuren 2a und 2b dieser Schrift (an ihrem oberen und an ihrem unteren Ende ist die Matrizenbohrung anwendungstechnisch zweckmäßig leicht abgerundet, um die Verletzungsgefahr durch scharfe Kanten zu minimieren; generell folgen die Figuren 1 bis 8 dieser Schrift in ihrer zeichnerischen Darstellung den Vorgaben im "Tabellenbuch Metall", Verlag Europa Lehrmittel, 41. Auflage, 1999 (D-42781-Haan Gruiten); aus Gründen der Übersichtlichkeit wurden in Figur 6 nicht alle Schnitte vollständig dargestellt; es wird diesbezüglich auf die Einzeldarstellungen verwiesen). Selbstverständlich können sich an die Längsabschnitte I und II der Matrizenbohrung einer Matrize sowohl nach oben als auch nach unten unmittelbar weitere Längsabschnitte anschließen. Wesentlich ist dabei lediglich, dass der unter Stempel (der obere Stempel) durch sich an den Längsabschnitt I (den Längsabschnitt II) der Matrizenbohrung gegebenenfalls nach unten (oben) anschließende weitere Längsabschnitte in den Längsabschnitt I (in den Längsabschnitt II) eingeführt werden kann.

Insbesondere aus ökonomischen Gründen besonders vorteilhaft ist zur Herstellung ringähnlicher Vorläuferformkörper die Verwendung von Matrizen, deren Matrizenbohrung so beschaffen ist, dass sich an ihren Längsabschnitt I nicht nur an dessen oberem Ende unmittelbar ein nach oben gerichteter Längsabschnitt II, sondern auch an dessen unterem Ende ein nach unten gerichteter Längsabschnitt anschließt (in dieser Schrift als Längsabschnitt II* (33) bezeichnet), bei dem die geometrische Form der Innenwand der Matrizenbohrung auf der Länge II* des Längsabschnitts II* ebenfalls der Mantelfläche eines Kegelstumpfes entspricht, dessen Querschnittsfläche an seinem oberen Ende der Querschnittsfläche des Kreiszylinders KZ an dessen unterem Ende entspricht (in dieser Schrift als Kegelstumpf KS* bezeichnet), jedoch mit einem sich nach unten erweiternden Querschnitt (vorzugsweise erfüllen auch der Durchmesser der Deckfläche, der Durchmesser der Grundfläche und die Höhe des Kegelstumpfes KS* wenigstens eine der Beziehungen (I), (II) oder (III)). Matrizen mit Matrizenbohrungen, die nur die Längsabschnitte II*, I und II aufweisen, werden in dieser Schrift als "Matrizen mit doppeltem Kegelstumpf" bezeichnet (selbstredend können sich auch an die Längsabschnitte II und II* grundsätzlich unmittelbar weitere Längsabschnitte anschließen, so lange der jeweilige Stempel in selbige eingeführt werden kann).

Vorteilhaft entsprechen dabei die geometrischen Ausmaße des Längsabschnitts II* denen des Längsabschnitts II. Matrizen der vorgenannten Art sind insofern besonders vorteilhaft, als die erfindungsgemäße Verdichtung damit z. B. zunächst in der oberen Hälfte des Längsabschnitts I und/oder im Längsabschnitt II der Matrizenbohrung durchgeführt werden kann. Ist die Innenwand der Matrizenbohrung im vorgenannten Bereich aufgrund vielfacher Verfahrensdurchführung in selbigem abgenutzt, kann die Matrize in einfacher Weise auf den Kopf gestellt (um eine senkrecht zur Matrizenbohrung liegende Achse um 180° gedreht werden) und die erfindungsgemäße Verdichtung anschließend in der anderen Hälfte des Längsabschnitts I und/oder im Längsabschnitt II* der Matrizenbohrung durchgeführt werden. Ganz besonders vorteilhaft ist die Verfahrensdurchführung mit Matrizen, deren Matrizenbohrung nur aus einem Längsabschnitt I und einem sich an dessen oberes Ende unmittelbar anschließenden Längsabschnitt II sowie einem sich an dessen unteres Ende unmittelbar anschließenden Längsabschnitt II* besteht und wobei die Geometrie des Längsabschnitts II der Matrizenbohrung zur Geometrie des Längsabschnitts II* der Matrizenbohrung kongruent ist (= "Matrize mit kongruentem doppeltem Kegelstumpf").

Ferner ist es anwendungstechnisch geschickt, wenn die Länge II (Höhe) des Längsabschnitts II (sowie die Länge II* des Längsabschnitts II*) der Matrizenbohrung der zur Durchführung des erfindungsgemäßen Verfahrens zur Herstellung ringähnlicher Vorläuferformkörper verwendeten Matrize (d. h., die Höhe H des Kegelstumpfes KS (sowie des Kegelstumpfes KS*)) bis zum Vierfachen, vorzugsweise bis zum Dreifachen oder bis zum Zwei- oder Eineinhalbfachen des axialen Endabstands E beträgt.

D. h., vorteilhaft sind Verfahren für die zutrifft:

$$4 \cdot \text{Endabstand E} \geq H \geq 1 \cdot \text{Endabstand E} \qquad \text{(IV)};$$

oder

$$3 \cdot \text{Endabstand E} \geq H \geq 1 \cdot \text{Endabstand E} \qquad \text{(V)};$$

oder

$$1{,}5 \cdot \text{Endabstand E} \geq H \geq 1 \cdot \text{Endabstand E} \qquad \text{(VI)};$$

oder

$$3 \cdot \text{Endabstand E} \geq H \geq 1{,}5 \cdot \text{Endabstand E} \qquad \text{(VII)};$$

oder

$$2 \cdot \text{Endabstand E} \geq H \geq 1{,}5 \cdot \text{Endabstand E} \qquad \text{(VIII)}.$$

In der Regel wird beim erfindungsgemäßen Verfahren zur Herstellung ringähnlicher Vorläuferformkörper die Länge I des Längsabschnitts I größer sein als die Länge II des Längsabschnitts II (sowie größer als die Länge II* des Längsabschnitts II*). Die Länge I des Längsabschnitts I kann aber auch kleiner sein als die Länge II des Längsabschnitts II (sowie kleiner als die Länge II* des Längsabschnitts II*).

Üblicherweise wird die Länge I jedoch nicht mehr als das Dreifache der Länge II (als das Dreifache der Länge II*) betragen.

Häufig beträgt die Länge I nicht mehr als das Zweifache (oder Einfache) der Länge II (als das Zweifache (oder Einfache) der Länge II*).

Normalerweise beträgt die Länge I nicht weniger als das 0,1-fache (bzw. nicht weniger als das 0,2-fache) der Länge II (als das 0,1-fache bzw. 0,2-fache der Länge II*).

Vielfach beträgt die Länge I das 0,1- bis 1-fache oder das 0,5 bis 1-fach der Länge II (bzw. der Länge II*).

Alles Vorgenannte gilt, wie sonst auch in dieser Schrift, insbesondere für den Fall einer erfindungsgemäßen Herstellung von ringähnlichen Vorläuferformkörpern F.

Im Besonderen gilt alles in dieser Schrift Gesagte für eine erfindungsgemäße Herstellung von ringähnlichen Vorläuferformkörpern F, bei deren Herstellung beim Erreichen des Endabstands E sowohl die obere Stirnfläche des unteren Stempels als auch die untere Stirnfläche des oberen Stempels sich im Längsabschnitt II (oder im Längsabschnitt II*) der Matrizenbohrung befinden. Eine derartige Herstellung von ringähnlichen Vorläuferformkörpern F wird in dieser Schrift im engeren Sinn als eine Herstellung von ringähnlichen Vorläuferformkörpern $F^{LII}$ bezeichnet (unabhängig von den quantitativen Ausmaßen der erfindungsgemäß hergestellten ringähnlichen Vorläuferformkörper, sollen diejenigen, bei deren Herstellung beim Erreichen des Endabstands E sowohl die obere Stirnfläche des untern Stempels als auch die unter Stirnfläche des oberen Stempels sich im Längsabschnitt II (oder im Längsabschnitt II*) der Matrizenbohrung befindet, in dieser Schrift als "ringähnliche Vorläuferformkörper LII" bezeichnet werden).

Die Vorteilhaftigkeit von Verfahren zur Herstellung von ringähnlicher Vorläuferformkörpern, auf die wenigstens eine der Beziehungen (IV) bis (VIII) zutrifft, liegt unter anderem darin begründet, dass insbesondere im Rahmen der Herstellung einer größeren Charge von ringähnlichen Vorläuferformkörpern LII mit der erfindungsgemäßen Verdichtung zunächst im oberen Teil des Längsabschnitts II begonnen werden kann (d.h., im Zustand des Ausgangsabstands A befinden sich sowohl die obere Stirnfläche des unteren Stempels als auch die untere Stirnfläche des oberen Stempels im oberen Teil des Längsabschnitts II; erfindungsgemäß vorteilhaft wird man am Beginn des Verfahrens im Zustand des Ausgangsabstands A die unter Fläche des oberen Stempels so positionieren, dass sie mit dem oberen Ende des Längsabschnitts II bündig abschließt). Mit zunehmender Abnutzung der Innenwand des oberen Teils des Längsabschnitts II der Matrizenbohrung wird man dann im Zustand des Ausgangsabstands A sowohl die untere Stirnfläche des oberen Stempels als auch die obere Stirnfläche des unteren Stempels innerhalb der Matrizenbohrung nach unten verschieben. Die im Rahmen einer solchen Vorgehensweise resultierenden ringähnlichen Vorläuferformkörper (z. B. ringähnlichen Vorläuferformkörper LII oder ringähnlichen Vorläuferformkörper $F^{LII}$) sind einander geometrisch so ähnlich, dass sie äquivalent zu geometrisch einheitlichen Vorläuferformkörpern (z. B. als Katalysatoren oder Katalysatorträger) verwendet werden können. In bestimmten Fällen (vgl. z. B. die Deutsche Anmeldung mit dem Aktenzeichen 102007017080.9) kann eine definierte Varianz der Formkörpergeometrie über eine Produktionscharge sogar vorteilhaft sein. Dabei ist zu beachten, dass beim Übergang vom ringähnlichen Vorläuferformkörper zum oxidischen Formkörper durch thermische Behandlung des ersteren in der Regel eine Veränderung der Formkörpergeometrie einhergeht.

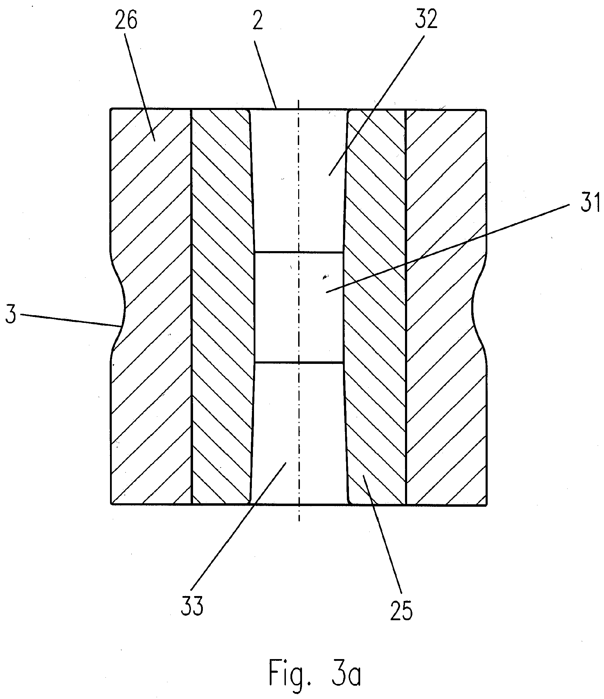

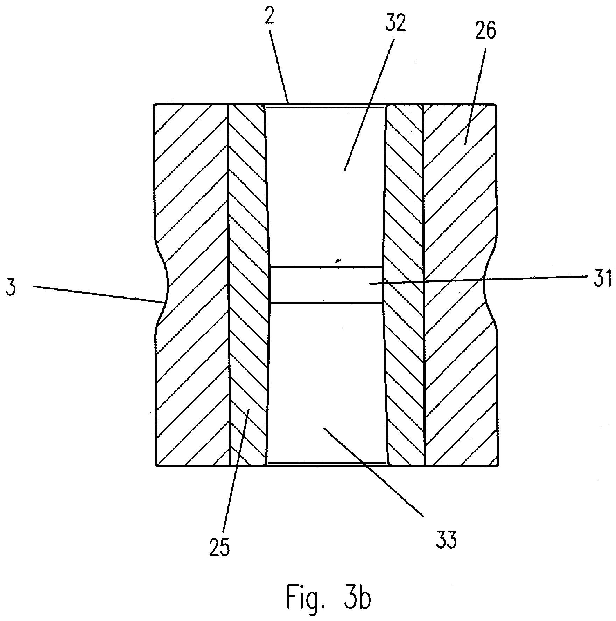

Einen Längsschnitt durch erfindungsgemäß geeignete Matrizen mit kongruentem doppeltem Kegelstumpf zeigen beispielhaft die Figuren 3a und 3b.

Wesentlich für das beschriebene Verfahren zur Herstellung ringähnlicher Vorläuferformkörper ist, dass in der geometrischen Mitte der unteren Stirnfläche des oberen Stempels eine in den oberen Stempel hineinführende und mit wenigstens einem Auslass aus dem oberen Stempel in Verbindung stehende Mittelbohrung $MB^O$ ausgebildet ist, die den Mittelstift MF bei der Verringerung des Ausgangsabstands A auf den Endabstand E im erforderlichen Umfang aufzunehmen vermag und in die der Mittelstift MF bereits im Ausgangsabstand A hineinragen kann. Der Mittelstift MF wird bei der Verfahrensausübung insbesondere dann im Zustand des Ausgangsabstands A bereits in die Mittelbohrung $MB^O$ hineinragen, wenn, wie vorstehend beschrieben, im oberen Teil des Längsabschnitts II die Innenwand der Matrizenbohrung bereits abgenutzt ist, und die erfindungsgemäße Verdichtung aus diesem Grund in den weiter unten gelegenen Teil des Längsabschnitts II verlagert wird.

Wird beim erfindungsgemäßen Verfahren zur Herstellung ringähnlicher Vorläuferformkörper im Rahmen des Verdichtungsvorgangs der obere Stempel abgesenkt, muss die Mittelbohrung $MB^O$ (35) den Mittelstift MF jedoch in jedem Fall in dem Umfang aufnehmen, indem die Absenkung des oberen Stempels erfolgt.

Da die Mittelbohrung $MB^O$ (im Unterschied zur Mittelbohrung $MB^U$ (37), die durch den unteren Stempel hindurchgeführt ist) normalerweise nicht durch den oberen Stempel hindurchgeführt ist, bedarf es anwendungstechnisch zweckmäßig wenigstens eines Auslasses (34), mit dem die Mittelbohrung $MB^O$ in Verbindung steht und über den diejenige Gasphase entweichen kann (ausgelassen wird), die der Mittelstift MF bei seiner Aufnahme in die Mittelbohrung $MB^O$ im Rahmen eines Absenkens des oberen Stempels verdrängt. In der Regel ist der wenigstens eine Auslass ebenfalls als Bohrung ausgestaltet, die schräg auf die Mittelbohrung $MB^O$ zuläuft.

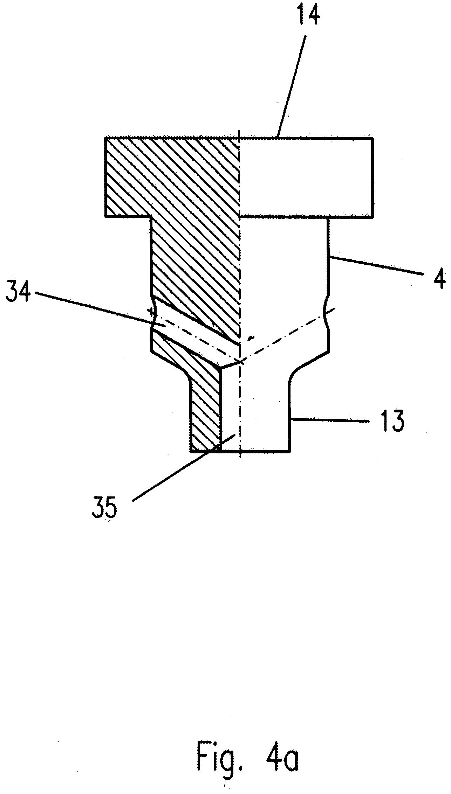

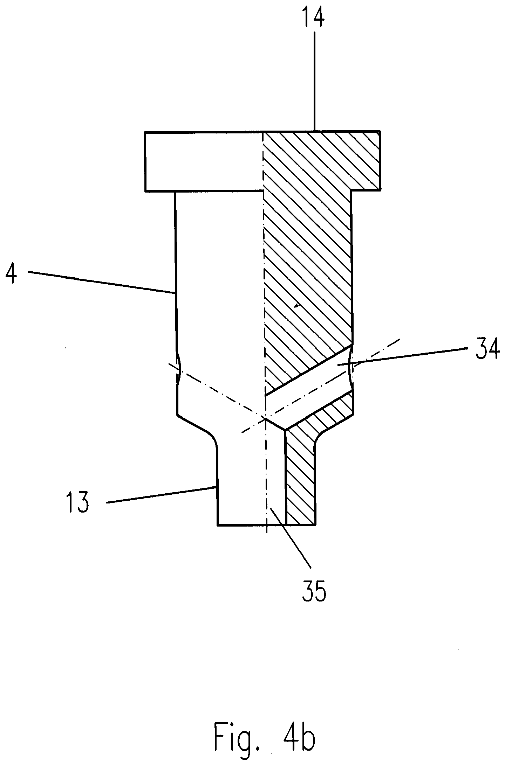

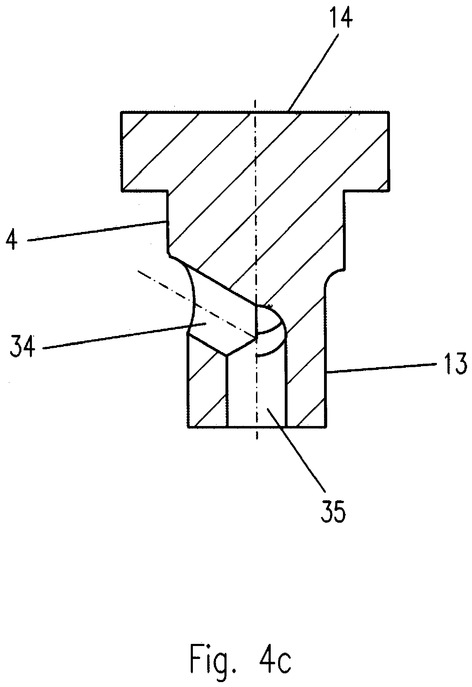

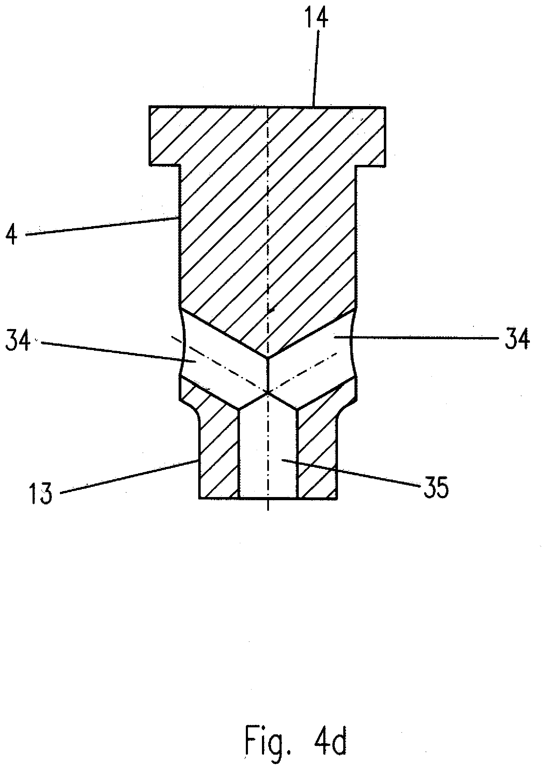

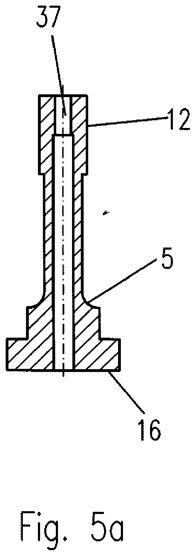

Die Figuren 4a, 4b, 4c und 4d zeigen Längsschnitte durch entsprechende mit wenigstens einem Auslass versehene Oberstempel, wobei der eigentliche obere Stempel im Sinne dieser Erfindung lediglich den nach unten kreiszylindrisch (bzw. ringförmig) auslaufenden Hals in diesen Figuren meint. Die Gesamtfigur zeigt jeweils die Ausgestaltung des erfindungsgemäßen oberen Stempels als sogenannter oberer "Einlegestempel", auf den im weiteren Verlauf dieser Schrift noch Bezug genommen werden wird. Die Verbindung der Mittelbohrung $MB^O$ zu wenigstens einem Auslass ist insbesondere dann von besonderer Bedeutung, wenn wenigstens der Eingang in die Mittelbohrung $MB^O$ vorzugsweise kreiszylindrisch so gestaltet ist, dass die Mantelfläche des Kreiszylinders Z (des kreiszylindrischen Mittelstiftes MF) bei seiner Aufnahme in die Mittelbohrung $MB^O$ wenigstens im Eingangsbereich derselben auf ihrer Innenwand gleitet. Erfindungsgemäß bevorzugt ist die Mittelbohrung $MB^O$ so gestaltet, dass die Geometrie ihrer Innenwand entlang ihrer gesamten Längsachse der Mantelfläche eines Kreiszylinders entspricht. In diesem Fall sind die Ausmaße des kreiszylindrischen Mittelstiftes MF und der Mittelbohrung $MB^O$ vorzugsweise so gestaltet, dass die Mantelfläche des kreiszylindrischen Mittelstiftes MF (des Kreiszylinders Z) im gesamten Umfang seiner Aufnahme in die Mittelbohrung $MB^O$ auf der Innenwand der Mittelbohrung $MB^O$ gleitet. Im Unterschied dazu ist die Mittelbohrung $MB^U$ unterhalb des Bereichs ihres Eingangs in die obere Stirnfläche des unteren Stempels häufig geringfügig erweitert gestaltet, wie es z. B. im Längsschnitt durch einen unteren Stempel in der Figur 5a gezeigt wird.

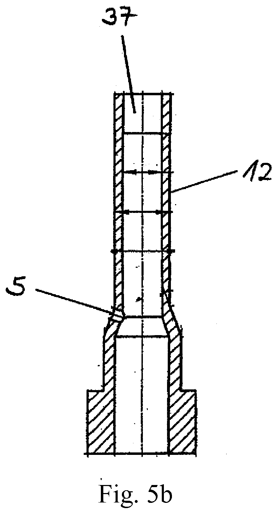

Im Unterschied dazu zeigt die Figur 5b einen Längsschnitt durch einen unteren Stempel, bei dem die Mittelbohrung $MB^U$ über die gesamte Länge des unteren Stempels mit konstantem zylindrischem Querschnitt gestaltet ist. Auch in den Figuren 5a und 5b ist mit dem unteren Stempel im Sinne dieser Erfindung lediglich der nach oben kreiszylindrisch (bzw. ringförmig) auslaufende Hals in diesen Figuren gemeint. Die Gesamtfigur zeigt jeweils die Ausgestaltung des erfindungsgemäßen unteren Stempels als sogenannter "unterer Einlegestempel", auf den im weiteren Verlauf dieser Schrift noch Bezug genommen werden wird. Die Öffnung der Mittelbohrung $MB^U$ in der oberen Stirnfläche des unteren Stempels und die Öffnung der Mittelbohrung $MB^O$ in der unteren Stirnfläche des oberen Stempels sind normalerweise kongruent gestaltet.

Ist die Matrize eine solche mit kongruentem doppeltem Kegelstumpf, ist die Länge einer Mantellinie des Kreiszylinders I in der Regel nicht größer als die Summe aus der Länge II und dem 0,7-fachen der Länge I (und dem 0,5-fachen der Länge I). Ist der untere Stempel dabei als ein unterer Einlegestempel ausgestaltet, ist es erfindungsgemäß vorteilhaft, wenn der Querschnitt des Einlegestempels dort, wo er das untere Ende des unteren Stempels hält, im Vergleich zum Querschnitt des Kreiszylinders I, einen kleineren Querschnitt aufweist. Eine derartige Ausgestaltung ermöglicht den Austrag von feinteiligem Korn, das sich zwischen der Wand der Matrizenbohrung und der Mantelfläche des Kreiszylinders I verfangen hat, im Zuge einer Absenkung des unteren Stempels in den Längsabschnitt II* hinein.

Erfindungsgemäß weist der Mittelstift MF von unten nach oben die geometrische Form eines Kreiszylinders Z mit einer kreiszylindrischen Mantelfläche MZ auf.

Des weiteren ist es erfindungsgemäß wesentlich, dass die Position des Mittelstiftes MF und die Position der Matrize einschließlich der Matrizenbohrung während des Verfahrens längs der Bohrachse B relativ zueinander fixiert sind. Die Fixierung der Matrize wird in der Praxis in der Regel so vorgenommen, dass die Matrize passgenau in eine entsprechende Aufnahmeöffnung innerhalb einer Matrizenscheibe eingelegt wird.

Zusätzlich wird sie normalerweise mittels einer Befestigungsschraube, die z. B. vom Matrizenscheibenrand horizontal zur Aufnahmeöffnung für die Matrize führen kann, fixiert. Weist die Matrizenscheibe mehrere, z.B. auf einem Kreisumfang äquidistant platzierte Aufnahmeöffnungen auf, kann die zusätzliche Fixierung von in selbige eingelegten Matrizen auch so erfolgen, dass die Befestigungsschraube auf einem Teilkreis zwischen zwei Aufnahmeöffnungen platziert ist, die die zwei in diese eingelegten Matrizen gegeneinander fixiert.

Zur Fixierung des Mittelstiftes MF werden in der Regel Mittelstifthalter eingesetzt. Um die Fixierung zu erleichtern, ist der Mittelstift MF an seinem unteren Ende normalerweise mit einem Kopf (27) ausgestattet, der von einem passgenau gefertigten Zwischenraum (28) (Schlitz) des Mittelstifthalters aufgenommen wird. Zum vorgenannten Kopf hin kann sich an den eigentlichen Mittelstift ein verbreiterter Querschnitt anschließen, der die Fixierung des Mittelstiftes erleichtert (vgl. z. B. in Figur 6 und in Figur 1). Der Mittelstifthalter selbst wird anwendungstechnisch zweckmäßig in der Regel ebenfalls an der Matrizenscheibe festgeschraubt.

Die vorliegende Erfindung soll jedoch auch diejenige Ausführungsform zur Herstellung ringähnlicher Vorläuferformkörper umfassen, bei der der Mittelstift MF von unten nach oben zunächst die geometrische Form eines Kreiszylinders Z mit einer kreiszylindrischen Mantelfläche MZ aufweist, sich daran anschließend jedoch nach oben hin konisch verjüngt. Dies gilt insbesondere dann, wenn der von unten nach oben zunächst kreiszylindrische Mittelstift MF sich innerhalb des Längsabschnitts II der Matrizenbohrung nach oben hin konisch verjüngt (und sich bis zu seinem oberen Ende nicht mehr erweitert). In diesem Fall kann der Mittelstift MF von unten nach oben die Geometrie eines Kreiszylinders Z aufweisen, dem dann innerhalb des Längsabschnitts II ein sich nach oben verjüngender Kegelstumpf KM (30) aufgesetzt ist (der Querschnitt des Kreiszylinders Z entspricht dabei dem Querschnitt der Grundfläche des Kegelstumpfs KM).

Dabei kann die Höhe des Kegelstumpfs KM der Länge des Längsabschnitts II entsprechen (was erfindungsgemäß bevorzugt ist), aber auch kürzer sein (im letzteren Fall erstreckt sich der eine kreiszylindrische Geometrie aufweisende Anteil des Mittelstiftes von unten nach oben bis in den Längsabschnitt II hinein. Die Vorteilhaftigkeit eines solchen Auslaufens des Mittelstiftes MF nach oben als Kegelstumpf KM liegt, in ähnlicher Weise wie die Vorteilhaftigkeit der Geometrie des Längsabschnitts II der Matrizenbohrung selbst, darin begründet, dass aufgrund der konischen Verjüngung des Mittelstiftes MF nach oben, beim Entfernen des gebildeten ringähnlichen Formkörpers aus der Matrizenbohrung durch Anheben des unteren Stempels die Rollreibung zwischen der Außenwand (der Mantelfläche) des Mittelstiftes MF und der Mantelfläche des Hohlraums des gebildeten ringähnlichen Formkörpers (z. B. über die Länge des Längsabschnitts II) im wesentlichen zum Verschwinden gebracht werden kann (z. B. im Fall der Herstellung von ringähnlichen Vorläuferformkörpern LII bzw. $F^{LII}$). Allerdings ist der aus diesem Sachverhalt resultierende Vorteilsgewinn vergleichsweise beschränkt, da die Mantelfläche des Kegelstumpfs KM im Vergleich zur Mantelfläche des Kegelstumpfs KS bei gleicher Höhe im Normalfall wesentlich kleiner ist.

Ist H* die Höhe des Kegelstumpfs KM, ist es vorteilhaft, wenn die Verjüngung des Kegelstumpfs KM von unten (von der Grundfläche) nach oben (zur Deckfläche) so beschaffen ist, dass zwischen dem Durchmesser DG* der Grundfläche und der Höhe H* sowie dem Durchmesser DD* der Deckfläche des Kegelstumpfes KM die nachfolgende Beziehung erfüllt ist:

$$0{,}005 \bullet H^* \leq DG^* - DD^* \leq 0{,}015 \bullet H^* \qquad \text{(IX)}.$$

Vorzugsweise gilt:

$$0{,}007 \bullet H^* \leq DG^* - DD^* \leq 0{,}013 \bullet H^* \qquad \text{(X)}.$$

Besonders bevorzugt gilt:

$$0{,}009 \bullet H^* \leq DG^* - DD^* \leq 0{,}011 \bullet H^* \qquad \text{(XI)}.$$

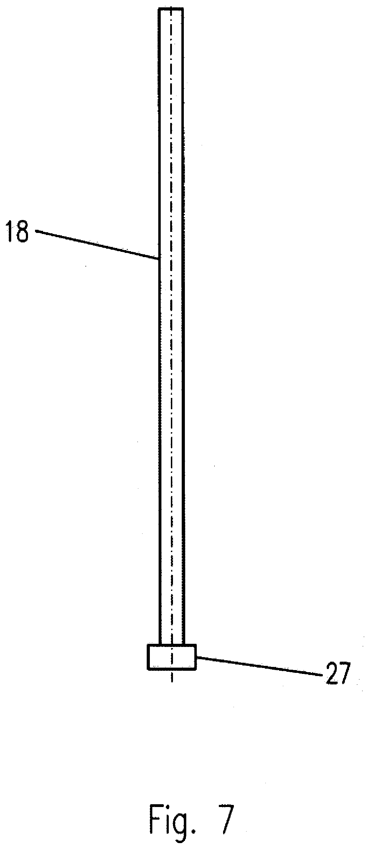

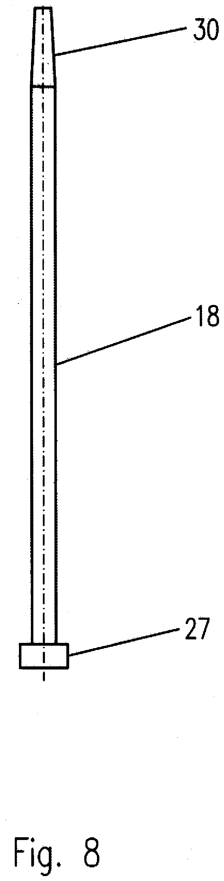

Zu beachten ist bei der Anwendung eines sich z. B. zu seinem oberen Ende hin konisch verjüngenden Mittelstiftes MF, dass aufgrund des über die Höhe H* des Kegelstumpfes KM nicht konstanten Querschnitts bei der Aufnahme des Kegelstumpfs KM in die Mittelbohrung $MB^O$ in notwendiger Weise ein Ringspalt verbleibt (die Mantelfläche des Kegelstumpfs KM nicht auf der Innenwand der Mittelbohrung $MB^O$ gleitet). Die noch tolerable Breite desselben muss sich wieder an der Korngröße des zu verdichtenden pulverförmigen Haufwerks orientieren. Im Normalfall wird man den Querschnitt der Mittelbohrung $MB^O$ im Fall eines sich nach oben konisch verjüngenden Mittelstiftes MF so bemessen, dass er bei einer Aufnahme seines kreiszylindrischen Abschnitts in die Mittelbohrung $MB^O$ mit seiner kreiszylindrischen Mantelfläche wenigstens im Bereich des Eingangs in die Mittelbohrung $MB^O$ auf deren Innenwand gleiten würde. Figur 7 zeigt beispielhaft einen Längsschnitt durch einen von unten nach oben ausschließlich die geometrische Form eines Kreiszylinders Z aufweisenden Mittelstiftes MF, während Figur 8 beispielhaft einen Längsschnitt durch einen solchen Mittelstift MF zeigt, der von unten nach oben zunächst die Geometrie eines Kreiszylinders Z aufweist, und sich anschließend bis zu seinem oberen Ende konisch verjüngt.

Ganz generell ist es beim erfindungsgemäßen Verfahren zur Herstellung ringähnlicher Vorläuferformkörper bevorzugt, wenn das obere Ende des Längsabschnitts II der Matrizenbohrung, die obere (plane) Stirnfläche des Mittelstiftes MF und die obere (plane) Stirnfläche der Matrize miteinander bündig (d. h., nicht überstehend) abschließen.

Dies gilt insbesondere für den Fall einer maschinellen Durchführung des Verfahrens, da vorgenannte Konstellation das maschinelle Einbringen des pulverförmigen Haufwerks in den Füllraum erleichtert (begünstigt).

In der Regel weist die Matrize eine plane obere Stirnfläche auf. Anwendungstechnisch zweckmäßig ist auch die untere Stirnfläche der Matrize plan gestaltet. Vorzugsweise weist die Matrize (sieht man von der Matrizenbohrung ab) die Form eines Kreiszylinders mit einer planen oberen und einer planen unteren Stirnfläche auf. In der äußeren Wand des Kreiszylinders verläuft auf halber Höhe vorteilhaft ein horizontal verlaufender Ring bzw. eine runde Vertiefung. Sie dient der Fixierung der Matrize in der Matrizenscheibe mittels einer oder mehreren Befestigungsschrauben.

Grundsätzlich kann beim erfindungsgemäßen Verfahren die Verringerung des Ausgangsabstands A auf den Endabstand E so erfolgen, dass beide (der untere und der obere Stempel) Stempel aktiv aufeinander zu bewegt werden. Selbstverständlich kann aber auch so vorgegangen werden, dass der untere Stempel seine Position beibehält und nur der obere Stempel bewegt (nach unten abgesenkt) wird.

In der Regel ist es für eine möglichst gleichmäßige Oberflächenhärte des beim erfindungsgemäßen Verfahren resultierenden Komprimats (ringähnlichen Vorläuferformkörpers) vorteilhaft, wenn bei der Verringerung des Ausgangsabstands A auf den Endabstand E Ober- und Unterstempel gemeinsam aktiv aufeinander zu bewegt werden (der obere Stempel wird abgesenkt, der untere Stempel wird angehoben). In diesem Fall wird der erforderliche Kompressionsdruck auf das pulverförmige Haufwerk vom oberen und vom unteren Stempel gleichmäßig ausgeübt, was eine einheitlichere Seitendruckfestigkeit des resultierenden Komprimats über dessen Höhe bedingt.

Auch resultiert dabei ein Vorläuferformkörper mit einer homogeneren Massendichte über seine gesamte Dimension. Letzteres bedingt nach der thermischen Behandlung ein homogeneres Porengefüge und auf letzterem basierend eine verbesserte Katalysatorperformance.

Grundsätzlich kann das erfindungsgemäße Verfahren sowohl manuell als auch maschinell durchgeführt werden. Aus Gründen der Wirtschaftlichkeit ist eine maschinelle Ausübung des erfindungsgemäßen Verfahrens bevorzugt. Im wesentlichen können dazu zwei Maschinentypen verwendet werden, die in der Fachliteratur als "Exzenterpresse" und als "Rundläufer" voneinander unterschieden werden. Bei der Exzenterpresse übt nur der Oberstempel den eigentlichen Kompressionsdruck durch seine Abwärtsbewegung mit Hilfe der Exzenterscheibe aus, wobei der Unterstempel während der Kompression stillsteht und sich lediglich zur abschließenden Ausstoßung des Komprimats (z. B. des ringähnlichen Vorläuferformkörpers) aufwärts bewegt (angehoben wird). Bei der Exzenterpresse steht die Matrize still. Sie ruht in der Matrizenplatte auf dem feststehenden Matrizentisch. Die Matrize kann eine oder (nebeneinander) mehrere Matrizenbohrungen (und damit Matrizenfüllräume) aufweisen. In jeder Matrizenbohrung bewegt sich im Rhythmus der Exzenterscheibe ein aus Oberstempel und Unterstempel bestehendes Stempelpaar. Im Fall einer Herstellung von ringähnlichen Vorläuferformkörpern ist der Mittelstift MF ebenfalls stillstehend durch die Matrizenbohrung und den unteren Stempel geführt und mit einem Mittelstifthalter an der Matrizenplatte befestigt. Je nachdem ob die Matrize eine oder mehrere Matrizenbohrungen (Matrizenfüllräume) aufweist, spricht man von einer einstempligen oder mehrstempligen Matrize. In entsprechender Weise wird zwischen Einfach- und Mehrfachwerkzeugen unterschieden. Das Einfachwerkzeug besteht im Fall einer Herstellung von ringähnlichen Vorläuferformkörpern aus einer Matrize mit einer Matrizenbohrung und einem Mittelstift MF sowie einem Ober- und einem Unterstempel. Ein Mehrfachwerkzeug besteht in entsprechender Weise aus einer Matrize mit zwei oder mehreren Matrizenbohrungen und mit entsprechend vielen Mittelstiften MF sowie Ober- und Unterstempeln. Ob Einfach- oder Mehrfachwerkzeuge verwendet werden, wird im wesentlichen anhand der Größe des z. B. ringähnlichen Vorläuferformkörpers sowie des Pressdrucks, den die Maschine leisten kann, entschieden. Die beim erfindungsgemäßen Verfahren anwendbare Obergrenze liegt im Fall einer Herstellung von ringähnlichen Vorläuferformkörpern bei einem ca. fünfzigfachen Werkzeug. Da bei der Exzenterpresse die Matrize stillsteht, gleiten normalerweise ein Fülltrichter samt Füllschuh, die das erfindungsgemäß zu verdichtende pulverförmige Haufwerk enthalten, auf dem Matrizentisch vorwärts und rückwärts, um eine gleichmäßige Füllung des Füllraums bzw. der Füllräume der Matrize zu erreichen. Füllung des Füllraums, Verdichtung (Kompression) und Auslass des z. B. ringähnlichen Formkörpers erfolgen bei der Exzenterpresse auf diese Weise zeitlich periodisch wiederkehrend nacheinander und entsprechen zusammen jeweils einer vollen Exzenterumdrehung.

Im einfachsten Fall verläuft der Arbeitsgang am Beispiel einer Herstellung von ringähnlichen Vorläuferformkörpern bei der Exzentermaschine daher wie folgt.

Der Unterstempel befindet sich innerhalb der Matrizenbohrung zunächst in seiner Füllstellung. Der Füllschuh gleitet über die Matrize, deren obere plane Stirnfläche mit der oberen planen Stirnfläche des Mittelstiftes MF bündig abschließt, wobei das Füllgut (das pulverförmige Haufwerk) in die Matrizenbohrung und auf die obere Stirnfläche des unteren Stempels gelangt. Beim Zurückgleiten des Füllschuhes bewegt sich der Oberstempel abwärts, bis er mit seiner unteren Stirnfläche das Füllgut berührt. Damit ist das pulverförmige Haufwerk in den Füllraum eingebracht und der Zustand des Ausgangsabstands A erreicht. Durch weitergehende Abwärtsbewegung des Oberstempels (bei feststehendem Unterstempel) wird das Füllgut zum ringähnlichen Vorläuferformkörper verdichtet, bis der Endabstand E samt zugehörigem Pressdruck erreicht ist. Anschließend wird der Oberstempel vom gebildeten ringähnlichen Vorläuferformkörper abgehoben und durch (in der Regel etwas verzögertes) Anheben des unteren Stempels der ringähnliche Vorläuferformkörper aus der Matrizenbohrung entfernt. In der Regel erfolgt das Anheben des unteren Stempels so weit, dass die Unterseite des gebildeten Vorläuferformkörpers das Niveau der Matrizenoberseite knapp erreicht. Während nun die Vorderkante des sich vorwärtsschiebenden Füllschuhes den Vorläuferformkörper von der Matrize schiebt, senkt sich der Unterstempel bereits wieder bis zu seiner Füllstellung, und die Matrizenbohrung wird neu gefüllt.

Beim Rundläufer steht dagegen der Fülltrichter samt Füllschuh fest, und eine Matrizenscheibe, in der die Matrizen ruhen, rotiert, wobei die Matrizenbohrungen unter dem Füllschuh vorbeigeführt werden. Bei einer Umdrehung der Matrizenscheiben werden die einzelnen Matrizen (bzw. deren Matrizenbohrungen) nacheinander gefüllt. Dann werden die Füllungen komprimiert und nachfolgend die resultierenden Komprimate ausgestoßen.

Es wird also eine Matrizenbohrung gefüllt, während eine andere Matrizenfüllung komprimiert und gleichzeitig bei wiederum einer anderen Matrize z. B. ein ringähnlicher Vorläuferformkörper (das Komprimat) ausgestoßen wird. Bei einer Umdrehung der Matrizenscheibe werden so viele z. B. ringähnliche Vorläuferformkörper hergestellt, wie - bei Verwendung von einstempligen Werkzeugen - Werkzeugsätze vorhanden sind. Bei mehrstempligen Werkzeugen ist mit der Anzahl der Bohrungen pro Matrize zu multiplizieren. Während die Exzenterpresse diskontinuierlich arbeitet, arbeiten Rundlaufpressen kontinuierlich. Ferner wird beim Rundläufer der Kompressionsdruck mit Hilfe von Druckrollen vom Ober- und Unterstempel gleichmäßig ausgeübt.

An Rundläufern sind am Markt bezüglich einer Herstellung von ringähnlichen Vorläuferformkörpern zur Durchführung des erfindungsgemäßen Verfahrens Modelle für 10 bis 100 (bzw. 80) Werkzeugsätze verfügbar, wobei jeder Werkzeugsatz normalerweise insgesamt bis zu sechsstempelig sein kann. Während bei einem normalen Rundläufer je Matrizenscheiben-Umdrehung so viele Komprimate gepresst werden, wie Matrizen (mal Bohrungen bei Mehrfachwerkzeug) vorhanden sind, haben sogenannte Doppelrundläufer (sie verfügen über eine besonders hohe Ausstoßleistung) zwei Pressstationen, und es werden - bei einstempeligen Werkzeugsätzen - während einer Matrizenscheiben - Umdrehung zur gleichen Zeit jeweils zwei Matrizen gefüllt, zwei Füllungen komprimiert und z. B. zwei ringähnliche Formkörper ausgestoßen. Beispielsweise können für das erfindungsgemäße Verfahren die Exzenterpressen KS, KIS und K III der Firma Kilian, D-50735 Köln, verwendet werden. Besonders geeignet sind für das erfindungsgemäße Verfahren jedoch Rundläufer der Firma Kilian (z. B. der T-Reihe, der R-Reihe, der S-Reihe und der X-Reihe).

Besonders geeignet ist für das erfindungsgemäße Verfahren der Kilian Doppelrundläufer vom Typ RX 73 sowie der Rundläufer Kilian Synthesis 700-77 A.

Ferner eigenen sich für das erfindungsgemäße Verfahren die Rundläufer der Korsch AG, D-13509 Berlin, wie z. B. die Korsch Rundläufer PH800 und PH865.

Die individuelle Gestaltung des oberen Stempels, des unteren Stempels und der Matrize samt Matrizenbohrung (d. h., des Werkzeugs) sowie gegebenenfalls des Mittelstiftes MF obliegt dabei dem Anwender. Zur Anwendung in einem Kilian Rundläufer kann deren Ausgestaltung in einer für das erfindungsgemäße Verfahren besonders vorteilhaften Weise im Fall einer Herstellung von ringähnlichen Vorläuferformkörpern z. B. wie folgt sein (die numerischen Adressen beziehen sich dabei (wie stets in dieser Schrift) auf die dieser Schrift beiliegenden Figuren).

Die einzelne Matrize (1) ist so gefertigt, dass sie genau in die in einer Matrizenscheibe befindliche Aufnahmeöffnung passt. Sieht man von der Matrizenbohrung (2) ab, weist die Matrize anwendungstechnisch zweckmäßig die Form eines Kreiszylinders mit einer planen oberen und einer planen unteren Stirnfläche auf, in dessen äußerer Wand auf halber Höhe entweder ein horizontal verlaufender Ring oder eine runde Vertiefung (3) ausgefräst ist. Sie dient der Fixierung der Matrize in der Matrizenscheibe (z.B. mittels wenigstens einer Befestigungsschraube, die z. B. vom Matrizenscheibenrand horizontal zur Aufnahmeöffnung für die Matrize führen oder auf einem Teilkreis von einer Matrize zu der zu dieser benachbarten Matrize verlaufen kann). Erfindungsgemäß zweckmäßig ist der (zu dieser Matrize gehörige) obere Stempel als ein oberer Einlegestempel (4), und der (zu dieser Matrize gehörige) untere Stempel als ein unterer Einlegestempel (5) gefertigt. Der untere Einlegestempel (der obere Einlegestempel) kann mittels einer unteren Schraubkappe (6) (mittels einer oberen Schraubkappe (7)), in die der untere Einlegestempel (der obere Einlegestempel) eingelegt werden kann, auf einen unteren Schaft (8) (auf einen oberen Schaft (9)) zentriert aufgeschraubt werden. Der unter Schaft (der obere Schaft) läuft in einen unteren Schaftkopf (10) (läuft in einen oberen Schaftkopf (11)) aus, der in den Führungsschienen des Rundläufers gleitet. Der untere Einlegestempel (der obere Einlegestempel) läuft in den eigentlichen unteren Stempel (12) (in den eigentlichen oberen Stempel (13)) aus (d. h., der erfindungsgemäß relevante Stempel ist in beiden Fällen der Hals, in den der jeweilige Einlegestempel ausläuft).

Der Boden (14) des oberen Einlegestempels (auch "Einsetzstempel" genannt) liegt im eingeschraubten Zustand auf der kreisförmigen Druckfläche (15) des oberen Schaftes auf. Die Gestaltung als Einlegestempel eröffnet bei Verwendung derselben Schafte eine hohe Flexibilität.

Der kreisringförmige Boden (16) des unteren Einsetzstempels liegt im eingeschraubten Zustand auf der kreisringförmigen Druckfläche (17) des unteren Schaftes auf. Die Kreisringöffnung der Druckfläche (17) ist als zylindrischer Hohlraum in den unteren Schaft fortgeführt. Er kann die Fortsetzung des Mittelstiftes MF (18) aufnehmen. Über eine seitliche Öffnung (eine Nut) des unteren Schaftes kann der Mittelstift MF mittels eines Mittelstifthalters (19) positioniert (längs der Bohrachse B relativ zur Matrize und deren Matrizenbohrung fixiert werden) werden.

Der Mittelstifthalter selbst wird mittels einer Schraube an der Matrizenscheibe befestigt. Figur 1 zeigt in einer Art Explosionsdarstellung die vorstehend aufgeführten Einzelelemente im Längsschnitt.

Figur 6 zeigt einen Ausschnitt eines durch die Matrizenscheibe geführten Längsschnitts. Er zeigt die in die Aufnahmeöffnung der Matrizenscheibe (20) eingelegte Matrize (1) sowie den horizontal verlaufenden Ring (3) zu ihrer Fixierung mittels einer Befestigungsschraube. Der Teil der Matrizenscheibe, in dessen Aufnahmeöffnungen die Matrizen (1) eingelegt sind, soll in dieser Schrift auch als Matrizenscheibenzunge (21) bezeichnet werden. Desweiteren zeigt die Figur 6 die in der Matrizenscheibe oberhalb und unterhalb der Aufnahmeöffnung für die Matrize (1) angebrachten Führungsbohrungen (22) für die Schafte (8) und (9). Auf der Innenwand der jeweiligen Führungsbohrung (22) mit seiner Mantelfläche vertikal gleitend, kann der untere Schaft (8) bzw. der obere Schaft (9) angehoben oder abgesenkt werden.

Der Teil der Matrizenscheiben, der die Führungsbohrungen für die oberen Schafte enthält, soll in dieser Schrift auch als Matrizenscheibenstirn (23) bezeichnet werden. Der Teil der Matrizenscheibe, der die Führungsbohrungen für die unteren Schafte enthält, soll in dieser Schrift auch als Matrizenscheibenkinn (24) bezeichnet werden. Der Mittelstifthalter (19) ist in der Figur 6 von unten mit der Matrizenscheibe (20) verschraubt. Der der zu seinem Kopf hin mit einem breiteren Querschnitt auslaufende Mittelstift MF (18) führt vom Mittelstifthalter (19) gehalten durch den unteren Schaft und den unteren Einsetzstempel hindurchgeführt bis zur planen Stirnfläche der Matrize (1), mit der die plane Stirn des Mittelstiftes MF (18) bündig abschließt. Insbesondere wenn Mehrfachwerkzeuge verwendet werden, sollten sich die Schafte in der Führungsbohrung (22) nicht drehen. Dies erreicht man durch Keilnuten im Schaft und Keile längs der Innenwand der Führungsbohrung. Der obere Schaftkopf (11) und der untere Schaftkopf (10) sitzen in einer feststehenden "Oberstempelführungsschiene" bzw. "Unterstempelführungsschiene", die die Figur 6 nicht zeigt. Die eingelegte Matrize (1) ist eine Matrize mit kongruentem doppeltem Kegelstumpf.

Die Arbeitsweise der Rundlaufpresse ist nun schematisch wie folgt (dieses Arbeitsprinzip ist bei allen Rundläufern im wesentlichen gleich).

Die z. B. durch eine Schnecke oder ein Zahnrad angetriebene Matrizenscheibe dreht sich in Horizontalebene um ihre Achse. Die mit ihrem jeweiligen Schaftkopf in feststehenden Führungsschienen (in der Regel Edelstahl- oder Kunststoffschienen) sitzenden Schäfte folgen beim Drehen der Matrizenscheibe in der jeweiligen Führungsschiene gleitend deren Höhenprofil. Der den unteren Stempel tragende untere Schaft gleitet im Rahmen der Drehbewegung der Matrizenscheibe auf seiner Gleitbahn zunächst bis zum Füllschuh, wo er und damit auch der untere Stempel nach unten gezogen wird, so dass sich die obere Stirnfläche des unteren Stempels in der Matrizenbohrung auf ihrer Füllhöhe befindet. Im weiteren Verlauf der Drehbewegung wird der oberhalb der oberen Stirnfläche des unteren Stempels befindliche freie Raum der Matrizenbohrung mit dem erfindungsgemäß zu verdichtenden feinteiligen Haufwerk (dem Füllgut) aus dem Füllschuh heraus gefüllt. Beim Weiterdrehen der Matrizenscheibe wird der untere Schaft und mit ihm der untere Stempel angehoben, so dass sich die obere Stirnfläche des unteren Stempels in der Matrizenbohrung auf ihrer Füllmengenhöhe befindet. Überschüssiges Füllgut wird nach oben gedrückt und im weiteren Verlauf der Drehbewegung abgestrichen. Dann wird der untere Schaft und mit ihm der untere Stempel wieder heruntergezogen, so dass sich die obere Stirnfläche des unteren Stempels in der Matrizenbohrung in derjenigen Höhe befindet, auf die sich der Ausgangsabstand A bezieht (hier auch "Presshöhe" genannt). Während der Füllung schwebt der obere Stempel über dem Füllschuh und gleitet nun entsprechend dem Verlauf der Führungsschiene für den oberen Schaft abwärts, bis er mit seiner unteren Stirnfläche das in der Matrizenbohrung befindliche feinteilige Haufwerk berührt. Damit ist das pulverförmige Haufwerk in den Füllraum eingebracht und der Zustand des Ausgangsabstands A erreicht. Sowohl der obere Schaftkopf als auch der unter Schaftkopf werden im Rahmen der Weiterdrehung der Matrizenscheibe über jeweils eine Druckrolle gefahren, und dadurch sowohl der obere Stempel als auch der unter Stempel gegen das in den Füllraum eingebrachte Füllgut gepresst (der untere Stempel wird angehoben, der obere Stempel weiter abgesenkt), bis der Endabstand E erreicht ist.

Während der Verdichtung zwischen den Druckrollen kann bedarfsgerecht eine Weghaltezeit erreicht werden, während der der Abstand von Ober- und Unterstempel konstant gehalten wird (bei Exzentertablettiermaschinen gibt es keine Zeitspanne, in der der Abstand zwischen Ober- und Unterstempel konstant bleibt; die Verdichtung wird nur durch die Eintauchtiefe des Oberstempels in das pulverförmige Haufwerk bedingt).

Die Weghaltezeit, während der der Verdichtungsdruck annähernd konstant ist, begünstigt zeitabhängige plastische Verformungsvorgänge im zu verdichtenden Haufwerk. Dann wird der obere Schaft bedingt durch den Verlauf seiner Führungsschiene wieder angehoben, um den oberen Stempel vom erzeugten ringähnlichen Formkörper abzuheben. Der untere Schaft und mit ihm der untere Stempel wird durch das Gleiten des unteren Schaftkopfs in der Aushebebahn der Führungsschiene angehoben und der auf seiner oberen Stirnfläche befindliche ringähnliche Vorläuferformkörper aus der Matrizenbohrung herausgeführt, und durch einen Abstreifer abgestreift (das erfindungsgemäße Verfahren ermöglicht in vorteilhafter Weise besonders geringe Auswurfkräfte; in der Regel liegen diese bei Verwendung von frischen Matrizen im Bereich von 0,15 bis 1,5 kN; im Verlauf der weiteren Ausübung des Verfahrens steigt die erforderliche Auswurfkraft in der Regel an; erreicht dieser Anstieg ca. 700 N, wird die Matrize normalerweise gedreht oder ausgetauscht). Über eine Rinne rutscht der ringähnliche Vorläuferformkörper anschließend in einen Lagerbehälter. Im Rahmen der weiteren Drehbewegung der Matrizenscheibe wird der obere Stempel durch das Gleiten des oberen Schaftkopfs in seiner Obergleitbahn bis zu seiner höchsten Stelle geschoben, bis er sich wieder über dem Füllschuh befindet.

Der untere Stempel wird inzwischen durch das Weitergleiten des unteren Schaftkopfes in seiner Untergleitbahn wieder nach unten gezogen (abgesenkt), so dass er sich auf der Untergleitbahn ebenfalls wieder unter dem Füllschuh und seine obere Stirnfläche sich in der Matrizenbohrung wieder auf ihrer Füllhöhe befindet. Anschließend wiederholt sich der beschriebene Vorgang mit der Periodizität der Drehbewegung der Matrizenscheibe.

Es ist vorteilhaft, wenn die Füllung der Matrizenbohrung mit Füllgut bereits während der Absenkung des unteren Stempels auf Füllhöhe erfolgt, damit beim Füllen der Matrizenbohrung nicht zuviel Luft eingeschlossen wird. Im Verlauf einer vollständigen Umdrehung der Matrizenscheibe werden die Schafte aus den Führungsbohrungen niemals vollständig herausgeführt.

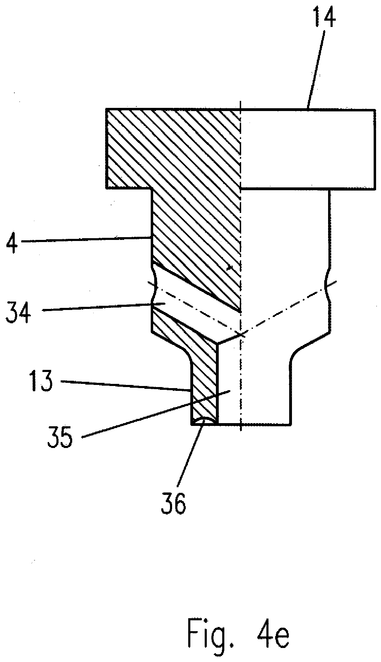

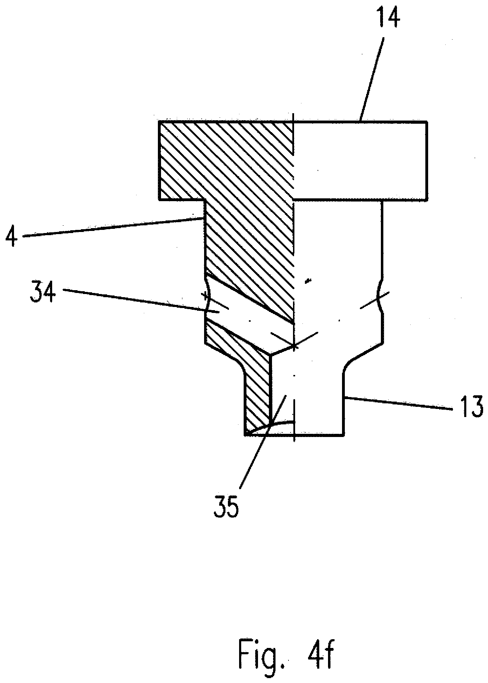

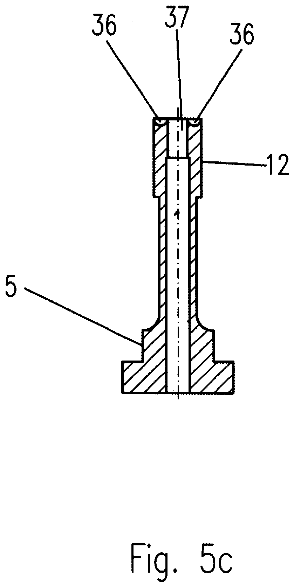

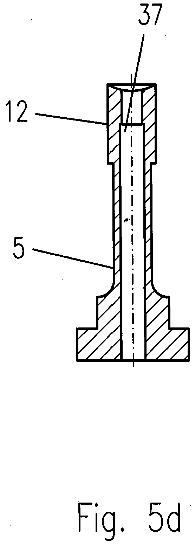

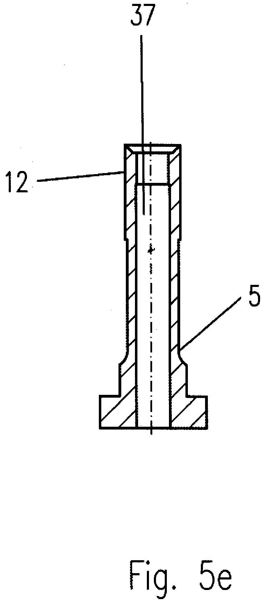

Erfindungsgemäß bevorzugt werden Rundläufer eingesetzt, bei denen nicht wie eben beschrieben durch Verwendung eines Paares von Druckrollen nur ein Verdichtungsvorgang je ringähnlichem Vorläuferformkörper, sondern durch Verwendung zweier dicht hintereinander angeordneter Druckrollenpaare eine Vor- (auf den vorläufigen Endabstand $E^V$ der beiden Stirnflächen) und eine Hauptverdichtung (auf den Endabstand E, wobei $E^V > E$) pro ringähnlichem Vorläuferformkörper durchgeführt wird (die Vordruckrolle ist in der Regel in einfacher Weise kleiner dimensioniert als die Hauptdruckrolle. Die Anwendung einer Vorverdichtung auf einen vorläufigen Endabstand $E^V$ (> E) der beiden Stirnflächen gewährleistet eine bessere Entlüftung beim Verdichten sowie eine gleichmäßigere Verdichtung des pulverförmigen Haufguts, da das Vorverdichten das Füllgut in einen vergleichsweise einheitlichen Ordnungszustand überführt. Generell ist ein langsames Verpressen vorteilhaft für eine gute Entgasung. Die Seitenwanddruckfestigkeit des resultierenden ringähnlichen Vorläuferformkörpers kann auch dadurch verbessert werden, dass man nach der Vorverdichtung zunächst eine Entlastung und sich an diese anschließend erst die Hauptverdichtung durchführt. Der Vollständigkeit halber sind in den Figuren 5c, 5d und 5e drei untere Einlegestempel dargestellt, bei denen die obere Stirnfläche des unteren Stempels nicht plan ist. Der untere Einlegestempel in Figur 5c läuft dabei in einen unteren Stempel aus, in dem die Lehre der EP-A 184790 umgesetzt ist. Die Figuren 4e und 4f zeigen entsprechend ausgestaltete obere Einlegestempel.

Als Füllschuhe kommen für das erfindungsgemäße Verfahren im Fall von Rundläufern z. B. Rüttelfüllschuhe, Vibrationsfüllschuhe und Rührwerkfüllschuhe in Betracht. Besonders bevorzugt werden jedoch Rührflügelfüllschuhe verwendet. Letztere wurden auch in allen Ausführungsbeispielen eingesetzt.

Ferner sei an dieser Stelle noch festgehalten, dass für das erfindungsgemäße Verfahren die Verwendung von solchen einfachen Rundläufern oder Doppelrundläufern besonders vorteilhaft ist, bei denen die Matrizenscheibe (20) auswechselbar gelagert ist. Ein solcher Doppelrundläufer ist z. B. der Doppelrundläufer Synthesis 700 der Firma Kilian. Günstig an diesem Doppelrundläufer ist auch, dass er mit Vorverdichtung und Hauptverdichtung arbeitet. Rundläufermaschinen sind z. B. in den Schriften DE-A 2624853, DE-A 19733969 und DE-A 2435777 beschrieben. Im übrigen sind die Werkzeuge für eine erfindungsgemäße Tablettierung sehr präzise zu fertigen und die diesbezüglich geltenden Normen des jeweiligen Landes (z. B. die DIN ISO 2768) zu erfüllen. Die jeweiligen Oberflächen der Werkzeuge sollten möglichst glatt gefertigt sein.

Insbesondere für den Fall, dass das erfindungsgemäß zu verdichtende pulverförmige Haufwerk wenigstens ein Metalloxid (z. B. aus der Gruppe umfassend Aluminiumoxid, Wolframoxid, Antimonoxid, Zirkoniumoxid, Wismutoxid, Molybdänoxid, Siliciumoxid, Magnesiumoxid sowie Mischoxide, die wenigstens zwei der in den vorgenannten Metalloxiden enthaltenen Metallelemente enthalten (z. B. Mischoxide des Wismuts und des Wolframs wie z. B. $Bi_2W_2O_9$)), ein Metallhydroxid, ein Metallhydrogenphosphat und/oder wenigstens ein Metallnitrat (unter diesem Begriff sollen auch Metallnitrathydrate subsumieren) wie z. B. Kobaltnitrat, Eisennitrat, Wismutnitrat, Nickelnitrat, Cäsiumnitrat, Kupfernitrat, Calciumnitrat und Magnesiumnitrat enthält (derartige pulverförmige Haufwerke sollen im Folgenden als pulverförmige Haufwerke HW* bezeichnet werden), ist es erfindungsgemäß vorteilhaft, wenn der obere Stempel und der untere Stempel für das erfindungsgemäße Verfahren aus dem Werkzeugstahl mit der DIN-Werkstoffnummer 1.2601 gefertigt sind (für den Fall, dass die vorgenannten Stempel als Einlegstempel gefertigt sind, ist der gesamte Einlegestempel anwendungstechnisch zweckmäßig aus dem DIN-Werkstoff 1.2601 gefertigt). Alternativ zum DIN-Werkstoff 1.2601 können die Stempel, insbesondere in den vorgenannten Fällen, auch aus dem DIN-Werkzeugstahl 1.2379 gefertigt sein. Enthält das erfindungsgemäß zu verdichtende pulverförmige Haufwerk Salpetersäure, soll es im Folgenden als pulverförmiges Haufwerk** bezeichnet werden.

Um den in der WO 2005/115733 beschriebenen Problemen Rechnung zu tragen, werden die Mittelstifte MF mit Vorteil aus einem DIN-Werkzeugstahl 1.2343 gefertigt. Dies gilt insbesondere im Fall einer Verdichtung von pulverförmigen Haufwerken HW* und HW** (insbesondere im Fall einer Herstellung von ringähnlichen Vorläuferformkörpern F; vor allem im Fall von ringähnlichen Vorläuferformkörpern $F^{LII}$). Die Matrizenscheibenzunge besteht beim erfindungsgemäßen Verfahren, insbesondere im Fall von pulverförmigem Haufwerk HW* oder HW**, vorteilhaft aus dem DIN Kugelgraphitgrauguß GGG 50 mit einer dünnen Auflage aus dem DIN Werkzeugstahl 1.0425, während die Matrizenscheibenstirn und das Matrizenscheibenkinn anwendungstechnisch zweckmäßig aus dem DIN Werkzeugstahl 1.6850 (nitriert) gefertigt sind. Der untere Schaft, der obere Schaft und die zugehörigen Schaftköpfe können beim erfindungsgemäßen Verfahren ebenso wie die Mittelstifthalter in an sich bekannter Weise z. B. aus dem DIN Werkzeugstahl 1.25550 (gehärtet und angelassen, Rockwellhärtegrad HRC 58 + 2) gefertigt sein.

Die bei der Durchführung des erfindungsgemäßen Verfahrens zur Herstellung ringförmiger oder ringähnlicher Vorläuferformkörper im Endabstand E von beiden Stempeln (bzw. wenigstens vom Oberstempel) in typischer Weise ausgeübten Pressdrucke liegen üblicherweise im Bereich von 50 bis 5000 kg/cm$^2$, vorzugsweise bei 200 bis 3500 kg/cm$^2$, besonders bevorzugt 500 bis 2500 kg/cm$^2$ und besonders bevorzugt 500 bis 1500 kg/cm$^2$. Die Vordrucke (sie werden im vorläufigen Endabstand E$^V$ ausgeübt) betragen typisch 5 - 500 kg/cm$^2$ und die Hauptdrucke liegen meist bei 1000 - 2000 kg/cm$^2$. Je höher der angewandte Hauptdruck ist, als desto vorteilhafter erweist sich das erfindungsgemäße Verfahren zur Herstellung ringförmiger oder ringähnlicher Vorläuferformkörper.

Wie in dieser Schrift bereits mehrfach angesprochen, eignet sich das erfindungsgemäße Verfahren insbesondere zur Herstellung von kreiszylindrischen, kreiszylinderähnlichen, ringförmigen oder ringähnlichen Vorläuferformkörpern, aus denen durch thermische Behandlung kreiszylindrische, kreiszylinderähnliche, ringförmige oder ringähnliche Katalysatoren erhältlich sind (z. B. dann, wenn die katalytisch aktive Komponente des oxidischen geometrischen Katalysatorformkörpers ein Multimetalloxid ist). Derartige erfindungsgemäß erhältliche geometrische (z. B. ringähnliche) Vorläuferformkörper sollen in dieser Schrift auch als geometrische (z. B. ringähnliche) Katalysatorvorläuferformkörper bezeichnet werden.

Dabei kann der oxidische geometrische (z. B. ringähnliche) Katalysatorformkörper im einfachsten Fall nur aus der katalytisch aktiven Komponente (z. B. dem Multimetalloxid) bestehen. Er kann die katalytisch aktive Komponente (z. B. das Multimetalloxid) aber auch mit inertem Material verdünnt enthalten. In beiden Fällen spricht man von geometrischen (z. B. ringähnlichen) Vollkatalysatorformkörpern. Ist die aktive Komponente ein Multimetalloxid soll in dieser Schrift von geometrischen (z. B. ringähnlichen) Multimetalloxidvollkatalysatoren gesprochen werden.

Geometrische (z. B. ringähnliche) Multimetalloxidvollkatalysatoren eignen sich insbesondere für die heterogene Katalyse von partiellen Gasphasenoxidationen (vgl. z.B. DE-A 102005037678, die Deutsche Anmeldung mit dem Aktenzeichen 102007028332.8, die Deutsche Anmeldung mit dem Aktenzeichen 102007025869.2, die Deutsche Anmeldung mit dem Aktenzeichen 102007017080.9 und die Deutsche Anmeldung mit dem Aktenzeichen 102007003778.5) organischer Verbindungen mit molekularem Sauerstoff.

Unter einer vollständigen Oxidation einer organischen Verbindung mit molekularem Sauerstoff soll in dieser Schrift verstanden werden, dass die organische Verbindung unter reaktiver Einwirkung von molekularem Sauerstoff so umgesetzt wird, dass der in der organischen Verbindung insgesamt enthaltene Wasserstoff in Oxide des Wasserstoffs umgewandelt wird. Alle davon verschiedenen exothermen Umsetzungen einer organischen Verbindung unter reaktiver Einwirkung von molekularem Sauerstoff werden hier als Partialoxidationen einer organischen Verbindung zusammengefasst (z.B. einschließlich der Ammoxidationen und Oxychlorierungen, die im gleichzeitigen Beisein von Ammoniak bzw. Chlorwasserstoff durchgeführt werden). Im besonderen sollen in dieser Schrift unter Partialoxidationen solche exothermen Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält, als vor Durchführung der Partialoxidation.

Als Beispiele für heterogen katalysierte partielle Gasphasenoxidationen seien an dieser Stelle beispielhaft diejenige von Propylen zu Acrolein, diejenige von iso-Buten zu Methacrolein, diejenige von Methacrolein zu Methacrylsäure und diejenige von $C_4$-Kohlenwasserstoffen zu Maleinsäureanhydrid genannt. Üblicherweise werden heterogen katalysierte partielle Gasphasenoxidationen in z.B. mit Salzschmelzen gekühlten Rohrbündelreaktoren durchgeführt. Die Katalysatoren befinden sich dabei, gegebenenfalls mit inerten Formkörpern verdünnt, in den vom Reaktionsgasgemisch durchströmten Reaktionsrohren.

Zur Herstellung von geometrischen (z. B. ringähnlichen) Multimetalloxidvollkatalysatoren kann nun so vorgegangen werden, dass man aus Quellen der elementaren Konstituenten des katalytisch aktiven Multimetalloxids sowie nach Bedarf mitzuverwendenden Formgebungshilfsmitteln (z. B. Porosierungsmittel, Gleitmittel und Verstärkungsmittel) ein feinteiliges Haufwerk erzeugt, und aus diesem nach dem erfindungsgemäßen Verfahren zunächst geometrische (z. B. ringähnliche) Multimetalloxid-Vollkatalysatorvorläuferformkörper herstellt. Als Quellen für die elementaren Konstituenten des Multimetalloxids können dabei (bei Normalbedingungen in der Regel im festen Aggregatzustand befindliche) Metalloxide und/oder solche Metallverbindungen verwendet werden, die durch Erhitzen (thermisches Behandeln) in (bei Normalbedingungen in der Regel im festen Aggregatzustand befindliche) Oxide überführbar sind (wenigstens durch thermisches Behandeln in Anwesenheit von gasförmigem molekularem Sauerstoff und/oder von gasförmigen Sauerstoff freisetzenden Komponenten).

Durch nachfolgende thermische Behandlung der geometrischen (z. B. ringähnlichen) Multimetalloxid-Vollkatalysatorvorläuferformkörper (z.B. im Temperaturbereich von 200 bis 800°C, oder 300 bis 600°C) sind dann die geometrischen (z. B. ringähnlichen) Multimetalloxidvollkatalysatoren erhältlich.

Die zur erfindungsgemäßen Herstellung von geometrischen (z. B. ringähnlichen) Multimetalloxid-Vollkatalysatorvorläuferformkörpern zu verwendenden pulverförmigen Haufwerke werden daher in der Regel Haufwerke HW* oder Haufwerke HW'' sein. Alle bezüglich der erfindungsgemäßen Verdichtung von pulverförmigen Haufwerken HW* und HW'' in dieser Schrift gemachten Ausführungen gelten daher in entsprechender Weise. Bei erfindungsgemäß bevorzugten ringähnlichen Multimetalloxid-Vollkatalysatorvorläuferformkörpern handelt es sich in der Regel um ringähnliche Formkörper F, vorzugsweise um ringähnliche Formkörper $F^{LII}$.

Unter anderem eignet sich das erfindungsgemäße Verfahren zur Herstellung der Vorläuferformkörper von solchen geometrischen (z. B. ringähnlichen) Multimetalloxidvollkatalysatoren, die als katalytisch aktive Komponente wenigstens ein Multimetalloxid enthalten, in welchem das Element Mo, oder das Element V, oder das Element P dasjenige von Sauerstoff verschiedene Element ist, das numerisch (molar gerechnet) am häufigsten enthalten ist (Multimetalloxid meint dabei, dass das Oxid wenigstens zwei von Sauerstoff verschiedene Elemente enthält).

Insbesondere eignet sich das erfindungsgemäße Verfahren zur Herstellung der Vorläuferformkörper (insbesondere von ringähnlichen Vorläuferformkörpern F bzw. $F^{LII}$) von solchen geometrischen (z. B. ringähnlichen) Multimetalloxidvollkatalysatoren, die als katalytisch aktive Komponente wenigstens ein Multimetalloxid enthalten, das die Elemente Mo und Fe, oder die Elemente Mo, Fe und Bi, oder die Elemente Mo und V, oder die Elemente Mo, V und P, oder die Elemente V und P enthält (vor allem, wenn gleichzeitig die vorgenannte Häufigkeitsbedingung erfüllt ist). Die ersteren geometrischen (z. B. ringähnlichen) Multimetalloxidvollkatalysatoren in der vorgenannten Reihe eignen sich vor allem für heterogen katalysierte partielle Gasphasenoxidationen von Methanol zu Formaldehyd. Die Zweitgenannten eignen sich vor allem für die heterogen katalysierte partielle Gasphasenoxidation von Propylen zu Acrolein. Die Drittgenannten eignen sich vor allem für heterogen katalysierte partielle Gasphasenoxidationen von Acrolein zu Acrylsäure, die Viertgenannten eignen sich vor allem für die heterogen katalysierte partielle Gasphasenoxidation von Methacrolein zu Methacrylsäure und die Letztgenannten in der vorstehenden Reihe eignen sich vor allem für heterogen katalysierte partielle Gasphasenoxidationen von n-Butan zu Maleinsäureanhydrid.

Zur erfindungsgemäßen Herstellung von ringähnlichen Multimetalloxid-Vollkatalysatorvorläuferformkörpern wird vorzugsweise ein unterer Stempel mit einer planen oberen Stirnfläche und ein oberer Stempel mit einer planen unteren Stirnfläche verwendet (vorzugsweise sind dabei die beiden Stirnflächen zueinander kongruent). Selbstverständlich können ringähnliche Multimetalloxid-Vollkatalysatorvorläuferformkörper aber auch, wie in dieser Schrift beschrieben, mit gekrümmten Stirnflächen hergestellt werden.

Katalytisch aktive Multimetalloxide der vorgenannte Art, einschließlich zur erfindungsgemäßen Herstellung von zugehörigen geometrischen (z. B. ringähnlichen) Multimetalloxid-Vollkatalysatorvorläuferformkörpern verwendbarer pulverförmiger Haufwerke, finden sich unter anderem in den Schriften WO 2005/030393, EP-A 467 144, EP-A 1 060 792, DE-A 198 55 913, WO 01/68245, EP-A 1060792, Research Disclosure RD 2005- 497012, DE-A 102005035978, DE-A 102005037678, WO 03/78059,

WO 03/078310, DE-A 199 22 113, WO 02/24620, WO 02/062737, der Deutschen Anmeldung mit dem Aktenzeichen 102007028332.8, der Deutschen Anmeldung mit dem Aktenzeichen 102007025869.2, der Deutschen Anmeldung mit dem Aktenzeichen 102007017080.9 und US-A 2005/0131253.

Die erfindungsgemäß zu verdichtenden pulverförmigen (Vorläufer)Haufwerke sind dabei in einfachster Weise dadurch erhältlich, dass man von Quellen der elementaren Konstituenten des angestrebten katalytisch aktiven Multimetalloxids ein erfindungsgemäß gefordertes feinteiliges, möglichst inniges, der Stöchiometrie des gewünschten Multimetalloxids entsprechend zusammengesetztes, formbares Gemisch erzeugt, dem noch die bereits erwähnten Formgebungshilfsmittel (einschließlich Verstärkungshilfsmittel) zugesetzt werden können (und/oder von Anfang an eingearbeitet werden können).

Als Quellen für die elementaren Konstituenten des gewünschten Multimetalloxids kommen erfindungsgemäß grundsätzlich solche Metallverbindungen in Betracht, bei denen es sich bereits um Oxide handelt und/oder um solche Metallverbindungen, die durch Erhitzen, wenigstens in Anwesenheit von gasförmigem molekularem Sauerstoff und/oder von gasförmigen Sauerstoff freisetzenden Komponenten, in Oxide überführbar sind. Prinzipiell kann die Sauerstoffquelle auch z. B. in Form eines Peroxids Bestandteil des Vorläufergemisches (des pulverförmigen Haufwerks) selbst sein. Auch kann das pulverförmige (Vorläufer)Haufwerk Verbindungen wie $NH_4OH$, $(NH_4)_2CO_3$, $NH_4NO_3$, Harnstoff, $NH_4CHO_2$, $NH_4CH_3CO_2$, Ammoniumoxalat und/oder organische Komponenten wie z. B. Stearinsäure zugesetzt enthalten, die bei der thermischen Behandlung als Porenbildner zu vollständig gasförmig entweichenden Verbindungen zerfallen und/oder zersetzt werden können.

Das, vorzugsweise innige, Vermischen der Ausgangsverbindungen (Quellen) zur Herstellung des feinteiligen erfindungsgemäß formbaren pulverförmigen (Vorläufer)Haufwerks, kann in trockener oder in nasser Form erfolgen. Erfolgt es in trockener Form, so werden die Ausgangsverbindungen zweckmäßigerweise als feinteilige Pulver (mit einem Partikeldurchmesser $d_{50}$ (Zur Bestimmung von Partikeldurchmesserverteilungen sowie den aus diesen entnommenen Partikeldurchmessern $d_{10}$, $d_{50}$ und $d_{90}$ (allgemein $d_x$) wurde das jeweilige feinteilige Pulver über eine Dispergierrinne in den Trockendispergierer Sympatec RODOS (Sympatec GmbH, System-Partikel-Technik, Am Pulverhaus 1, D-38678 Clausthal-Zellerfeld) geführt und dort mit Druckluft trocken dispergiert und im Freistrahl in die Messzelle geblasen. In dieser wird dann nach ISO 13320 mit dem Laserbeugungsspektrometer Malvern Mastersizer S (Malvern Instruments, Worcestshire WR14 1AT, United Kingdom) die volumenbezogene Partikeldurchmesserverteilung bestimmt. Die als Messergebnis angegebenen Partikeldurchmesser $d_x$ sind dabei so definiert, dass X % des Gesamtpartikelvolumens aus Partikeln mit diesem oder einem kleineren Durchmesser bestehen.) zweckmäßig im Bereich 1 bis 200 $\mu m$, vorzugsweise 2 bis 180 $\mu m$, besonders bevorzugt 3 bis 170 $\mu m$ und ganz besonders bevorzugt 4 bis 160 $\mu m$, oder 5 bis 150 $\mu m$ bzw. 10 bis 150 $\mu m$, oder 15 bis 150 $\mu m$ liegend) eingesetzt. Nach Zusatz der Formgebungshilfsmittel kann anschließend die erfindungsgemäße Formgebung erfolgen. Solche Hilfsmittel können beispielsweise Graphit als Gleitmittel sowie Mikrofasern aus Glas, Asbest, Siliciumcarbid und/oder Kaliumtitanat sein. Ganz generell kann eine Ausgangsverbindung Quelle für mehr als einen elementaren Konstituenten sein.

Anstatt das durch Mischen von pulverförmigen Quellen erzeugte Gemisch als solches unmittelbar zur gewünschten Vorläufergeometrie zu formen, ist es häufig zweckmäßig, als einen ersten Formgebungsschritt zunächst eine Zwischenkompaktierung desselben durchzuführen, um das Pulver zu vergröbern (in der Regel auf Partikeldurchmesser $d_{50}$ von 100 bis 2000 $\mu m$, bevorzugt 150 bis 1500 $\mu m$, besonders bevorzugt 400 bis 1000 $\mu m$).

Dabei kann bereits vor der Zwischenkompaktierung z. B. Graphit als Kompaktierhilfsmittel zugesetzt werden. Anschließend erfolgt mit dem vergröberten Pulver die erfindungsgemäße Formgebung, wobei bei Bedarf zuvor nochmals z. B. feinteiliger Graphit (sowie gegebenenfalls weitere Formgebungshilfsmittel (einschließlich Verstärkungsmittel)) zugegeben werden kann.

Vorzugsweise erfolgt das innige Vermischen der Quellen jedoch in nasser Form. Üblicherweise werden dabei die Ausgangsverbindungen z. B. in Form einer wässrigen (aber auch Flüssigkeiten wie iso-Butanol kommen als Lösungs- und/oder Dispergiermedium in Betracht) Lösung und/oder Suspension miteinander vermischt. Besonders innige formbare Gemische werden dabei dann erhalten, wenn ausschließlich von in gelöster Form vorliegenden Quellen der elementaren Konstituenten ausgegangen wird. Als Lösungsmittel wird bevorzugt Wasser eingesetzt (aber auch Flüssigkeiten wie iso-Butanol kommen als Lösungsmittel in Betracht). Anschließend wird die erhaltene Lösung oder Suspension getrocknet, wobei der Trocknungsprozess vorzugsweise durch Sprühtrocknung mit Austrittstemperaturen von 100 bis 150 °C erfolgt (in manchen Fällen kann die Trocknung aber auch durch Filtration und anschließende Trocknung des Filterkuchens erfolgen). Der Partikeldurchmesser $d_{50}$ des resultierenden Sprühpulvers beträgt in typischer Weise 10 bis 50 $\mu$m. War Wasser die Basis des flüssigen Mediums, wird das resultierende Sprühpulver normalerweise nicht mehr als 20 % seines Gewichtes, vorzugsweise nicht mehr als 15% seines Gewichtes und besonders bevorzugt nicht mehr als 10 Gew.-% seines Gewichtes an Wasser enthalten. Diese Prozentsätze gelten in der Regel auch bei Anwendung anderer flüssiger Lösungs- bzw. Suspendierhilfsmittel. Nach Zusatz (oder auch ohne einen solchen Zusatz) der gewünschten Formgebungshilfsmittel zur jeweiligen pulverförmig gestalteten Trockenmasse kann das pulverförmige Gemisch als feinteiliges Vorläufergemisch (pulverförmiges Haufwerk) erfindungsgemäß zum gewünschten Multimetalloxid-Vollkatalysatorvorläuferformkörper verdichtet (geformt) werden. Die feinteiligen Formgebungshilfsmittel können aber auch bereits vorab in die Sprühmaische (teilweise oder vollständig) zugesetzt werden.

Eine nur teilweise Entfernung des Lösungs- bzw. Suspendiermittels kann auch dann zweckmäßig sein, wenn seine Mitverwendung als Formgebungshilfsmittel beabsichtigt ist.

Vorab einer Zugabe von z. B. feinteiligem Graphit als Gleitmittel kann auch bereits eine erste thermische Behandlung des Trockenpulvers erfolgen. Nach Zugabe des z. B. Graphits erfolgt dann die erfindungsgemäße Formgebung sowie die sich daran anschließende thermische Weiterbehandlung.

Anstatt das auf dem Sprühpulver fußende feinteilige Vorläufergemisch als solches unmittelbar zur gewünschten Geometrie zu formen, ist es häufig zweckmäßig, als einen ersten Formgebungsschritt zunächst eine Zwischenkompaktierung durchzuführen, um das Pulver zu vergröbern (in der Regel auf Partikeldurchmesser von 100 bis 2000 $\mu$m, bevorzugt 150 bis 1500 $\mu$m, besonders bevorzugt 400 bis 1000 $\mu$m).

Dabei kann bereits vor der Zwischenkompaktierung z. B. Graphit als Kompaktierhilfsmittel zugesetzt werden. Anschließend erfolgt auf der Grundlage des vergröberten Pulvers die endgültige (eigentliche), die erfindungsgemäße Formgebung, wobei bei Bedarf zuvor nochmals feinteiliger Graphit (sowie gegebenenfalls weitere Formgebungshilfsmittel wie z. B. Verstärkungsmittel) zugegeben werden kann.

Selbstverständlich können als Quellen der elementaren Konstituenten auch Ausgangsverbindungen eingesetzt werden, die ihrerseits durch thermische Behandlung von Vorläuferverbindungen (Elementquellen) erzeugt wurden, und multimetalloxidischer Natur sind. Insbesondere können die Ausgangsverbindungen der elementaren Konstituenten multimetallischer Natur sein.

Alles bisher in dieser Schrift Gesagte hat vor allem dann Gültigkeit, wenn das katalytisch wirksame (aktive) Multimetalloxid des geometrischen (z. B. ringähnlichen) Multimetalloxid-Vollkatalysators eine Stöchiometrie der allgemeinen Formel XII,

$$Mo_{12}Bi_aFe_bX^1_cX^2_dX^3_eX^4_fO_n \qquad (XII),$$

mit

X$^1$ = Nickel und/oder Kobalt,
X$^2$ = Thallium, Samarium, ein Alkalimetall und/oder ein Erdalkalimetall,
X$^3$ = Zink, Phosphor, Arsen, Bor, Antimon, Zinn, Cer, Blei, Vanadium, Chrom, Niob und/oder Wolfram,
X$^4$ = Silicium, Aluminium, Titan und/oder Zirkonium,

a = 0,2 bis 5,
b = 0,01 bis 5,
c = 0 bis 10,
d = 0 bis 2,
e = 0 bis 8,
f = 0 bis 10, und
n = eine Zahl, die durch die Wertigkeit und Häufigkeit der von Sauerstoff verschiedenen Elemente in XII bestimmt wird,

oder eine Stöchiometrie der allgemeinen Formel XIII,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (XIII),$$

mit

Y$^1$ = nur Wismut oder Wismut und wenigstens eines der Elemente Tellur, Antimon, Zinn und Kupfer,
Y$^2$ = Molybdän oder Wolfram, oder Molybdän und Wolfram,
Y$^3$ = ein Alkalimetall, Thallium und/oder Samarium,
Y$^4$ = ein Erdalkalimetall, Nickel, Kobalt, Kupfer, Mangan, Zink, Zinn, Cadmium und/oder Quecksilber,

24

Y$^5$ = Eisen oder Eisen und wenigstens eines der Elemente Vanadium, Chrom und Cer,

Y$^6$ = Phosphor, Arsen, Bor und/oder Antimon,

Y$^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

Y$^8$ = Molybdän oder Wolfram, oder Molybdän und Wolfram,

a' = 0,01 bis 8,

b' = 0,1 bis 30,

c' = 0 bis 4,

d' = 0 bis 20,

e' > 0 bis 20,

f' = 0 bis 6,

g' = 0 bis 15,

h' = 8 bis 16,

x', y' = Zahlen, die durch die Wertigkeit und Häufigkeit der von Sauerstoff verschiedenen Elemente in XIII bestimmt werden, und

p, q = Zahlen, deren Verhältnis p/q 0,1 bis 10 beträgt,

aufweist.

Solche z. B. ringähnlichen Multimetalloxid-Vollkatalysatoren eignen sich vor allem als Katalysatoren mit erhöhter Selektivität und Aktivität für die gasphasenkatalytische Partialoxidation von Propen zu Acrolein sowie von iso-Buten bzw. tert.-Butanol bzw. dessen Methylether zu Methacrolein.

Zur erfindungsgemäßen Herstellung der zugehörigen z. B. ringähnlichen Multimetalloxid-Vollkatalysatorvorläuferformkörper wird man aus Quellen der elementaren Konstituenten des aktiven Multimetalloxids ein feinteiliges, durch Verdichten formbares erfindungsgemäßes Vorläufergemisch erzeugen (ein pulverförmiges Haufwerk) und dieses Gemisch, nach Zugabe von Formgebungshilfsmittel (vgl. dazu z.B. DE-A 10 2005 037 678, DE-A 10 2007 003 778, DE-A 10 2007 028 332 und den in diesen Schriften zitierten Stand der Technik), das gegebenenfalls auch Verstärkungsmittel umfasst, in erfindungsgemäßer Weise verdichten (vorzugsweise zu ringähnlichen Vollkatalysatorvorläuferformkörpern F bzw. F$^{LII}$).

Die erfindungsgemäße Formgebung erfolgt dabei vorteilhaft so, dass die Seitendruckfestigkeit des resultierenden ringähnlichen Multimetalloxid-Vollkatalysatorvorläuferformkörpers ≥ 10 und ≤ 40 N, besser ≥ 10 und ≤ 35 N, noch besser ≥ 12 N und ≤ 30 N beträgt. Vorzugsweise beträgt die Seitendruckfestigkeit der ringähnlichen Multimetalloxid-Vollkatalysatorvorläuferformkörper ≥ 13 N und ≤ 27 N, bzw. ≥ 14 N und ≤ 25 N. Ganz besonders bevorzugt beträgt die Seitendruckfestigkeit der ringähnlichen Multimetalloxid-Vollkatalysatorvorläuferformkörper ≥ 15 N und ≤ 22 N.

Die Körnung (der Partikeldurchmesser) des erfindungsgemäß zu verdichtenden pulverförmigen Haufwerks (ausgenommen die zuzusetzenden Hilfsmittel) wird dabei vorteilhaft auf 200 bis 1500 μm, besonders vorteilhaft auf 400 bis 1000 μm eingestellt (z. B. durch Zwischenkompaktierung). In günstiger Weise liegen wenigstens 80 Gew.-%, besser wenigstens 90 Gew.-% und besonders vorteilhaft 95 oder 98 oder mehr Gew.-% des pulverförmigen Haufwerks in diesem Körnungsbereich.

Als Seitendruckfestigkeit wird dabei in dieser Schrift die Druckfestigkeit bei Stauchung des ringähnlichen Multimetalloxid-Vollkatalysatorvorläuferformkörpers senkrecht zur Symmetrieachse (d.h., parallel zur Fläche der Öffnung) verstanden. Dabei beziehen sich alle Seitendruckfestigkeiten dieser Schrift auf eine Bestimmung mittels einer Material-Prüfmaschine der Firma Zwick GmbH & Co (D-89079 Ulm) des Typs Z 2.5/TS15. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wird dabei entsprechend der DIN EN ISO 7500-1 kalibriert und ist für den Messbereich 1-500 N einsetzbar (relative Messunsicherheit: ± 0,2 %).

Die Messungen werden mit folgenden Parametern durchgeführt:

Vorkraft: 0,5 N.

Vorkraft-Geschwindigkeit: 10 mm/min.

Prüfgeschwindigkeit: 1,6 mm/min.

Dabei wird der obere Stempel zunächst langsam bis kurz vor die Mantelfläche des ringähnlichen Vollkatalysatorvorläuferformkörpers abgesenkt. Dann wird der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Betreffend die Aktivmassen der Stöchiometrie XII betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient a vorzugsweise 0,4 bis 2. Der stöchiometrische Koeffizient f beträgt vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Besonders bevorzugt weisen alle stöchiometrischen Koeffizienten a bis f und alle Variablen $X^1$ bis $X^4$ gleichzeitig ihre vorgenannten vorteilhaften Bedeutungen auf.

Innerhalb der Stöchiometrien der allgemeinen Formel XIII sind jene bevorzugt, die der allgemeinen Formel XIV

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (XIV),$$

mit

$Z^2$ = Molybdän oder Wolfram, oder Molybdän und Wolfram,
$Z^3$ = Nickel und/oder Kobalt,
$Z^4$ = Thallium, ein Alkalimetall und/oder ein Erdalkalimetall, vorzugsweise K, Cs und/oder Sr,
$Z^5$ = Phosphor, Arsen, Bor, Antimon, Zinn, Cer, Vanadium, Chrom und/oder Bi,
$Z^6$ = Silicium, Aluminium, Titan und/oder Zirkonium, vorzugsweise Si,
$Z^7$ = Kupfer, Silber und/oder Gold,
$Z^8$ = Molybdän oder Wolfram, oder Wolfram und Molybdän,
$a''$ = 0,1 bis 1,
$b''$ = 0,2 bis 2,
$c''$ = 3 bis 10,
$d''$ = 0,02 bis 2,
$e''$ = 0,01 bis 5, vorzugsweise 0,1 bis 3,
$f''$ = 0 bis 5,
$g''$ = 0 bis 10, vorzugsweise > 0 bis 10, besonders bevorzugt 0,2 bis 10 und ganz besonders bevorzugt 0,4 bis 3,
$h''$ = 0 bis 1,
$x'', y''$ = Zahlen, die durch die Wertigkeit und Häufigkeit der von Sauerstoff verschiedenen Elemente in XIV bestimmt werden, und
$p'', q''$ = Zahlen, deren Verhältnis $p'' / q''$ 0,1 bis 5, vorzugsweise 0,5 bis 2 beträgt,

entsprechen.

Ferner sind katalytisch aktive Multimetalloxide der Stöchiometrie XIII bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte katalytisch aktive Multimetalloxide der Stöchiometrie XIII sind solche, in denen $Y^1$ nur Wismut ist.

Innerhalb der katalytisch aktiven Multimetalloxide der Stöchiometrie XIV sind diejenigen erfindungsgemäß bevorzugt, in denen $Z^2_{b''}$ = (Wolfram)$_{b''}$ und $Z^8_{12}$ = (Molybdän)$_{12}$ ist.

Ferner sind katalytisch aktive Multimetalloxide der Stöchiometrie XIV bevorzugt, die dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Bi_{a''}Z^2_{b''}O_{x''}$ enthalten, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Ferner ist es von Vorteil, wenn wenigstens 25 mol-% (bevorzugt wenigstens 50 mol-% und besonders bevorzugt 100 mol-%) des gesamten Anteils $[Y^1_{a'}Y^2_{b'}O_{x'}]_p([Bi_{a''}Z^2_{b''}O_{x''}]_p)$ der wie beschrieben erhältlichen katalytisch aktiven Multimetalloxide der Stöchiometrie XIII (der Stöchiometrie XIV) in den katalytisch aktiven Multimetalloxiden der Stöchiometrie XIII (der Stöchiometrie XIV) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}([Bi_{a''}Z^2_{b''}O_{x''}])$ vorliegen, deren Größtdurchmesser im Bereich 1 nm bis 100 $\mu$m liegt.

Im Fall eines katalytisch aktiven Multimetalloxids einer der Stöchiometrien XII bis XIV kommen für das erfindungsgemäße Verfahren der Herstellung ringähnlicher Vorläuferformkörper als Gleitmittel neben Graphit auch Ruß, Polyethylenglycol, Stearinsäure, Stärke, Polyacrylsäure, Mineral- oder Pflanzenöl, Wasser, Bortrifluorid und/oder Bornitrid in Betracht. Auch Glycerin und Celluloseether können als weitere Gleitmittel eingesetzt werden. Erfindungsgemäß bevorzugt wird Graphit als alleiniges Formgebungshilfsmittel zugesetzt. Bezogen auf die erfindungsgemäß zum geometrischen (vorzugsweise ringähnlichen) Vollkatalysatorvorläuferformkörper zu formende Masse werden insgesamt in der Regel ≤ 15 Gew.-%, meist ≤ 9 Gew.-%, vielfach ≤ 5 Gew.-%, oft ≤ 4 Gew.-% an Graphit zugesetzt. Üblicherweise beträgt die vorgenannte Zusatzmenge ≥ 0,5 Gew.-%, meist ≥ 2,5 Gew.-%. Bevorzugt zugesetzte Graphite sind Asbury 3160 und Asbury 4012 der Firma Asbury Graphite Mills, Inc. New Jersey 08802, USA und Timrex® T44 der Firma Timcal Ltd., 6743 Bodio, Schweiz.

Bei Bedarf können noch feinteilige Verstärkungsmittel wie Mikrofasern aus Glas, Asbest, Siliciumcarbid oder Kaliumtitanat zugesetzt werden.

Die thermische Behandlung von wie eben beschrieben erfindungsgemäß erhältlichen geometrischen (z. B. ringähnlichen) Multimetalloxid-Vollkatalysatorvorläuferformkörpern erfolgt in der Regel bei Temperaturen, die 350 °C überschreiten. Normalerweise wird im Rahmen der thermischen Behandlung die Temperatur von 650 °C jedoch nicht überschritten. Erfindungsgemäß vorteilhaft wird im Rahmen der thermischen Behandlung die Temperatur von 600 °C, bevorzugt die Temperatur von 550 °C und besonders bevorzugt die Temperatur von 510 °C nicht überschritten. Ferner wird im Rahmen der thermischen Behandlung des geometrischen (z. B. ringähnlichen) Vollkatalysatorvorläuferformkörpers vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten. Dabei kann die thermische Behandlung in ihrem zeitlichen Ablauf auch in mehrere Abschnitte gegliedert sein. Beispielsweise kann zunächst eine thermische Behandlung bei einer Temperatur von 150 bis 350 °C, vorzugsweise 220 bis 290 °C, und daran anschließend eine thermische Behandlung bei einer Temperatur von 400 bis 600 °C, vorzugsweise 430 bis 550 °C durchgeführt werden.

Normalerweise nimmt die thermische Behandlung des geometrischen (z. B. ringähnlichen) Multimetalloxid-(XII bis XIV)-Vollkatalysatorvorläuferformkörpers mehrere Stunden (meist mehr als 5 h) in Anspruch. Häufig erstreckt sich die Gesamtdauer der thermischen Behandlung auf mehr als 10 h. Meist werden im Rahmen der thermischen Behandlung des ringähnlichen Vollkatalysatorvorläuferformkörpers Behandlungsdauern von 50 h bzw. 30 h nicht überschritten. Oft liegt die Gesamtbehandlungsdauer unterhalb von 25 h. Erfindungsgemäß vorteilhaft werden im Rahmen der thermischen Behandlung des relevanten geometrischen (z. B. ringähnlichen) Vollkatalysatorvorläuferformkörpers 510°C (470°C) nicht überschritten und die Behandlungsdauer im Temperaturfenster von ≥ 400°C (≥ 440°C) erstreckt sich auf 5 bis 20 h.

Die thermische Behandlung (auch die nachfolgend angesprochene Zersetzungsphase) der vorstehend ausgeführten ringähnlichen Multimetalloxid (XII bis XIV)-Vollkatalysatorvorläuferformkörper kann sowohl unter Inertgas als auch unter einer oxidativen Atmosphäre wie z. B. Luft (Gemisch aus Inertgas und Sauerstoff) sowie auch unter reduzierender Atmosphäre (z.B. Gemisch aus Inertgas, $NH_3$, CO und/oder $H_2$ oder Methan, Acrolein, Methacrolein) erfolgen. Selbstredend kann die thermische Behandlung auch unter Vakuum ausgeführt werden. Selbstverständlich kann im Verlauf einer thermischen Behandlung die Gasatmosphäre auch verändert werden.

Prinzipiell kann die thermische Behandlung von geometrischen (z. B. ringähnlichen) Multimetalloxid-Vollkatalysatorvorläuferformkörpern in den unterschiedlichsten Ofentypen wie z. B. beheizbare Umluftkammern, Hordenöfen, Drehrohröfen, Bandcalcinierern oder Schachtöfen durchgeführt werden. Bevorzugt erfolgt die thermische Behandlung der geometrischen (z. B. ringähnlichen) Multimetalloxid-Vollkatalysatorvorläuferformkörper in einer Bandcalciniervorrichtung, wie sie die DE-A 100 46 957 und die WO 02/24620 empfehlen.

Die thermische Behandlung von geometrischen (z. B. ringähnlichen) Multimetalloxid-Vollkatalysatorvorläuferformkörpern unterhalb von 350 °C verfolgt in der Regel das Ziel der thermischen Zersetzung der in den Vollkatalysatorvorläuferformkörpern enthaltenen Quellen der elementaren Konstituenten des angestrebten ringähnlichen Multimetalloxid-Vollkatalysators. Häufig erfolgt diese Zersetzungsphase im Rahmen des Aufheizens auf Temperaturen ≥ 350 °C.

Insbesondere zur Herstellung von katalytisch aktiven Multimetalloxiden der Stöchiometrie der allgemeinen Formel XIII oder XIV ist es vorteilhaft, ein Mischoxid $Y^1_{a'}Y^2_{b'}O_{x'}$ bzw. $Bi_{a''}Z^2_{b''}O_{x''}$ als Quelle der Elemente $Y^1$, $Y^2$ bzw. Bi, $Z^2$ in Abwesenheit der übrigen Konstituenten des Multimetalloxids vorzubilden und damit nach seiner Vorbildung, wie bereits beschrieben, mit Quellen der übrigen Konstituenten des Multimetalloxids ein feinteiliges formbares Gemisch zu erzeugen, um daraus, nach Zusatz von Formgebungshilfsmittel, den geometrischen (z. B. ringähnlichen) Multimetalloxid-Vollkatalysatorvorläuferformkörper erfindungsgemäß zu formen.

Bei einer solchen Vorgehensweise ist lediglich darauf zu achten, dass für den Fall, dass die Herstellung des feinteiligen formbaren Gemischs nass erfolgt (in Suspension), die vorgebildeten Mischoxide $Y^1_{a'}Y^2_{b'}O_{x'}$ bzw. $Bi_{a''}Z^2_{b''}O_{x''}$ nicht in nennenswertem Umfang in Lösung gehen.

Ausführlich wird eine wie vorstehend beschriebene Herstellweise in den Schriften DE-A 44 07 020, EP-A 835, EP-A 575 897 und DE-C 33 38 380 sowie in der Deutschen Anmeldung mit dem Aktenzeichen 102007003778.5 beschrieben. Beispielsweise kann man wasserlösliche Salze von $Y^1$ wie Nitrate, Carbonate, Hydroxide oder Acetate mit $Y^2$-Säuren oder deren Ammoniumsalzen in Wasser mischen, die Mischung trocknen (vorzugsweise sprühtrocknen) und die getrocknete Masse anschließend thermisch behandeln. Die thermisch behandelte Masse wird nachfolgend zweckmäßig zerkleinert (z. B. in einer Kugelmühle oder durch Strahlmahlen) und aus dem dabei erhältlichen, in der Regel aus im wesentlichen kugelförmigen Partikeln bestehenden, Pulver die Kornklasse mit einem im für das aktive Multimetalloxid der Stöchiometrie der allgemeinen Formel XIII bzw. XIV gewünschten Größtdurchmesserbereich liegenden Korngrößtdurchmesser durch in an sich bekannter Weise durchzuführendes Klassieren (z. B. Nass- oder Trockensiebung) abgetrennt und vorzugsweise mit, bezogen auf die Masse dieser abgetrennten Kornklasse, 0,1 bis 3 Gew.-%, feinteiligem $SiO_2$ (der Partikeldurchmesser $d_{50}$ der üblicherweise im wesentlichen kugelförmigen $SiO_2$-Partikel beträgt zweckmäßigerweise 100 nm bis 15 μm) vermischt und so eine Ausgangsmasse 1 hergestellt. Die thermische Behandlung erfolgt zweckmäßig bei Temperaturen von 400 bis 900 °C, vorzugsweise bei 600 bis 900 °C. Letzteres gilt insbesondere dann, wenn es sich bei dem vorgebildeten Mischoxid um ein solches der Stöchiometrie $BiZ^2O_6$, $Bi_2Z^2_2O_9$ und/oder $Bi_2Z^2_3O_{12}$ handelt, unter denen das $Bi_2Z^2_2O_9$ bevorzugt ist, insbesondere wenn $Z^2$ = Wolfram.

Üblicherweise erfolgt die thermische Behandlung im Luftstrom (z. B. in einem Drehrohrofen, wie er in der DE-A 103 25 487 beschrieben ist). Die Dauer der thermischen Behandlung erstreckt sich in der Regel auf einige Stunden.

Von den übrigen Bestandteilen des gewünschten aktiven Multimetalloxids der allgemeinen Formel XIII bzw. XIV wird normalerweise ausgehend von in an sich bekannter Weise geeigneten Quellen (vgl. EP-A 835 und DE-C 33 38 380 sowie DE-A 44 07 020 und die Deutsche Anmeldung mit dem Aktenzeichen 102007003778.5) in erfindungsgemäß zweckmäßiger Weise z. B. ein möglichst inniges, vorzugsweise feinteiliges Trockengemisch hergestellt (z. B. wasserlösliche Salze wie Halogenide, Nitrate, Acetate, Carbonate oder Hydroxide in einer wässrigen Lösung vereinen und anschließend die wässrige Lösung z. B. sprühtrocknen oder nicht wasserlösliche Salze, z. B. Oxide, in wässrigem Medium suspendieren und anschließend die Suspension z. B. sprühtrocknen), das hier als Ausgangsmasse 2 bezeichnet wird. Wesentlich ist nur, dass es sich bei den Bestandteilen der Ausgangsmasse 2 entweder bereits um Oxide handelt, oder um solche Verbindungen, die durch Erhitzen, gegebenenfalls in Anwesenheit von Sauerstoff und/oder Sauerstoffquellen, in Oxide überführbar sind. Ferner ist darauf zu achten, dass die erfindungsgemäß erforderlichen Nitrat- und/oder Ammoniumsalze enthalten sind. Anschließend werden die Ausgangsmasse 1 und die Ausgangsmasse 2 im gewünschten Mengenverhältnis sowie unter Zusatz von Formgebungshilfsmittel zum zum ringähnlichen Vollkatalysatorvorläuferformkörper formbaren Gemisch vermischt.

Die Formung kann, wie bereits beschrieben, anwendungstechnisch zweckmäßig über die Stufe einer Zwischenkompaktierung erfolgen.

In einer weniger bevorzugten Ausführungsform kann das Vorgebildete Mischoxid $Y^1_{a'}Y^2_{b'}O_{x'}$ bzw. $Bi_{a''}Z^2_{b''}O_{x''}$ mit Quellen der übrigen Bestandteile der gewünschten Aktivmasse auch in flüssigem, vorzugsweise wässrigem, Medium innig vermischt werden. Dieses Gemisch wird anschließend z. B. zu einem innigen Trockengemisch getrocknet und sodann, wie bereits beschrieben, geformt und thermisch behandelt. Dabei können die Quellen der übrigen Konstituenten in diesem flüssigen Medium gelöst und/oder suspendiert vorliegen, wohingegen das Vorgebildete Mischoxid in diesem flüssigen Medium im wesentlichen unlöslich sein sollte, d. h., suspendiert vorliegen muss.

Die vorgebildeten Mischoxidpartikel sind im fertig gestellten geometrischen (z. B. ringförmigen) Vollkatalysator in der durch die Klassierung eingestellten Längstausdehnung im wesentlichen unverändert enthalten. Im Übrigen kann wie in der Deutschen Anmeldung mit dem Aktenzeichen 102007003778.5 ausgeführt verfahren werden. Die in der Deutschen Anmeldung mit dem Aktenzeichen 102007003778.5 bezüglich ringförmiger Multimetalloxid-Vollkatalysatorvorläuferformkörper sowie den aus diesen resultierenden ringförmigen Multimetalloxidvollkatalysatoren gemachten Ausführungen gelten für die ringförmigen Zielgegenstände dieser Anmeldung in entsprechender Weise.

In typischer Weise betragen die Seitendruckfestigkeiten von wie beschrieben erhältlichen ringähnlichen Multimetalloxid (XII bis XIV)- Vollkatalysatoren 5 bis 13 N, häufig 8 bis 11 N.

Wie bereits erwähnt, eignen sich die wie beschrieben erhältlichen geometrischen (vorzugsweise ringähnlichen) Vollkatalysatoren insbesondere als Katalysatoren für die Partialoxidation von Propen zu Acrolein bzw. von iso-Buten und/oder tert. Butanol zu Methacrolein. Die Partialoxidation kann dabei z. B. wie in den Schriften WO 00/53557, WO 00/53558, DE-A 199 10 506, EP-A 1 106 598, WO 01/36364, DE-A 199 27 624, DE-A 199 48 248, DE-A 199 48 523, DE-A 199 48 241, EP-A 700 714,

DE-A 10313213, DE-A 103 13 209, DE-A 102 32 748, DE-A 103 13 208,

WO 03/039744. EP-A 279 374, DE-A 33 38 380, DE-A 33 00 044, EP-A 575 897, DE-A 10 2004 003 212, DE-A 10 2005 013 039, DE-A 10 2005 009 891, der Deutschen Anmeldung mit dem Aktenzeichen 102007003778.5, DE-A 10 2005 010 111, DE-A 10 2005 009 885 sowie DE-A 44 07 020 für ringförmige Vollkatalysatoren beschrieben durchgeführt werden, wobei die Katalysatorbeschickung z. B. nur wie beschrieben erhältliche ringähnliche Vollkatalysatoren oder z. B. mit inerten Formkörpern verdünnte ringähnliche Vollkatalysatoren umfassen kann. Im letzteren Fall wird die Katalysatorbeschickung vorteilhaft in der Regel so gestaltet, dass ihre volumenspezifische Aktivität in Strömungsrichtung des Reaktionsgasgemisches kontinuierlich, sprunghaft und/oder stufenförmig zunimmt.

Für das Verfahren der Propylenpartialoxidation zu Acrolein besonders vorteilhafte Multimetalloxidstöchiometrien sind:

a) $[Bi_2W_2O_9 x 2WO_3]_{0,4}[Mo_{12}Co_{5,5}Fe_{2,94}Si_{1,59}K_{0,08}O_x]_1$;

b) $Mo_{12}Ni_{6,5}Zn_2Fe_2Bi_1P_{0,0065}K_{0,06}O_x \cdot 10SiO_2$;

c) $Mo_{12}CO_7Fe_{2,94}Bi_{0,6}Si_{1,59}K_{0,08}O_x$;

d) wie Multimetalloxid II-Vollkatalysator gemäß Beispiel 1 der DE-A 197 46 210; und

e) wie Beispiel 1c aus der EP-A 015 565.

Das in dieser Schrift Gesagte hat aber auch dann Gültigkeit, wenn das katalytisch wirksame (aktive) Multimetalloxid des ringähnlichen Multimetalloxid-Vollkatalysators eine Stöchiometrie der allgemeinen Formel XV,

$$Mo_{12}P_aV_bX_c^1X_d^2X_e^3Sb_fRe_gS_hO_n \qquad (XV),$$

aufweist, in der Variablen folgende Bedeutung haben:

$X^1 =$ Kalium, Rubidium und/oder Cäsium,

$X^2 =$ Kupfer und/oder Silber,

$X^3 =$ Cer, Bor, Zirkonium, Mangan und/oder Wismut,

a = 0,5 bis 3,

b = 0,01 bis 3,

c = 0,2 bis 3,

d = 0,01 bis 2,

e = 0 bis 2,

f = 0 bis 2, vorzugsweise 0,01 bis 2,

g = 0 bis 1,

h = 0 bis 0,5, vorzugsweise 0,001 bis 0,5, und

n = eine Zahl, die durch die Wertigkeit und Häufigkeit der von Sauerstoff verschiedenen Elemente in XV bestimmt wird.

Bevorzugt sind Multimetalloxide XV, in denen h 0,03 bis 0,5 ist.

Besonders bevorzugte Stöchiometrien der allgemeinen Formel XV sind jene der Ausführungsbeispiele B1 bis B15 aus der EP-A 467 144 und dies auch dann, wenn diese beispielhaften Multimetalloxide kein K und/oder kein Re enthalten.

Vorgenannte EP-A 467 144 und die Deutsche Anmeldung mit dem Aktenzeichen 102007003778.5 beschreiben auch die Herstellung von ringförmigen Multimetalloxid (XV)-Vollkatalysatorformkörpern und deren Verwendung als Katalysatoren für die heterogen katalysierte Gasphasenpartialoxidation von Methacrolein zu Methacrylsäure.

Diese Beschreibungen sind auch im in der vorliegenden Anmeldung gegebenen Kontext z. B. bezüglich der Herstellung entsprechender ringähnlicher Multimetalloxid (XV)-vollkatalysatoren zutreffend.

D.h., ringähnliche Multimetalloxid (XV)-Vollkatalysatorvorläuferformkörper können erfindungsgemäß dadurch hergestellt werden, dass man als Ausgangsverbindungen geeignete Salze der sie konstituierenden elementaren Bestandteile, gegebenenfalls bei erhöhter Temperatur und unter Zusatz von Säuren oder Basen, in wässrigem Medium durch Lösen und/oder Suspendieren fein verteilt und, zur Vermeidung unerwünschter Oxidationsprozesse ggf. unter Inertgas, mischt, das Gemisch trocknet (z. B. eindampft oder sprühtrocknet), der resultierenden, feinteilige Form aufweisenden oder in feinteilige Form überführten Trockenmasse z. B. Graphit als Gleitmittel sowie gegebenenfalls sonstige der bereits genannten Formgebungshilfsmittel zusetzt, und die dabei erhaltene feinteilige Masse, die erfindungsgemäß wenigstens ein Nitratsalz und/oder Ammoniumsalz enthalten muss, erfindungsgemäß zur gewünschten ringähnlichen Geometrie formt (verdichtet). Die dabei resultierenden Katalysatorvorläuferformkörper werden anschließend zur Überführung in die aktiven ringähnlichen Katalysatorformkörper thermisch behandelt. Bevorzugt wird die thermische Behandlung bei Temperaturen von 180 bis 480°C, besonders bevorzugt bei Temperaturen von 250 bis 450 °C durchgeführt. Die thermische Behandlung kann dabei unter den bereits beschriebenen Gasatmosphären erfolgen. Beispielhaft erwähnt seien nochmals strömende Luft, strömende Inertgasatmosphäre (z. B. $N_2$, oder $CO_2$, oder Edelgase) oder Vakuum. Die thermische Behandlung kann in mehreren Temperaturstufen und/oder in verschiedenen Atmosphären durchgeführt werden. So kann z. B. in einer ersten Stufe bei 200 bis 260 °C in Luft, in einer zweiten Stufe bei 420 bis 460 °C in Stickstoff und in einer dritten Stufe bei 350 bis 410 °C wiederum in Luft thermisch behandelt werden. Im Regelfall ist strömende Luft die bevorzugte Atmosphäre für die thermische Behandlung.

Im Übrigen gilt das in dieser Schrift bei der Herstellung von ringähnlichen Vollkatalysatorformkörpern von Multimetalloxiden XII bis XIV Gesagte hier in entsprechender Weise, jedoch mit dem Unterschied, dass hier die erhöhten Seitendruckfestigkeiten für die ringförmigen Vollkatalysatorvorläuferformkörper bevorzugt werden.

Ebenso gelten die in der Deutschen Anmeldung mit dem Aktenzeichen 102007003778.5 bezüglich der Herstellung von ringförmigen Vollkatalysatorformkörpern von Multimetalloxiden XV gemachten Aussagen hier in entsprechender Weise.

D. h., z. B. bevorzugtes Trocknungsverfahren für die wässrige Lösung oder Suspension der Quellen der elementaren Konstituenten des gewünschten aktiven Multimetalloxids XV ist die Sprühtrocknung. Das resultierende Sprühpulver mit einem Partikeldurchmesser $d_{50}$ zwischen 10 bis 50 $\mu$m wird vorteilhaft nach Zusatz von feinteiligem Graphit als Hilfsmittel zwischenkompaktiert, um das Pulver zu vergröbern. Bevorzugt erfolgt die Zwischenkompaktierung hier auf Partikeldurchmesser von 100 bis 2000 $\mu$m, bevorzugt von 150 bis 1500 $\mu$m und besonders bevorzugt von 400 bis 1000 $\mu$m. Anschließend erfolgt auf der Grundlage des vergröberten Pulvers die erfindungsgemäße Formgebung, wobei bei Bedarf zuvor nochmals feinteiliger Graphit (sowie gegebenenfalls weitere Formgebungshilfsmittel) zugegeben werden kann.

Bei der beschriebenen Herstellungsweise von ringähnlichen oder anderen geometrischen Vollkatalysatorformkörpern von aktiven Multimetalloxiden der allgemeinen Formel XV wird Antimon üblicherweise als Antimontrioxid, Rhenium z. B. als Rhenium(VII)oxid, Molybdän vorzugsweise als Ammoniumsalz der Molybdän- oder Phosphormolybdänsäure, Bor z. B. als Borsäure, Vanadin in der Regel als Ammoniumvanadat oder Vanadinoxalat, Phosphor mit Vorteil als ortho-Phosphorsäure oder Di-Ammonium-Phosphat, Schwefel z.B. als Ammoniumsulfat und die kationischen Metalle normalerweise als Nitrate, Oxide, Hydroxide, Carbonate, Chloride, Formiate, Oxalate und/oder Acetate bzw. deren Hydrate eingesetzt.

Das erfindungsgemäße Verfahren eignet sich ferner zur Herstellung von z. B. ringähnlichen Multimetalloxid-Vollkatalysatorvorläuferformkörpern mit gekrümmter und/oder nicht gekrümmter Stirnfläche von ringähnlichen Multimetalloxidvollkatalysatoren, deren aktives Multimetalloxid ein Vanadium, Phosphor und Sauerstoff enthaltendes Multimetalloxid ist, und die sich als Katalysatoren für die heterogen katalysierte Gasphasenoxidation wenigstens eines Kohlenwasserstoffs mit mindestens vier Kohlenstoffatomen (insbesondere n-Butan, n-Butene und/oder Benzol) zu Maleinsäureanhydrid eignen. Die Stöchiometrie des aktiven Multimetalloxids kann dabei z. B. eine solche der allgemeinen Formel XVI sein,

$$V_1 P_b Fe_c X^1_d X^2_e O_n \qquad (XVI),$$

in der die Variablen folgende Bedeutung aufweisen:

$X^1 =$      Mo, Bi, Co, Ni, Si, Zn, Hf, Zr, Ti, Cr, Mn, Cu, B, Sn und/oder Nb,
$X^2 =$      K, Na, Rb, Cs und/oder Tl,
$b =$      0,9 bis 1,5,
$c =$      0 bis 0,1,
$d =$      0 bis 0,1,
$e =$      0 bis 0,1, und
$n =$      eine Zahl, die durch die Wertigkeit und Häufigkeit der von Sauerstoff verschiedenen Elemente in XVI bestimmt wird.

Zur Herstellung von diesbezüglich geeigneten und erfindungsgemäß zu z. B. ringähnlichen Vorläuferformkörpern (insbesondere F bzw. $F^{LII}$) zu verdichtenden pulverförmigen Haufwerken sei an dieser Stelle auf die WO 03/078310 und Deutschen Anmeldung mit dem Aktenzeichen 102007003778.5, die WO 01/68245 und die DE-A 10 2005 035 978 verwiesen, die die Herstellung entsprechender ringförmiger Systeme betreffen.

Beispielsweise kann wie folgt vorgegangen werden:

a) Umsetzung einer fünfwertigen Vanadiumverbindung (z. B. $V_2O_5$) mit einem organischen, reduzierenden Lösungsmittel (z. B. iso-Butanol) in Gegenwart einer fünfwertigen Phosphorverbindung (z. B. Ortho- und/oder Pyrophosphorsäure) unter Erwärmen auf 75 bis 205 °C, bevorzugt auf 100 bis 120 °C;
b) Abkühlen des Reaktionsgemisches auf vorteilhaft 40 bis 90 °C;
c) Zugabe von Eisen(III)phosphat;
d) Erneutes Erwärmen auf 75 bis 205°C, bevorzugt 100 bis 120 °C;
e) Isolierung der gebildeten festen Vanadium-, Phosphor-, Eisen- und Sauerstoff enthaltenden Vorläufermasse (z. B. durch Filtrieren);
f) Trocknung und/oder thermische Vorbehandlung der Vorläufermasse (gegebenenfalls bis zu beginnender Vorformierung durch Wasserabspaltung aus der Vorläufermasse);
g) Zugabe von feinteiligem Graphit und feinteiligem Ammoniumnitrat und anschließend erfindungsgemäße Formgebung zum ringähnlichen Multimetalloxid-Vollkatalysatorvorläuferformkörper;

daran anschließend thermische Behandlung der gebildeten Katalysatorvorläuferformkörper durch Erhitzen in einer Atmosphäre, welche Sauerstoff, Stickstoff, Edelgase, Kohlendioxid, Kohlenmonoxid und/oder Wasserdampf enthält (z. B. wie in der WO 03078310 auf Seite 20, Zeile 16 bis Seite 21, Zeile 35 beschrieben).

Das erfindungsgemäße Verfahren umfasst weiterhin Verfahren zur Herstellung von z. B. ringähnlichen Multimetalloxid-Vollkatalysatorvorläuferformkörpern mit gekrümmter und/oder nicht gekrümmter Stirnfläche von ringähnlichen MultimetalloxidVollkatalysatoren, deren aktives Multimetalloxid ein Mo, V und wenigstens eines der Elemente Te und Sb enthaltendes Multimetalloxid ist, wie es z.B. die Schriften EP-A 962 253, DE-A 101 22 027, EP-A 608 838, DE-A 198 35 247, EP-A 895 809, EP-A 1 254 709, EP-A 1 192 987, EP-A 1 262 235, EP-A 1 193 240, JP-A 11-343261, JP-A 11-343262, EP-A 1 090 684, EP-A 1 301 457, EP-A 1 254 707, EP-A 1 335 793, DE-A 100 46 672, DE-A 100 34 825, EP-A 1 556 337, DE-A 100 33 121, WO 01/98246 und EP-A 1 558 569 beschreiben, mit der Maßgabe, dass das erfindungsgemäß zu verdichtende pulverförmige Haufwerk wenigstens ein Nitratsalz und/oder ein Ammoniumsalz enthält.

Häufig enthalten die vorgenannten Mo, V und wenigstens eines der Elemente Te und Sb enthaltenden Multimetalloxide noch das Element Nb. Die vorgenannten resultierenden ringähnlichen Multimetalloxidvollkatalysatoren eignen sich für alle in den vorgenannten Schriften aufgeführten heterogen katalysierten Gasphasenreaktionen (insbesondere partiellen Oxidationen). Dies sind im besonderen die heterogen katalysierte partielle Gasphasenoxidation von Propan zu Acrylsäure sowie von Acrolein zu Acrylsäure, von Methacrolein zu Methacrylsäure und von iso-Butan zu Methacrylsäure.

Das erfindungsgemäße Verfahren eignet sich aber auch, wie in dieser Schrift bereits mehrfach angesprochen, zur Herstellung von z. B. ringähnlichen (oder sonstigen geometrischen) Vorläuferformkörpern (z. B. von ringähnlichen Vorläuferformkörpern F oder von ringähnlichen Vorläuferformkörpern $F^{LII}$), aus denen durch thermische Behandlung ringähnliche (oxidische) oder sonstige geometrische Trägerformkörper erhältlich sind, die z. B. zur Herstellung ringähnlicher Schalenkatalysatoren oder zur Herstellung ringähnlicher Tränkkatalysatoren verwendet werden können. Derartige ringähnliche Trägerformkörper können aber selbstredend auch als inerte Formkörper zum Verdünnen eines Katalysatorfestbetts verwendet werden.

Zur erfindungsgemäßen Herstellung solcher z. B. ringähnlicher Träger-Vorläuferformkörper wird als erfindungsgemäß zu verdichtendes pulverförmiges Haufwerk in der Regel ein solches verwendet, das aus (unter Normalbedingungen üblicherweise festen) Metalloxiden und/oder aus solchen Metallverbindungen (z. B. Salzen) besteht, die durch Erhitzen (thermisches Behandeln) in (unter Normalbedingungen üblicherweise feste) Oxide überführbar sind (wenigstens durch thermisches Behandeln in Anwesenheit von gasförmigem molekularem Sauerstoff und/oder von gasförmigen Sauerstoff freisetzenden Komponenten). Zusätzlich kann das pulverförmige Haufwerk die in dieser Schrift bereits erwähnten Formgebungshilfsmittel wie z. B. Gleitmittel, Porosierungsmittel und Verstärkungsmittel zugesetzt enthalten. Erfindungsgemäß wesentlich ist auch hier, dass das erfindungsgemäß zu verdichtende pulverförmige Haufwerk wenigstens ein Nitratsalz und/oder wenigstens ein Ammoniumsalz enthält.

Die zur erfindungsgemäßen Herstellung von z. B. ringähnlichen Träger-Vorläuferformkörpern zu verwendenden pulverförmigen Haufwerke werden daher in der Regel ebenfalls Haufwerk HW* oder Haufwerke HW** sein. Alle bezüglich der erfindungsgemäßen Verdichtung von pulverförmigen Haufwerken HW* und HW** in dieser Schrift gemachten Ausführungen gelten daher in entsprechender Weise. Bei erfindungsgemäß bevorzugten ringähnlichen Träger-Vorläuferformkörpern handelt es sich in der Regel um ringähnliche Vorläuferformkörper F, vorzugsweise um ringähnliche Vorläuferformkörper $F^{LII}$.

Die thermische Behandlung der geometrischen (z. B. ringähnlichen) Träger-Vorläuferformkörper zur Überführung derselben in den geometrischen (z. B. ringähnlichen) Träger erfolgt in der Regel bei Temperaturen ≥ 500 °C, häufig ≥ 600 °C und vielfach ≥ 700 °C. In der Regel wird die vorgenannte thermische Behandlung jedoch bei Temperaturen ≤ 1500 °C durchgeführt. Die thermische Behandlung kann dabei sowohl unter Inertgas als auch unter einer oxidativen Atmosphäre wie z. B. Luft (Gemisch aus Inertgas und Sauerstoff) erfolgen. Selbstredend kann die thermische Behandlung auch unter Vakuum oder reduzierender Atmosphäre erfolgen.

Üblicherweise erfolgt die thermische Behandlung unter oxidierender Atmosphäre (im allgemeinen unter Luft).

Von den katalytisch aktiven Multimetalloxiden unterscheiden sich die Trägeroxide normalerweise dadurch, dass die thermische Behandlung zu ihrer Erzeugung bei wesentlich höheren Temperaturen und/oder über eine längere Zeitdauer erfolgt (wodurch sie häufig weitgehend unporös sind) und/oder dass das in ihnen numerisch (molar gerechnet) am häufigsten enthaltene, von Sauerstoff verschiedene, Element kein Übergangsmetall der 5. bis 11. Nebengruppe (das sind die Vanadiumgruppe, die Chromgruppe, die Mangangruppe, die Eisengruppe, die Kobaltgruppe und die Nickelgruppe) und nicht Phosphor ist. Vielfach ist das in ihnen numerisch (molar gerechnet) am häufigsten enthaltene, von Sauerstoff verschiedene, Metall ein Element aus der Gruppe bestehend aus Erdalkali (z.B. Mg, Ca), Zn, Zr, Al, Si und Ti.

Die bei der Herstellung der z. B. ringähnlichen Träger-Vorläuferformkörper mitverwendeten organischen (einschließlich Graphit) Formgebungshilfsmittel zersetzen sich bei der vorgenannten thermischen Behandlung in der Regel zu gasförmig entweichenden Verbindungen (und/oder setzen sich zu gasförmig entweichenden chemischen Verbindungen um). Häufig besteht der z. B. ringähnliche Trägerformkörper aus einem keramischen Werkstoff. Beispielhaft genannt seien Silikatkeramiken und sonstige Metalloxidkeramiken. In dem entsprechender Weise enthält das zur Herstellung eines ringähnlichen Träger-Vorläuferformkörpers erfindungsgemäß zu verdichtende pulverförmige Haufwerk als mineralische Ausgangsrohstoffe vielfach pulverförmige Silikate wie z. B. Zirkonsilikat, Aluminiumsilikat (z. B. Mullit), Magnesium-Silikat (z. B. Steatit) und sonstige pulverförmige Metalloxide wie z. B. Aluminiumoxid, Magnesiumoxid und Zirkonoxid.

Beispielhaft näher ausgeführt sei an dieser Stelle die Herstellung der in der WO 99/48606 in Ringform ausgeführten Trägerformkörper, die sich zur Herstellung von Trägerkatalysatoren für die Umsetzung von Ethylen und Chlorwasserstoff im Beisein von molekularem Sauerstoff zu 1,2-Dichlorethan eignen ("Oxychlorierung").

Als feinteiliger mineralischer Rohstoff wird ein feinteiliges Gemisch von Pseudoböhmit und $\gamma$-$Al_2O_3$ im Gewichtsverhältnis 4:1 bis 1:4, vorzugsweise 1:1 bis 1:3 verwendet. Diesem Gemisch, dessen $d_{50}$ Partikeldurchmesser anwendungstechnisch zweckmäßig 10 bis 100 $\mu$m beträgt, werden, bezogen auf sein Gewicht, 0,5 bis 7 Gew.-% (vorzugsweise 2 bis 5 Gew.-%) Magnesiumstearat sowie 0,5 bis 3 Gew.-% (vorzugsweise 1 bis 1,5 Gew.-%) feinteiliger Graphit ($d_{50}$ Partikeldurchmesser 15 bis 30 $\mu$m) und 1 bis 2 Gew.-% Ammoniumnitrat als Formgebungshilfsmittel zugesetzt.

Das dabei resultierende pulverförmige Haufwerk wird anschließend in erfindungsgemäßer Weise zu den z. B. ringähnlichen Träger-Vorläuferformkörpern verdichtet (der Lehre der EP-A 184790 folgend ist die obere Stirnfläche des unteren Stempels und die untere Stirnfläche des oberen Stempels dabei vorteilhaft konkav gestaltet (d. h., sie weisen vorzugsweise eine kreisförmige Rille auf)). Diese werden danach in oxidierender Atmosphäre (vorzugsweise unter Luft) bei Temperaturen von 500 bis 800 °C, vorzugsweise 700 bis 750 °C thermisch behandelt (in der Regel 0,5 bis 10 h).

Der so erhaltene ringähnliche Trägerformkörper wird anschließend mit einer wässrigen $CuCl_2$/KCl-Lösung getränkt. Nach der Tränkung werden die ringähnlichen Formkörper zu den aktiven ringähnlichen Katalysatoren getrocknet (in der Regel bei Temperaturen von 80 bis 300 °C, vorzugsweise 100 bis 200 °C). Die Trocknung erfolgt normalerweise an Luft.

Typische Trocknungsdauern betragen 0,2 bis 10 h, im Bereich erhöhter Temperaturen 0,5 bis 2 h. Die Konzentration und das Volumen der Tränklösung werden bei der Tränkung anwendungstechnisch zweckmäßig so gewählt, dass die resultierenden Trägerkatalysatoren einen Cu-Gehalt von 1 bis 15 Gew.-%, vorzugsweise von 2 bis 10 Gew.-%, und einen K-Gehalt von 0,1 bis 8 Gew.-%, vorzugsweise von 0,3 bis 3 Gew.-% aufweisen. Im übrigen kann wie in der WO 99/48606 beschrieben verfahren werden.

Das in dieser Schrift Gesagte hat aber auch dann Gültigkeit, wenn das katalytisch wirksame (aktive) Multimetalloxid des ringähnlichen Multimetalloxid-Vollkatalysators die Stöchiometrie $(Fe_2O_3)_1 \cdot (MoO_3)_{5,25}$ aufweist. Als Ausgangsverbindungen zur Herstellung derselben eignen sich z. B. Eisen-(III)-nitrat und Molybdäntrioxid. Besonders bevorzugte Fe-Quelle ist Eisen-(III)-nitrat-nonahydrat-Schmelze gemäß der Lehre der PCT/EP2008/050341. Bevorzugt werden beide in wässriger ammoniakalischer Lösung miteinander vermischt. Selbige wird anschließend sprühgetrocknet und das resultierende Sprühpulver in erfindungsgemäßer Weise zu vorzugsweise ringähnlichen Vorläuferformkörpern verdichtet. Der Endabstand E ist dabei bevorzugt 5 mm, die Länge der Umrisslinie des Kreiszylinders Z ist vorzugsweise 2·$\pi$ mm ($\pi$ ist dabei das Verhältnis von Kreisumfang zu Kreisdurchmesser) und der Durchmesser DD der Deckfläche des Kegelstumpfes KS ca. 5 mm. Abschließend werden die ringähnlichen Vorläuferformkörper im Temperaturbereich von 400 bis 500 °C unter Luft thermisch behandelt. Die resultierenden ringähnlichen Multimetalloxid-Vollkatalysatorformkörper eignen sich z. B. als Katalysatoren für die Partialoxidation von Methanol zu Formaldehyd.

Das erfindungsgemäße Verfahren ist dann besonders vorteilhaft, wenn der im erfindungsgemäß zu verdichtenden pulverförmigen Haufwerk insgesamt enthalte Gewichtsanteil an Nitrationen und an Ammoniumionen $\geq$ 0,1 Gew.-%, oder $\geq$ 0,2 Gew.-%, oder $\geq$ 0,5 Gew.-%, oder $\geq$ 0,75 Gew.-%, oder $\geq$ 1,0 Gew.-%, oder $\geq$ 1,5 Gew.-%, oder $\geq$ 2 Gew.-%, oder $\geq$ 3 Gew.-%, oder $\geq$ 5 Gew.-%, oder $\geq$ 10 Gew.-% beträgt.

Damit umfasst die vorliegende Patentanmeldung insbesondere die nachfolgenden erfindungsgemäßen Ausführungsformen:

1. Verfahren zur Herstellung eines oxidischen geometrischen Formkörpers, umfassend das mechanische Verdichten eines in den Füllraum einer Matrize eingebrachten pulverförmigen Haufwerks, aus Bestandteilen, die wenigstens eine Metallverbindung, die durch thermische Behandlung bei einer Temperatur $\geq$ 100 °C in ein Metalloxid überführbar ist, oder wenigstens ein Metalloxid, oder wenigstens ein Metalloxid und wenigstens eine solche Metallverbindung mit der Maßgabe umfassen, dass wenigstens ein Bestandteil des pulverförmigen Haufwerks ein Nitratsalz, ein Ammoniumsalz oder Ammoniumnitrat ist, zu einem geometrischen Vorläuferformkörper, bei dem sich der Füllraum in einer mit einer vertikalen Bohrachse B von oben nach unten durch das Matrizenmaterial hindurchgeführten Martrizenbohrung befindet und wenigstens durch

- die Innenwand der Matrizenbohrung,
- die obere Stirnfläche eines von unten entlang der Bohrachse B in die Matrizenbohrung hub- und senkbeweglich eingeführten unterem Stempels, auf der das in den Füllraum eingebrachte pulverförmige Haufwerk aufliegt, und
- die längs der Bohrachse B in einem axialen Ausgangsabstand A oberhalb der oberen Stirnfläche des unteren Stempels befindliche unter Stirnfläche eines entlang der Bohrachse B hub- und senkbeweglich angebrachten oberen Stempels, dessen untere Stirnfläche das in den Füllraum eingebrachte pulverförmige Haufwerk von oben berührt, begrenzt wird,

indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt und nach beendeter Verdichtung der obere Stempel vom gebildeten geometrischen Vorläuferformkörper abgehoben und der geometrische Vorläuferformkörper durch Anheben des unteren Stempels aus der Matrizenbohrung entfernt wird,

sowie ein sich daran anschließendes Verfahren der thermischen Behandlung des geometrischen Vorläuferformkörpers bei einer Temperatur ≥ 100 °C, bei dem sich wenigstens eine Teilmenge seiner Bestandteile unter Ausbildung wenigstens einer gasförmigen Verbindung zersetzt und/oder chemisch umsetzt und der oxidische geometrische Formkörper sich ausbildet,

dadurch gekennzeichnet, dass das die Matrizenbohrung berührende Matrizenmaterial ein Hartmetall ist, das zu ≥ 80 Gew.-% aus dem Hartstoff Wolframcarbid und zu wenigstens 5 Gew.-% aus dem metallischen Bindemittel Nickel besteht (d.h., ≥ 80 Gew.-% des Hartmetalls Wolframcarbid und ≥ 5 Gew.-% des metallischen Bindemittels Ni enthält).

2. Verfahren gemäß Ausführungsform 1, dadurch gekennzeichnet, dass das Hartmetall neben dem Hartstoff Wolframcarbid zusätzlich wenigstens einen weiteren Hartstoff aus der Gruppe bestehend aus Metallnitriden, Metallboriden und von Wolframcarbid verschiedenen Metallcarbiden enthält.

3. Verfahren gemäß Ausführungsform 2, dadurch gekennzeichnet, dass der metallische Anteil des wenigstens einen weiteren Hartstoffs wenigstens ein Metall aus der Gruppe bestehend aus Ti, Zr, Hf, V, Nb, Ta, Mo, Cr und W ist.

4. Verfahren gemäß einer der Ausführungsformen 1 bis 3, dadurch gekennzeichnet, dass das Hartmetall als metallisches Bindemittel entweder nur Nickel oder neben Nickel noch wenigstens ein metallisches Bindemittel aus der Gruppe bestehend aus Fe, Co und Cr enthält.

5. Verfahren gemäß Ausführungsform 4, dadurch gekennzeichnet, dass der auf das metallische Bindemittel Ni im Hartmetall entfallende Gewichtsanteil größer ist, als der auf jedes andere im Hartmetall enthaltene metallische Bindemittel entfallende Gewichtsanteil.

6. Verfahren gemäß einer der Ausführungsformen 1 bis 5, dadurch gekennzeichnet, dass das Hartmetall neben dem Hartstoff Wolframcarbid zusätzlich wenigstens einen weiteren Hartstoff aus der Gruppe bestehend aus TiC, TaC, NbC, VC, $Cr_3C_2$ und Mischmetallcarbide, die wenigstens zwei der in den vorgenannten Metallcarbiden enthaltenen Metalle enthalten, enthält.

7. Verfahren gemäß einer der Ausführungsformen 1 bis 6, dadurch gekennzeichnet, dass das Hartmetall zu ≥ 7 Gew.-% aus dem metallischen Bindemittel Nickel besteht.

8. Verfahren gemäß einer der Ausführungsformen 1 bis 7, dadurch gekennzeichnet, dass das Hartmetall zu ≥ 85 Gew.-% aus Wolframcarbid besteht.

9. Verfahren gemäß einer der Ausführungsformen 1 bis 7, dadurch gekennzeichnet, dass das Hartmetall zu ≥ 90 Gew.-% aus Wolframcarbid besteht.

10. Verfahren gemäß einer der Ausführungsformen 1 bis 9, dadurch gekennzeichnet, dass das Hartmetall zu 90 bis 95 Gew.-% aus Wolframcarbid, zu ≥ 0 bis 1 Gew.-% aus wenigstens einem Metallcarbid aus der Gruppe bestehend aus TiC, TaC, NbC, VC, $Cr_3C_2$ und Mischmetallcarbiden, die wenigstens zwei der in den vorgenannten Metallcarbiden enthaltenen Metalle enthalten, sowie bis zu 10 Gew.-% aus den metallischen Bindemitteln Ni, Fe, Co und/oder Cr mit der Maßgabe besteht, dass der Gewichtsanteil des Ni am Hartmetall ≥ 5 Gew.-% beträgt.

11. Verfahren gemäß Ausführungsform 10, dadurch gekennzeichnet, dass das Hartmetall als metallisches Bindemittel nur Nickel oder nur Nickel und Chrom enthält.

12. Verfahren gemäß einer der Ausführungsformen 1 bis 11, dadurch gekennzeichnet, dass die Korngröße der Hartstoffe im Hartmetall im Bereich 0,5 $\mu$m bis 2 $\mu$m liegt.

13. Verfahren gemäß einer der Ausführungsformen 1 bis 12, dadurch gekennzeichnet, dass die Matrize ausschließlich aus dem Hartmetall besteht.

14. Verfahren nach einer der Ausführungsformen 1 bis 12, dadurch gekennzeichnet, dass die Matrize nur auf ihrer die Matrizenbohrung berührenden Seite aus dem Hartmetall und auf ihrer von der Matrizenbohrung abgewandten Seite aus einem Werkzeugstahl der Elementzusammensetzung

| 1,50 bis 1,80 Gew.-% | C, |
| 0,10 bis 0,40 Gew.-% | Si, |
| 0,10 bis 0,50 Gew.-% | Mn, |

(fortgesetzt)

| ≥ 0 bis 0,05 Gew.-% | P, |
| ≥ 0 bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| ≥ 0 bis 0,60 Gew.-% | W, und |
| ≥ 0 bis 0,10 Gew.-% | eines oder mehrere seltene Erdmetalle, und im übrigen Fe und herstellungsbedingte Verunreinigungen, |

besteht.

15. Verfahren gemäß einer der Ausführungsformen 1 bis 14, dadurch gekennzeichnet, dass das pulverförmige Haufwerk wenigstens ein Nitratsalz aus der Gruppe bestehend aus Kobaltnitrat, Eisennitrat, Wismutnitrat, Nickelnitrat, Cäsiumnitrat, Kupfernitrat, Calciumnitrat, Magensiumnitrat und den Hydraten der vorgenannten Nitrate, enthält.

16. Verfahren gemäß einer der Ausführungsformen 1 bis 15, dadurch gekennzeichnet, dass das pulverförmige Haufwerk wenigstens ein Ammoniumsalz aus der Gruppe bestehend aus $NH_4HCO_3$, $NH_4OH$, $(NH_4)_2CO_3$, $NH_4HSO_4$, $(NH_4)_2SO_4$, $NH_4CHO_2$, $NH_4CH_3CO_2$, Ammoniumoxalat und die Hydrate der vorgenannten Ammoniumsalze enthält.

17. Verfahren gemäß einer der Ausführungsformen 1 bis 16, dadurch gekennzeichnet, dass sich bei der thermischen Behandlung der geometrischen Vorläuferformkörper wenigstens eine gasförmige Verbindung aus der Gruppe bestehend aus Ammoniak, Wasserdampf, $CO_2$, CO und Stickoxiden bildet.

18. Verfahren gemäß einer der Ausführungsformen 1 bis 17, dadurch gekennzeichnet, dass mit der thermischen Behandlung der Vorläuferformkörper ein Gewichtsverlust einhergeht, der, auf deren Ausgangsgewicht vor deren thermischen Behandlung bezogen, 0,5 bis 40 Gew.-% beträgt.

19. Verfahren gemäß einer der Ausführungsformen 1 bis 18, dadurch gekennzeichnet, dass der Endabstand E 2 bis 10 mm beträgt.

20. Verfahren gemäß einer der Ausführungsformen 1 bis 18, dadurch gekennzeichnet, dass der Endabstand E 2 bis 8 mm beträgt.

21. Verfahren gemäß einer der Ausführungsformen 1 bis 20, dadurch gekennzeichnet, dass der Mittenrauhwert $R_a$ der Innenwand der Matrizenbohrung ≤ 0,1 μm beträgt.

22. Verfahren gemäß einer der Ausführungsformen 1 bis 21, dadurch gekennzeichnet, dass im Endabstand E von beiden Stempeln ein Pressdruck ausgeübt wird, der im Bereich 50 bis 5000 $kg/cm^2$ liegt.

23. Verfahren gemäß einer der Ausführungsformen 1 bis 22, dadurch gekennzeichnet, dass im Endabstand E von beiden Stempeln ein Pressdruck ausgeübt wird, der im Bereich 500 bis 2500 $kg/cm^2$ liegt.

24. Verfahren gemäß einer der Ausführungsformen 1 bis 23, dadurch gekennzeichnet, dass das Verfahren der thermischen Behandlung der hergestellten Vorläuferformkörper bei einer Temperatur ≥ 200 °C erfolgt.

25. Verfahren gemäß einer der Ausführungsformen 1 bis 24, dadurch gekennzeichnet, dass das Verfahren der thermischen Behandlung der hergestellten Vorläuferformkörper bei einer Temperatur ≥ 300 °C erfolgt.

26. Verfahren gemäß einer der Ausführungsformen 1 bis 25, dadurch gekennzeichnet, dass das pulverförmige Haufwerk Graphit, Stärke, gemahlene Nussschale, feinteiliges Kunststoffgranulat, Cellulose, Stearinsäure, Malonsäure, Salz der Stearinsäure und/oder Salz der Malonsäure zugesetzt enthält.

27. Verfahren gemäß einer der Ausführungsformen 1 bis 26, dadurch gekennzeichnet, dass der in dem mechanisch zu verdichtenden pulverförmigen Haufwerk insgesamt enthaltene Gewichtsanteil an Nitrationen und Ammoniumionen ≥ 0,1 Gew.-% beträgt.

28. Verfahren gemäß einer der Ausführungsformen 1 bis 26, dadurch gekennzeichnet, dass der in dem mechanisch zu verdichtenden pulverförmigen Haufwerk insgesamt enthaltene Gewichtsanteil an Nitrationen und Ammoniumionen ≥ 0,2 Gew.-% beträgt.

29. Verfahren gemäß einer der Ausführungsformen 1 bis 28, dadurch gekennzeichnet, dass bei dem anschließenden Verfahren der thermischen Behandlung der hergestellten geometrischen Vorläuferformkörper in selbigen ein Multimetalloxid ausgebildet wird, das die Elemente Mo und Fe, oder die Elemente Mo, Fe und Bi, oder die Elemente Mo und V, oder die Elemente Mo, V und P, oder die Elemente V und P enthält.

30. Verfahren gemäß einer der Ausführungsformen 1 bis 29, dadurch gekennzeichnet, dass bei dem anschließenden Verfahren der thermischen Behandlung der hergestellten geometrischen Vorläuferformkörper in selbigen ein Multimetalloxid ausgebildet wird, in welchem das Element Mo, oder das Element V, oder das Element P dasjenige von Sauerstoff verschiedene Element ist, das molar gerechnet das numerisch am häufigsten enthaltene ist.

31. Verfahren gemäß einer der Ausführungsformen 1 bis 30, dadurch gekennzeichnet, dass bei dem anschließenden Verfahren der thermischen Behandlung der hergestellten geometrischen Vorläuferformkörper in selbigen ein Multimetalloxid der allgemeinen Formel XII,

$$Mo_{12}Bi_aFe_bX^1{}_cX^2{}_dX^3{}_eX^4{}_fO_n \qquad (XII),$$

mit

$X^1$ = Nickel und/oder Kobalt,
$X^2$ = Thallium, Samarium, ein Alkalimetall und/oder ein Erdalkalimetall,
$X^3$ = Zink, Phosphor, Arsen, Bor, Antimon, Zinn, Cer, Blei, Vanadium, Chrom, Niob und/oder Wolfram,
$X^4$ = Silicium, Aluminium, Titan und/oder Zirkonium,
a = 0,2 bis 5,
b = 0,01 bis 5,
c = 0 bis 10,
d = 0 bis 2,
e = 0 bis 8,
f = 0 bis 10, und
n = eine Zahl, die durch die Wertigkeit und Häufigkeit der von Sauerstoff verschiedenen Elemente in XII bestimmt wird,

ausgebildet wird.

32. Verfahren gemäß einer der Ausführungsformen 1 bis 30, dadurch gekennzeichnet, dass bei dem anschließenden Verfahren der thermischen Behandlung der hergestellten geometrischen Vorläuferformkörper in selbiglen ein Multimetalloxid der allgemeinen Formel XIII,

$$[Y^1{}_{a'}Y^2{}_{b'}O_{x'}]_p[Y^3{}_{c'}Y^4{}_{d'}Y^5{}_{e'}Y^6{}_{f'}Y^7{}_{g'}Y^8{}_{h'}O_{y'}]_q \qquad (XIII),$$

mit

$Y^1$ = nur Wismut oder Wismut und wenigstens eines der Elemente Tellur, Antimon, Zinn und Kupfer,
$Y^2$ = Molybdän oder Wolfram, oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Vanadium, Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
$Y^8$ = Molybdän oder Wolfram, oder Molybdän und Wolfram,
a' = 0,01 bis 8,
b' = 0,1 bis 30,
c' = 0 bis 4,

d' = 0 bis 20,
e' > 0 bis 20,
f' = 0 bis 6,
g' = 0 bis 15,
h' = 8 bis 16,

x', y' = Zahlen, die durch die Wertigkeit und Häufigkeit der von Sauerstoff verschiedenen Elemente in XIII bestimmt werden, und
p, q = Zahlen, deren Verhältnis p/q 0,1 bis 10 beträgt,

ausgebildet wird.

33. Verfahren gemäß einer der Ausführungsformen 1 bis 30, dadurch gekennzeichnet, dass bei dem anschließenden Verfahren der thermischen Behandlung der hergestellten geometrischen Vorläuferformkörper in selbigen ein Multimetalloxid der allgemeinen Formel XIV,

$$[Bi_{a''}Z^2{}_{b''}O_{x''}]_{p}[Z^8{}_{12}Z^3{}_{c''}Z^4{}_{d''}Fe_{e''}Z^5{}_{f''}Z^6{}_{g''}Z^7{}_{h''}O_{y''}]_{q''} \qquad (XIV),$$

mit

| | | |
|---|---|---|
| $Z^2$ = | Molybdän oder Wolfram, oder Molybdän und Wolfram, |
| $Z^3$ = | Nickel und/oder Kobalt, |
| $Z^4$ = | Thallium, ein Alkalimetall und/oder ein Erdalkalimetall, |
| $Z^5$ = | Phosphor, Arsen, Bor, Antimon, Zinn, Cer, Vanadium, Chrom und/oder |
| Bi, $Z^6$ = | Silicium, Aluminium, Titan und/oder Zirkonium, |
| $Z^7$ = | Kupfer, Silber und/oder Gold, |
| $Z^8$ = | Molybdän oder Wolfram, oder Wolfram und Molybdän |
| a" = | 0,1 bis 1, |
| b" = | 0,2 bis 2, |
| c" = | 3 bis 10, |
| d" = | 0,02 bis 2, |
| e" = | 0,01 bis 5, |
| f" = | 0 bis 5, |
| g" = | 0 bis 10, |
| h" = | 0 bis 1, |
| x", y" = | Zahlen, die durch die Wertigkeit und Häufigkeit der von Sauerstoff verschiedenen Elemente in XIV bestimmt werden, und |
| p", q" = | Zahlen, deren Verhältnis p" / q" 0,1 bis 5 beträgt, |

ausgebildet wird.

34. Verfahren gemäß einer der Ausführungsformen 1 bis 30, dadurch gekennzeichnet, dass bei dem anschließenden Verfahren der thermischen Behandlung der hergestellten geometrischen Vorläuferformkörper in selbigen ein Multimetalloxid der allgemeinen Formel XV,

$$Mo_{12}P_aV_bX^1{}_cX^2{}_dX^3{}_eSb_fRe_gS_hO_n \qquad (XV),$$

mit

| | | |
|---|---|---|
| $X^1$ = | Kalium, Rubidium und/oder Cäsium, |
| $X^2$ = | Kupfer und/oder Silber, |
| $X^3$ = | Cer, Bor, Zirkonium, Mangan und/oder Wismut, |
| a = | 0,5 bis 3, |
| b = | 0,01 bis 3, |
| c = | 0,2 bis 3, |
| d = | 0,01 bis 2, |
| e = | 0 bis 2, |
| f = | 0 bis 2, |
| g = | 0 bis 1, |
| h = | 0 bis 0,5, und |
| n = | eine Zahl, die durch die Wertigkeit und Häufigkeit der von Sauerstoff verschiedenen Elemente in XV bestimmt wird, |

ausgebildet wird.

35. Verfahren gemäß einer der Ausführungsformen 1 bis 30, dadurch gekennzeichnet, dass bei dem anschließenden Verfahren der thermischen Behandlung der hergestellten geometrischen Vorläuferformkörper in selbigen ein Multimetalloxid der allgemeinen Formel XVI,

$$V_1P_bFe_cX^1{}_dX^2{}_eO_n \qquad (XVI),$$

mit

$X^1 =$ Mo, Bi, Co, Ni, Si, Zn, Hf, Zr, Ti, Cr, Mn, Cu, B, Sn und/oder Nb,

$X^2 =$ Li, K, Na, Rb, Cs und/oder Tl,

b = 0,9 bis 1,5,

c = 0 bis 0,1,

d = 0 bis 0,1,

e = 0 bis 0,1, und

n = eine Zahl, die durch die Wertigkeit und Häufigkeit der von Sauerstoff verschiedenen Elemente in XVI,

ausgebildet wird.

36. Verfahren gemäß einer der Ausführungsformen 1 bis 28, dadurch gekennzeichnet, dass bei dem anschließenden Verfahren der thermischen Behandlung der hergestellten geometrischen Vorläuferformkörper in selbigen ein bei Normalbedingungen festes Oxid ausgebildet wird, in welchem kein Übergangsmetall der 5. bis 11. Nebengruppe und auch nicht Phosphor dasjenige von Sauerstoff verschiedene Element ist, das molar gerechnet das numerisch am häufigsten enthaltene ist.

37. Verfahren gemäß einer der Ausführungsformen 1 bis 28, dadurch gekennzeichnet, dass das pulverförmige Haufwerk wenigstens ein Metalloxid aus der Gruppe bestehend aus Aluminiumoxid, Wolframoxid, Antimonoxid, Zirkoniumoxid, Wismutoxid, Molybdänoxid, Siliciumoxid, Magnesiumoxid und Mischoxiden, die wenigstens zwei der in den vorgenannten Metalloxiden enthaltenen Metallelemente enthalten, enthält.

38. Oxidischer geometrischer Formkörper, erhältlich nach einem Verfahren gemäß einer der Ausführungsformen 1 bis 37.

39. Verfahren der heterogen katalysierten partiellen Gasphasenoxidation wenigstens einer organischen Verbindung an einem Katalysatorfestbett, dadurch gekennzeichnet, dass das Katalysatorfestbett einen geometrischen oxidischen Formkörper gemäß Ausführungsform 38 enthält.

40. Verfahren gemäß der Ausführungsform 39, dadurch gekennzeichnet, dass die heterogen katalysierte partielle Gasphasenoxidation diejenige

> a) von Propylen zu Acrolein und/oder Acrylsäure
> oder
> b) von Acrolein zu Acrylsäure,
> oder
> c) von Methacrolein zu Methacrylsäure,
> oder
> d) von iso-Buten zu Methacrolein und/oder Methacrylsäure,
> oder
> e) von Propan zu Acrolein und/oder Acrylsäure,
> oder
> f) von iso-Butan zu Methacrolein und/oder Methacrylsäure,
> oder
> g) von wenigstens einem $C_4$-Kohlenwasserstoff und/oder Benzol zu Maleinsäureanhydrid,
> oder
> h) von Methanol zu Formaldehyd
> oder
> i) die Oxichlorierung von Ethylen zu 1,2-Dichlorethan
> ist.

41. Rohrbündelreaktor, dessen Reaktionsrohre wenigstens einen oxidischen geometrischen Formkörper gemäß Ausführungsform 38 enthalten.

Beispiele und Vergleichsbeispiele

I. Herstellung von ringähnlichen Mulitmetalloxid-Vollkatalysatorvorläuferformkörpern, wobei das aktive Multimetalloxid die Stöchiometrie

$$[Bi_2W_2O_9 \cdot 2WO_3]_{0,40}[Mo_{12}Co_{5,4}Fe_{3,1}Si_{1,5}K_{0,08}O_x]_1 \text{ aufweist}$$

1. Herstellung einer Ausgangsmasse 1

In 780 kg einer 25 °C aufweisenden wässrigen salpetersauren Wismutnitratlösung (11,2 Gew.-% Bi; freie Salpetersäure 3 bis 5 Gew.-%; Massendichte: 1,22 bis 1,27 g/ml, hergestellt mit Salpetersäure aus Wismutmetall der Firma Sidech S.A., 1495 Tilly, Belgien, Reinheit: > 99,997 Gew.-% Bi, < 7 mg/kg Pb, je < 5 mg/kg Ni, Ag, Fe, je < 3 mg/kg Cu, Sb und < 1 mg/kg Cd, Zn) wurden bei 25°C innerhalb von 20 min portionsweise 214,7 kg einer 25 °C aufweisenden Wolframsäure (74,1 Gew.-% W, H.C. Starck, D-38615 Goslar, Reinheit > 99,9 Gew.-% $WO_3$ nach Glühen bei 750 °C, 0,4 $\mu$m < $d_{50}$ < 0,8 $\mu$m) eingerührt (70 U/min oder "70 UPM"). Das resultierende wässrige Gemisch wurde anschließend noch 3 h bei 25°C gerührt und dann sprühgetrocknet.

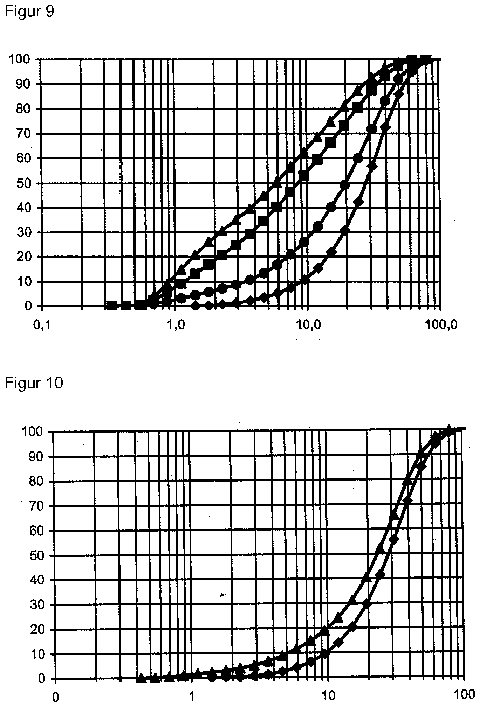

Die Sprühtrocknung erfolgte in einem Drehscheibensprühturm im Heißluftgleichstrom bei einer Gaseintritts-temperatur von 300 $\pm$ 10 °C, einer Gasaustrittstemperatur von 100 $\pm$ 10°C, einer Scheibendrehzahl von 18000 U/min und einem Durchsatz von 200 l/h. Das resultierende Sprühpulver wies einen Glühverlust von 12,8 Gew.-% (3 h bei 600°C im Porzellantiegel (der bei 900 °C bis zur Gewichtskonstanz geglüht worden war) unter Luft glühen) und (bei einem Dispergierdruck von 1,1 bar absolut) einen $d_{50}$ von 28,0 $\mu$m ($d_{10}$ = 9,1 $\mu$m , $d_{90}$ = 55,2 $\mu$m) auf. Figur 9 zeigt die Partikeldurchmesserverteilung des resultierenden Sprühpulvers in Abhängigkeit vom angewendeten Dispergierdruck.

Die Abszisse zeigt die Partikeldurchmesser in logarithmischer Auftragung in $\mu$m.

Die Ordinate zeigt den Volumenanteil in % des Gesamtpartikelvolumens, der den entsprechenden Partikeldurchmesser aufweist in Abhängigkeit vom angewandten Dispergierdruck:

| | | |
|---|---|---|
| ▲ | : | Dispergierdruck = 2 bar abs.. |
| ■ | : | Dispergierdruck = 1,5 bar abs |
| ● | : | Dispergierdruck = 1,2 bar abs |
| ♦ | : | Dispergierdruck = 1,1 bar abs |

Die nachfolgende Tabelle gibt einen Überblick über repräsentative dx-Werte in Abhängigkeit vom angewandten absoluten Dispergierdruck:

| | 2 bar | 1,5 bar | 1,2 bar | 1,1 bar |
|---|---|---|---|---|
| $d_{10}$ ($\mu$m) | 0,91 | 1,17 | 3,4 | 9,1 |
| $d_{50}$ ($\mu$m) | 5,8 | 8,5 | 19,7 | 28,0 |
| $d_{90}$ ($\mu$m) | 27,5 | 34,3 | 47,2 | 55,2 |

Das erhaltene Sprühpulver wurde anschließend mit 16,7 Gew.-% (bezogen auf das Pulver) an 25 °C aufweisendem Wasser in einem Kneter (20 U/min) für 30 min angeteigt und mittels eines Extruders (Drehmoment: $\leq$ 50 Nm) zu Strängen des Durchmessers 6 mm extrudiert. Diese wurden in Abschnitte von 6 cm geschnitten, auf einem 3-zonigen Bandtrockner bei einer Verweilzeit von 120 min je Zone bei Temperaturen von 90-95°C (Zone 1), 115 °C (Zone 2) und 125 °C (Zone 3) an Luft getrocknet und dann bei einer Temperatur im Bereich um 830°C thermisch behandelt (calciniert; im luftdurchströmten Drehrohrofen (0,3 mbar Unterdruck, 1,54 m$^3$ Innenvolumen, 200 Nm$^3$/h Luft, 50 kg/h Extrudat, Drehzahl: 1 U/min, bei 4 m Länge des Drehrohres 7 cm Neigung)). Wesentlich bei der genauen Einstellung der Calcinationstemperatur ist, dass sie an der angestrebten Phasenzusammensetzung des Calcinationsprodukts orientiert zu erfolgen hat. Gewünscht sind die Phasen $WO_3$ (monoklin) und $Bi_2W_2O_9$ (orthorhombisch), unerwünscht ist das Vorhan-densein von $\gamma$-$Bi_2WO_6$ (Russellit). Sollte daher nach der Calcination die Verbindung $\gamma$-$Bi_2WO_6$ anhand eines Reflexes im Röntgenpulverdiffraktogramm bei einem Reflexwinkel von $2\Theta$ = 28,4° (CuK$\alpha$-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde mit einer Biplexmühle BQ500 mit 2500 U/min gemahlen, so dass der $d_{50}$-Wert 2,45 $\mu$m ($d_{10}$ = 1,05 $\mu$m, $d_{90}$ = 5,9 $\mu$m, gemessen bei einem Dispergierdruck von 2 bar absolut) und die BET-Oberfläche 0,8 m$^2$/g betrug.

Das Mahlgut wurde dann in Portionen von 20 kg in einem Schräglagen-Mischer (Typ VIS, Füllvolumen: 60 l, Aachener Misch- und Knetmaschinenfabrik) mit Misch- und Schneidflügel (Drehzahl Mischflügel:60 U/min, Drehzahl Schneidflügel: 3000 U/min) innerhalb von 5 min homogen mit 0,5 Gew.-% (bezogen auf das Mahlgut) feinteiligem $SiO_2$ der Fa. Degussa vom Typ Sipernat® D17 (Rüttelgewicht 150 g/l; $d_{50}$-Wert der $SiO_2$-Partikel (Laserbeugung nach ISO 13320-1) betrug 10 $\mu$m, die spezifische Oberfläche (Stickstoffadsorption nach ISO 5794-1, Annex D) betrug 100 m$^2$/g) vermischt.

2. Herstellung einer Ausgangsmasse 2

Eine Lösung A wurde hergestellt, indem man bei 60°C unter Rühren (70 U/min) zu 660 l eine Temperatur von 60°C aufweisendem Wasser innerhalb einer Minute 1,075 kg einer eine Temperatur von 60°C aufweisenden wässrigen Kaliumhydroxidlösung (47,5 Gew.-% KOH) und anschließend mit einer Dosiergeschwindigkeit von 600 kg/h 237,1 kg Ammoniumheptamolybdat-tetrahydrat (weiße Kristalle mit einer Körnung d < 1 mm, 81,5 Gew.-% $MoO_3$, 7,0-8,5 Gew.-% $NH_3$, max. 150 mg/kg Alkalimetalle, H.C. Starck, D-38642 Goslar) dosierte und die resultierende leicht trübe Lösung bei 60°C 60 min rührte.

Eine Lösung B wurde hergestellt, indem man bei 60°C in 282,0 kg einer eine Temperatur von 60 °C aufweisenden wässrigen Kobalt(-II)-nitratlösung (12,5 Gew.-% Co, hergestellt mit Salpetersäure aus Kobaltmetall der Firma MFT Metals & Ferro-Alloys Trading GmbH, D-41747 Viersen, Reinheit, >99,6 Gew.-%, < 0,3 Gew.-% Ni, < 100 mg/kg Fe, < 50 mg/kg Cu) vorlegte und zu dieser unter Rühren (70 U/min) 142,0 kg einer 60 °C warmen Eisen-(III)-nitrat-nonahydrat-Schmelze (13,8 Gew.-% Fe, < 0,4 Gew.-% Alkalimetalle, < 0,01 Gew.-% Chlorid, < 0,02 Gew.-% Sulfat, Dr. Paul Lohmann GmbH, D-81857 Emmerthal) dosierte. Anschließend wurde unter Aufrechterhaltung der 60 °C 30 Minuten nachgerührt. Dann wurde unter Beibehalt der 60 °C die Lösung B in die vorgelegte Lösung A abgelassen und weitere 15 Minuten bei 60°C gerührt. Anschließend wurden dem resultierenden wässrigen Gemisch 19,9 kg eines Kieselgels der Fa. Grace GmbH in D-67547 Worms vom Typ Ludox® TM-50 (50 Gew.-% $SiO_2$; stabilisierendes Gegen-Ion: $Na^+$; Partikelladung: negativ; Verhältnis Si : Na als Gewichtsverhältnis $SiO_2/Na_2O$ : 225; $SiO_2$-Gehalt: 50 Gew.-%; pH-Wert: 9,0; Massendichte (25 °C, 1 atm) : 1,40 $g/cm^3$; Sulfate (als $Na_2SO_4$) : 0,08 Gew.-%; titrierbares Alkali (als $Na_2O$) : 0,21 Gew.-%; Viskosität (25 °C, 1 atm) : 40 cP; spezifische Oberfläche der $SiO_2$-Partikel: 140 $m^2/g$) zugegeben und danach noch weitere 15 Minuten bei 60 °C gerührt.

Anschließend wurde in einem Drehscheibensprühturm vom Typ FS-15 der Firma Niro im Heißluftgegenstrom sprühgetrocknet (Gaseintrittstemperatur: 350 ± 10°C, Gasaustrittstemperatur: 140 ± 5°C, Scheibendrehzahl: 18000 U/min, Durchsatz: 270 kg/h). Das resultierende Sprühpulver wies einen Glühverlust von 30,5 Gew.-% (3 h bei 600°C im Porzellantiegel (der bei 900 °C bis zur Gewichtskonstanz geglüht worden war) unter Luft glühen) und (bei einem Dispergierdruck von 2,0 bar absolut) einen $d_{50}$ von 23,6 $\mu$m ($d_{10}$ = 5,2 $\mu$m, $d_{90}$ = 49,5 $\mu$m) auf. Figur 10 zeigt die Partikeldurchmesserverteilung des resultierenden Sprühpulvers in Abhängigkeit vom angewendeten Dispergierdruck. Die Abszisse zeigt die Partikeldurchmesser in logarithmischer Auftragung in $\mu$m. Die Ordinate zeigt den Volumenanteil in % des Gesamtpartikelvolumens, der den entsprechenden Partikeldurchmesser aufweist in Abhängigkeit vom angewandten Dispergierdruck:

▲ : Dispergierdruck = 2 bar abs..
♦ : Dispergierdruck = 1,1 bar abs..

Die nachfolgende Tabelle gibt einen Überblick über repräsentative dx-Werte in Abhängigkeit vom angewandten absoluten Dispergierdruck:

|  | 2 bar | 1,1 bar |
|---|---|---|
| $d_{10}$ ($\mu$m) | 5,2 | 9,9 |
| $d_{50}$ ($\mu$m) | 23,6 | 28,5 |
| $d_{90}$ ($\mu$m) | 49,5 | 56,3 |

3. Herstellung der Multimetalloxidkatalysatorformkörper und ihrer Vorläufer

110 kg der Ausgangsmasse 2 wurden dann in einem Schräglagen-Mischer (Typ VIL, Füllvolumen: 200 l, Aachener Misch- und Knetmaschinenfabrik) mit Misch- und Schneidflügel (Drehzahl Mischflügel: 39 U/min, Drehzahl Schneidflügel: 3000 U/min) vorgelegt und 1 min vorgemischt. Innerhalb von 10 min wurde hierzu bei fortgesetztem Mischen über eine Zellenradschleuse die Ausgangsmasse 1 in der für eine Multimetalloxidaktivmasse der Stöchiometrie:

$$[Bi_2W_2O_9 \cdot 2Wo_3]_{0,40}[Mo_{12}Co_{5,4}Fe_{3,1}Si_{1,5}K_{0,08}O_x]_1$$

erforderlichen Menge innerhalb von 10 min zudosiert. Dann wurde der Mischvorgang weitere 15 min fortgesetzt um eine (zur Erreichung einer hohen Aktivität und Acroleinselektivität erforderliche) intensive und vollständige Homogenisierung (einschließlich des Zerschlagens evtl. vorhandener Agglomerate) der beiden Ausgangsmassen zu erreichen. Bezogen auf die vorgenannte Gesamtmasse wurde innerhalb von weiteren 2 min 1 Gew.-% Graphit TIMREX T44 der Firma Timcal AG untergemischt.

Das resultierende Gemisch wurde dann in einem Kompaktor Typ K200/100 der Fa. Hosokawa Bepex GmbH) mit konkaven, geriffelten Glattwalzen (Spaltweite: 2,8 mm, Walzendrehzahl: 9 UPM, Presskraftsollwert: ca. 75 kN) verdichtet. Über integrierte Schwingsiebe der Fa. Allgaier (Siebweite Überkorn: 1,5 mm, Siebweite Unterkorn: 400 $\mu$m) mit Kugel-Siebhilfen (Durchmesser 22 mm) wurde ein Kompaktat mit einer großteils zwischen 400 $\mu$m und 1,5 mm liegenden Partikelgröße isoliert.

Für die Tablettierung wurden dem Kompaktat in einem Turbulent-Mischer der Firma Drais innerhalb von 2 min weitere 2,5 Gew.-% des Graphits TIMREX T44 der Firma Timcal AG zugemischt.

Anschließend wurde das wie beschrieben erzeugte pulverförmige Haufwerk mit Hilfe eines Korsch PH 865 Rundläufers unter Luft[g1]atmosphäre erfindungsgemäß verdichtet (Einfachwerkzeug, 65 Matrizen). Der grundsätzliche Vorrichtungsaufbau war wie in Figur 6. Der Durchmesser der Vordruckrolle betrug 100 mm und der Durchmesser der Hauptdruckrolle betrug 300 mm. Der Abstand zweier auf der Matrixenscheibe einander gegenüberliegender Matrizen betrug 780 mm.

Die verwendeten Matrizen waren Matrizen mit kongruentem doppeltem Kegelstumpf wie in den Figuren 3a, 3b schematisch gezeigt.

Die Länge I des Längsabschnitts I betrug 6,22 mm.

Die Länge II (die Länge II*) des Längsabschnitts II (des Längsabschnitts II*) betrug 8 mm.

Die Umrisslinie des Kreiszylinders I und des Kreiszylinders II betrugen 15,7 mm.

Der Durchmesser DD der Deckfläche des Kegelstumpfes KS betrug 5,1 mm.

Der Durchmesser DG der Grundfläche des Kegelstumpfes KS betrug 5,0 mm.

Die Länge der Umrisslinie des Kreiszylinders Z (des durchgehend kreiszylindrischen Mittelstiftes) betrug $2,5 \cdot \pi$ mm. Die plane obere Stirnfläche des Mittelstiftes MF schloss mit der planen oberen Matrizenstirnfläche bündig ab.

Es wurden nur ringähnliche Vorläuferformkörper $F^{LII}$ gefertigt, wobei der Endabstand E stets 3 mm betrug. Die obere Stirnfläche des unteren Stempels und die untere Stirnfläche des oberen Stempels waren beide plan gestaltet. Die Bohrachse B stand auf beiden Stirnflächen senkrecht.

Beide Mittelbohrungen, $MB^U$ und $MB°$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Die einzelne Matrize war aus einem Werkstoffverbund gefertigt. Dieser bestand auf seiner die Matrizenbohrung berührenden Seite aus dem gesinterten Hartmetall G10-Ni (6,9 bis 7 mm Wanddicke), das bei einer Korngröße von 1,2 $\mu$m zu 92,8 Gew.-% aus WC, zu 0,2 Gew.-% aus TiC und TaNbC sowie zu 7 Gew.-% aus Ni bestand, mit $R_a = 0,1$ $\mu$m und auf seiner von der Matrizenbohrung abgewandten Seite aus DIN-Werkzeugstahl 1.2379 (9 mm Wanddicke) mit $R_a = 0,8$ $\mu$m. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a = 0,4$ $\mu$m). $R_a$ der beiden Stirnflächen betrug ebenfalls 0,4 $\mu$m.

Die in den Füllraum eingebrachte Menge an pulverförmigem Haufwerk betrug 129 mg.

Während der gesamten Verfahrensausübung schloss die untere Stirnfläche des oberen Stempels im Zustand des Ausgangsabstands A mit dem oberen Ende des Längsabschnitts II bündig ab.

Die angewandte Vordruckkraft (Presskraft) betrug bei jedem der beiden Stempel 0,3 kN, die angewandte Hauptdruckkraft (Presskraft) betrug bei jedem der beiden Stempel 4,2 kN.

Die Seitendruckfestigkeiten der resultierenden ringähnlichen Multimetalloxid-Vollkatalysatorvorläuferformkörper lagen im Bereich von 21 bis 23 N.

Die Umdrehungsrate des Rundläufers lag bei 35 bis 45 UPM.

Hinsichtlich des Materials von Matrizenscheibenzunge, Matrizenscheibenstirn und Matrizenscheibenkinn gilt das in der Beschreibung Gesagte.

Um Staubfreisetzung zu vermeiden wurde die Tablettiermaschine abgesaugt (300 bis 400 Nm$^3$/h). Die Abluft wurde über einen Filter geführt, der periodisch abgereinigt wurde.

Das Verfahren wurde über einen Zeitraum von 5 Tagen (120 h), abgesehen von zwei kurzen (jeweils ca. 30 Minuten) Unterbrechungen zum Zweck des Absaugens von angesammeltem Feinstaub, ohne Unterbrechung durchgeführt.

Anschließend wurden die in diesem Zeitraum hergestellten ringähnlichen Multimetalloxid-Vollkatalysatorvorläuferformkörper wie in Beispiel 1 der DE-A 100 46 957 (die Schütthöhe in der Zersetzung (Kammern 1 bis 4) belief sich dabei jedoch auf 53 mm bei einer Verweilzeit pro Kammer von 1,23 h und in der Kalzination (Kammern 5 bis 8) belief sie sich auf 153 mm bei einer Verweilzeit von 3,89 h) beschrieben mittels einer Bandkalziniervorrichtung thermisch behandelt; die Kammern besaßen eine Grundfläche (bei einer einheitlichen Kammerlänge von 1,40 m) von 1,29 m$^2$ (Zersetzung) und 1,40 m$^2$ (Kalzination) und wurden von unten durch das grobmaschige Band von 50-210 Nm$^3$/h auf 100 °C (Zersetzung) bzw. 450 °C (Kalzination) vorgeheizte Zuluft durchströmt; zusätzlich wurde die Luft durch rotierende Ventilatoren (900 bis 1450 U/min) umgewälzt. Innerhalb der Kammern war die zeitliche und örtliche Abweichung der Temperatur vom Sollwert (typische Werte für die Zonen 1-8 sind: 140 °C, 190 °C, 220 °C, 265 °C, 380 °C, 425 °C, 460 °C, 460 °C) stets ≤ 2°C. Hinter Kammer 8 schloss sich eine auf 70 °C temperierte 2m lange Kühlzone an. Im Übrigen wurde wie in Beispiel 1 der DE-A 100 46 957 beschrieben verfahren.

Anschließend wurden die gebildeten ringähnlichen Multimetalloxidvollkatalysatorformkörper einer Unterkornsiebung unterworfen. Bei den verwendeten Sieben handelte es sich um Langlochsiebe. Ihre geradlinige Kantenlänge betrug 20 mm und der Abstand der beiden Kanten war 1,8 mm. Bezogen auf das Gewicht des insgesamt zur Siebung aufgegebenen Siebgutes betrug der anfallende Unterkornanteil 0,52 Gew.-%.

Wurde in gleicher Weise wie vorstehend beschrieben verfahren, die Verdichtung des pulverförmigen Haufwerks jedoch mittels einer Matrize entsprechender Geometrie und Werkstoffbeschaffenheit, jedoch mit dem Hartmetall G10-Co (Wanddicke 6,9 bis 7 mm, $R_a$ = 0,1 $\mu$m) auf der die Matrizenbohrung berührenden Seite (Korngröße = 2,5 $\mu$m; 93,5 Gew.-% WC, 6,5 Gew.-% Co, 0,5 Gew.-% TiC und TaNbC) durchgeführt, lag der bei der Siebung anfallende Unterkornanteil bei 0,78 Gew.-%.

Die wie beschrieben hergestellten ringähnlichen Multimetalloxidvollkatalysatoren eignen sich z. B. für die heterogen katalysierte partielle Gasphasenoxidation von Propylen zu Acrolein.

Alternativ zur erfindungsgemäßen Verdichtung mit Hilfe eines Korsch PH 865 Rundläufers kann die erfindungsgemäße Verdichtung auch mit einem Kilian Synthesis 700-77 A Rundläufers ausgeführt werden. Als Vordruckkraft (Vordruckpresskraft) können dabei bei jedem der beiden Stempel 0,6 kN und als Hauptdruckkraft (Hauptdruckpresskraft) bei jedem der beiden Stempel 5,0 kN angewendet werden. Weiterhin kann die erfindungsgemäße Verdichtung auch in Stickstoffatmosphäre durchgeführt werden.

II. Herstellung von ringähnlichen Multimetalloxid-Vollkatalysatorvorläuferformkörpern, wobei das aktive Multimetalloxid die Stöchiometrie $Mo_{12}Co_7Fe_{2,94}Bi_{0,6}Si_{1,59}K_{0,08}O_x$ aufwies

Bei 60 °C wurden 213 kg Ammoniumheptamolybdattetrahydrat (81,5 Gew.-% $MoO_3$) in 600 l Wasser gelöst. In diese Lösung wurden unter Aufrechterhaltung der 60 °C 0,97 kg einer 46,8 gew.-%igen wässrigen Kaliumhydroxidlösung von 20 °C eingerührt (dabei wurde eine Lösung A erhalten).

Eine zweite Lösung B wurde hergestellt, indem man unter Rühren zu 333,7 kg einer wässrigen Kobalt-(II)-nitratlösung (12,4 Gew.-% Co) bei 30°C 116,25 kg einer 20°C aufweisenden wässrigen Eisen-(III)-nitratlösung (14,2 Gew.-% Fe) gab. Nach beendeter Zugabe wurde noch 30 min. bei 30°C gerührt. Danach wurden bei 60°C 112,3 kg einer 20°C aufweisenden wässrigen Wismutnitratlösung (11,2 Gew.-% Bi) unter Erhalt der Lösung B eingerührt. Innerhalb von 30 min. wurde bei 60°C die Lösung B in die Lösung A eingerührt. 15 min. nach beendetem Einrühren wurden bei 60°C 19,16 kg Kieselsol (vom Typ Ludox TM-50 der Fa. Grace GmbH in D-67547 Worms) in die erhaltene Maische gegeben. Unter Aufrechterhaltung der 60°C wurde noch 15 min. nachgerührt. Dann wurde die erhaltene Maische im Heißluftgegenstromverfahren sprühgetrocknet (Gaseingangstemperatur: 400 ± 10°C, Gasausgangstemperatur: 140 ± 5°C) wobei ein Sprühpulver erhalten wurde, dessen Glühverlust (3 h bei 600°C unter Luft) 30 % seines Gewichtes betrug. Das Sprühpulver wies einen $d_{50}$ von 20,3 $\mu$m sowie einen $d_{10}$ von 3,24 $\mu$m und einen $d_{90}$ von 53,6 $\mu$m (gemessen bei einem Dispergierdruck von 2 bar absolut) auf.

In das Sprühpulver wurden zusätzlich 1,0 Gew.-% (bezogen auf die Sprühpulvermenge) Graphit Asbury 3160[g2] der Firma Asbury Graphite Mills, Inc. New Jersey 08802, USA eingemischt.

Das dabei resultierende Trockengemisch wurde mittels eines Kompaktors der Fa. Hosokawa Bepex GmbH (D-74211 Leingarten) vom Typ Kompaktor K 200/100 unter den Bedingungen von 2,8 mm Spaltbreite, 1,0 mm Siebweite, 200 $\mu$m Siebweite Unterkorn, 35 kN Presssollkraft und 65 bis 70 Upm Schneckendrehzahl durch Vorkompaktieren auf eine im wesentlichen einheitliche Korngröße von 200 $\mu$m bis 1 mm vergröbert.

Das Kompaktat wurde anschließend mit, bezogen auf sein Gewicht, weiteren 2 Gew.-% des selben Graphit vermischt und anschließend mit Hilfe eines Kilian Rundläufers vom Typ RX 73, der Fa. Kilian, D-50735 Köln, unter Luftatmosphäre zu ringähnlichen Multimetalloxid-Vollkatalysatorvorläuferformkörpern $F^{LII}$ mit nicht gekrümmter (d.h., mit planer) Stirnfläche verdichtet. Der grundsätzliche Vorrichtungsaufbau war wie in Figur 6. Das dabei verwendete Werkzeug (Matrize, Stempel etc.) sowie die Werkstoffe für Matrizenscheibenzunge, Matrizenscheibenstirn und Matrizenscheibenkinn entsprachen jenen aus Beispiel I. Dies gilt auch für die anderen Verdichtungsbedingungen einschließlich des Endabstands E von 3 mm. Die Seitendruckfestigkeit der resultierenden ringähnlichen Multimetalloxidvorläuferformkörper betrug 19 bis 21 N.

Zu ihrer nachfolgenden thermischen Behandlung wurden jeweils 1900 g der ringähnlichen Multimetalloxid-Vollkatalysatorvorläuferformkörper in einer beheizbaren Umluftkammer (0,12 m$^3$ Innenvolumen) aufgeschüttet (2 Nm$^3$ Luft/min.). Anschließend wurde die Temperatur in der Schüttung wie folgt verändert:

- mit 1°C/min. von 25°C auf 160°C erhöht;
- dann 100 min. bei 160°C gehalten;
- danach mit 3°C/min. von 160°C auf 200°C erhöht;

- dann 100 min. bei 200°C gehalten;
- danach mit 2°C/min. von 200°C auf 230°C erhöht;
- dann 100 min. bei 230°C gehalten;
- danach mit 3°C/min. von 230°C auf 270°C erhöht;
- dann 100 min. bei 270°C gehalten;
- danach mit 1°C/min. auf 380°C erhöht;
- dann 4,5 h bei 380°C gehalten;
- danach mit 1°C/min. auf 430°C erhöht;
- dann 4,5 h bei 430°C gehalten;
- danach mit 1°C/min. auf 500°C erhöht;
- dann 9 h bei 500°C gehalten;
- danach innerhalb von 4 h auf 25°C abgekühlt.

Dabei wurden aus den ringähnlichen Multimetalloxid-Vollkatalysatorvorläuferformkörpern ringähnliche Multimetalloxid-Vollkatalysatorformkörper erhalten. Diese eignen sich z.B. als Katalysatoren für die heterogen katalysierte partielle Gasphasenoxidation von Propylen zu Acrolein.

Diese wurden der Unterkornsiebung gemäß Beispiel I unterworfen. Bezogen auf das Gewicht des insgesamt zur Siebung aufgegebenen Siebgutes betrug der anfallende Unterkornanteil 0,68 Gew.-%.

Wurde in gleicher Weise wie vorstehend beschrieben verfahren und die Verdichtung mit entsprechenden Matrizen durchgeführt, deren Hartmetallbestandteil jedoch G10-Co war, lag der bei der Siebung anfallende Unterkornanteil bei 0,99 Gew.-%.

III. Herstellung von ringähnlichen Multimetalloxid-Vollkatalysatorvorläuferformkörpern, wobei das aktive Multimetalloxid die Stöchiometrie $Mo_{12}P_{1,5}V_{0,6}Cs_{1,0}Cu_{0,5}Sb_1S_{0,04}O_x$ aufwies

In 619 l auf 45°C temperiertes Wasser in einem wassertemperierten Doppelmantelbehälter wurden unter Rühren (70 Umdrehungen pro Minute (UPM)) 537,5 kg Ammoniumheptamolybdattetrahydrat ($(NH_4)_6Mo_7O_{24} \cdot 4H_2O$ (81 Gew.-% $MoO_3$, 8 Gew.-% $NH_3$, $\leq$ 50 Gew.-ppm Na und $\leq$ 100 Gew.-ppm K) eindosiert. Die Temperatur der Lösung sank hierbei auf 37°C ab. Um ein sicheres Auflösen des Ammoniumheptamolybdats zu gewährleisten, wurde nach Ende der Zudosierung noch 15 Minuten nachgerührt, wobei die Temperatur von 37°C beibehalten wurde. Unter weiterem Rühren wurden bei derselben Temperatur innerhalb von 3 Minuten 17,82 kg Ammoniummetavanadat ($NH_4VO_3$, 77 Gew.-% $V_2O_5$, 14,5 Gew.-% $NH_3$, $\leq$ 150 Gew.-ppm Na und $\leq$ 500 Gew.-ppm K) zudosiert. Es wurde 2 Minuten nachgerührt. Dann wurde innerhalb einer Minute eine in einem separaten Lösebehälter hergestellte, farblose, klare, 60°C warme Lösung von 49,6 kg Cäsiumnitrat ($CsNO_3$ mit 72 Gew.-% $Cs_2O$ und $\leq$ 50 Gew.-ppm Na, $\leq$ 100 Gew.-ppm K, < 10 Gew.-ppm Al sowie $\leq$ 20 Gew.-ppm Fe) in 106 l Wasser eingerührt. Hierbei stieg die Temperatur der resultierenden Suspension auf 39°C. Nach einminütigem Nachrühren wurden innerhalb einer weiteren Minute unter fortgesetztem Rühren 31,66 l 75 gew.-%ige Phosphorsäure (Dichte bei 25°C und 1 atm: 1,57 g/ml, Viskosität bei 25°C und 1 atm: 0,147 $cm^2$/S) zudosiert. Aufgrund der exothermen Reaktion stieg die Temperatur hierbei auf 42°C. Erneut wurde 1 Minute nachgerührt. Dann wurden innerhalb einer Minute 1,34 kg Ammoniumsulfat (($(NH_4)2SO_4$ (> 99 Gew.-%)) eingerührt und 1 weitere Minute nachgerührt. Unter fortgesetztem Rühren bei identischer Temperatur wurden innerhalb von 3 Minuten 37,04 kg Antimontrioxid ($Sb_2O_3$, Partikeldurchmesser $d_{50}$ = ca. 2$\mu$m, Kristallstruktur laut XRD: > 75 % Senarmontit, < 25 % Valentinit, Reinheit: > 99,3 Gew.-%, $\leq$ 0,3 Gew.-% $As_2O_3$, $\leq$ 0,3 Gew.-% PbO und $\leq$ 300 Gew.-ppm FeO) zugegeben (käuflich erhältlich als Triox White, Code No. 639000 der Fa. Antraco, D-10407 Berlin). Nun wurde die Rührerdrehzahl von 70 auf 50 UPM zurückgenommen. Anschließend wurde die gerührte Suspension mittels Dampf im Doppelmantel innerhalb von 30 Minuten linear auf 95°C aufgeheizt. Bei dieser Temperatur und 50 UPM wurden innerhalb von 4 Minuten 51,64 kg Kupfernitratlösung (wässrige $Cu(NO_3)_2$-Lösung mit 15,6 Gew.-% Cu) zugegeben. Nach 56-minütigem Nachrühren bei 95 °C wurde die Rührgeschwindigkeit weiter von 50 auf 35 UPM zurückgenommen. Anschließend wurde die gesamte Suspension innerhalb von 4 Minuten in einen mit Stickstoff überlagerten, auf 85 °C temperierten und mit 35 UPM gerührten Sprühturmvorlagenbehälter abgelassen und es wurde mit 20 l Wasser (25°C) nachgespült. Aus diesem heraus wurde die Suspension in einem Drehscheibensprühturm vom Typ FS-15 der Firma Niro im Heißluftgleichstrom sprühgetrocknet (Gaseintrittstemperatur: 285 $\pm$ 10°C, Gasaustrittstemperatur: 110 $\pm$ 5°C, Scheibendrehzahl: 18000 U/min, Durchsatz: 270 kg/h), wobei das resultierende Sprühpulver einen Glühverlust (1 h bei 500 °C in Luft) von 17,2 Gew.-% und einen $d_{50}$ von 35,9 $\mu$m aufwies ($d_{10}$ = 14,3 $\mu$m, $d_{90}$ = 65,6 $\mu$m, gemessen bei einem Dispergierdruck von 2 bar absolut) aufwies.

Das Sprühpulver wurde mit 1,5 Gew.-% des Graphits Timrex 44 der Firma Timcal homogen vermischt und kompaktiert (Kompaktor der Fa. Hosokawa Bepex GmbH, D-74211 Leingarten, Typ K200/100 mit konkaven, geriffelten Glattwalzen, Spaltweite: 2,8 mm, Siebweite: 1,25 mm, Siebweite Unterkorn: 400 $\mu$m, Schneckendrehzahl: 65 bis 70 UPM). Für die Tablettierung wurden dem Kompaktat weitere 1 Gew.-% desselben Graphits zugemischt.

Anschließend wurde das wie beschrieben erzeugte pulverförmige Haufwerk mit Hilfe eines Korsch PH 865 Rundläufers unter Luftatmosphäre erfindungsgemäß verdichtet (Einfachwerkzeug, 65 Matrizen). Der grundsätzliche Vorrichtungsaufbau war wie in Figur 6. Der Durchmesser der Vordruckrolle betrug 100 mm und der Durchmesser der Hauptdruckrolle betrug 300 mm. Der Abstand zweier auf der Matrizenscheibe einander gegenüber liegender Matrizen betrug 780 mm.

Die verwendeten Matrizen waren Matrizen mit kongruentem doppeltem Kegelstumpf wie in den Figuren 3a, 3b schematisch gezeigt. Die Länge I des Längsabschnitts I betrug 2,2 mm. Die Länge II (die Länge II*) des Längsabschnitts II (des Längsabschnitts II*) betrug 10 mm.

Die Umrisslinie des Kreiszylinders I und des Kreiszylinders II betrugen 22 mm. Der Durchmesser DD der Deckfläche des Kegelstumpfes KS betrug 7,1 mm. Der Durchmesser DG der Grundfläche des Kegelstumpfes KS betrug 7,0 mm. Die Länge der Umrisslinie des Kreiszylinders Z (des durchgehend kreiszylindrischen Mittelstiftes) betrug $3,0 \cdot \pi$ mm. Die plane obere Stirnfläche des Mittelstiftes MF schloss mit der planen oberen Matrizenstirnfläche bündig ab.

Es wurden nur ringähnliche Vorläuferformkörper $F^{LII}$ gefertigt, wobei der Endabstand E stets 7 mm betrug. Die obere Stirnfläche des unteren Stempels und die untere Stirnfläche des oberen Stempels waren beide plan gestaltet. Die Bohrachse B stand auf beiden Stirnflächen senkrecht.

Beide Mittelbohrungen, $MB^U$ und $MB^O$ (die letztere stand mit zwei gasdurchlässigen Auslässen in Verbindung (vgl. Figur 4d)), wiesen im Eingangsbereich in die zugehörige Stirnfläche eine kreiszylindrische Geometrie mit identischem Radius auf. Der Kontakt ihrer Innenwände zur äußeren Mantelfläche des Mittelstiftes MF war im möglichen Kontaktbereich aufeinander gleitend.

Die einzelne Matrize war aus einem Werkstoffverbund gefertigt. Dieser bestand auf seiner die Matrizenbohrung berührenden Seite aus dem Hartmetall G10-Ni (2,5 bis 2,6 mm Wanddicke) mit $R_a = 0,1\ \mu m$ und auf seiner von der Matrizenbohrung abgewandten Seite aus DIN-Werkzeugstahl 1.2379 (9 mm Wanddicke) mit $R_a = 0,8\ \mu m$. Der obere Stempel und der untere Stempel waren aus DIN-Werkstoff 1.2601 gefertigt. Der auf seiner Gesamtlänge kreiszylindrische Mittelstift MF war aus DIN-Werkzeugstahl 1.2343 gefertigt ($R_a = 0,4\ \mu m$). $R_a$ der beiden Stirnflächen betrug ebenfalls $0,4\ \mu m$.

Die in den Füllraum eingebrachte Menge an pulverförmigem Haufwerk betrug 576 mg.

Während der gesamten Verfahrensausübung schloss die untere Stirnfläche des oberen Stempels im Zustand des Ausgangsabstands A mit dem oberen Ende des Längsabschnitts II bündig ab. Die bei jedem der beiden Stempel angewandte Vordruckkraft (Presskraft) betrug 0,3 kN, die bei jedem der beiden Stempel angewandte Hauptdruckkraft (Presskraft) betrug 3,5 kN.

Die Seitendruckfestigkeiten der resultierenden ringähnlichen Multimetalloxid-Vollkatalysatorvorläuferformkörper lagen im Bereich von 33 bis 37 N. Die Umdrehungszahl des Rundläufers lag bei 20 bis 25 UPM. Hinsichtlich des Materials von Matrizenscheibenzunge, Matrizenscheibenstirn und Matrizenscheibenkinn gilt das in der Beschreibung Gesagte.

Das Verfahren wurde über einen Zeitraum von 5 Tagen (120 h), abgesehen von zwei kurzzeitigen (jeweils ca. 30 Minuten) Unterbrechungen zum Zweck des Absaugens von angesammeltem Feinstaub, ohne Unterbrechung durchgeführt.

Anschließend wurden jeweils 8 kg der ringähnlichen Multimetalloxid-Vollkatalysatorvorläufer-formkörper in einem Drahtbehälter der Grundfläche 33,0 cm x 49,5 cm gleichmäßig verteilt, wobei sich eine Schütthöhe von 4 cm ergab. Der Drahtbehälter wurde in einem Kammerofen (Fa. Elino Industrie-Ofenbau, Carl Hanf GmbH & Co, D-52355 Düren, Typ KA-040/006-08 EW.OH, Abmessungen: Länge = 57 cm, Breite = 57 cm, Höhe = 80 cm) so angeordnet, dass die Schüttung der Tabletten gleichmäßig durchströmbar war. Es wurden 2 Nm$^3$/h Frischluft zugeführt und die Luftumwälzung im Ofen so eingestellt, dass die Schüttung mit einer Geschwindigkeit von 0,9 m/s (bestimmt mittels Aerometer, Fa. Testo, Typ 445) durchströmt wurde. Der Ofen wurde nun mit der folgenden Temperaturrampe auf 380 °C aufgeheizt: innerhalb von 40 min auf 180 °C aufheizen, 30 min halten, innerhalb von 10 min auf 220 °C aufheizen, 30 min halten, innerhalb von 13 min auf 270 °C aufheizen, 30 min halten, innerhalb von 25 min auf 340 °C und dann innerhalb von 40 min auf 380 °C aufheizen. Diese Temperatur wurde dann 390 min gehalten. Währenddessen wurde der NH$_3$-Gehalt in der abgesaugten Atmosphäre der thermischen Behandlung kontinuierlich durch FTIR-Spektroskopie überwacht (Spektrometer der Fa. Nicolet, Typ "Impact", IR-Edelstahlzelle mit CaF$_2$-Fenster, 10 cm Schichtdicke, Temperierung auf 120 °C, Bestimmung der Konzentration anhand der Intensität der Bande bei 3.333 cm$^{-1}$). Der NH$_3$-Gehalt blieb während der gesamten thermischen Behandlung $\leq$ 2,4 Vol.-%. Dieser Maximalwert wurde bei 220 °C erreicht.

Die erhaltenen ringähnlichen Multimetalloxid-Vollkatalysatorformkörper eignen sich z.B. als Katalysatoren für die heterogen katalysierte partielle Gasphasenoxidation von Methacrolein zu Methacrylsäure.

Danach wurden die gebildeten ringähnlichen Multimetalloxidvollkatalysatorformkörper einer Unterkornsiebung unterworfen. Bei den verwendeten Sieben handelte es sich um Langlochsiebe. Ihre geradlinige Kantenlänge betrug 20 mm und der Abstand der beiden Kanten war 6 mm. Bezogen auf das Gewicht des insgesamt zur Siebung aufgegebenen Siebgutes betrug der anfallende Unterkornanteil 15,6 Gew.-%.

Wurde in gleicher Weise wie vorstehend beschrieben verfahren und die Verdichtung des pulverförmigen Haufwerks mit entsprechenden Matrizen durchgeführt, deren Hartmetallbestandteil jedoch G10-Co war, lag der bei der Siebung anfallende Unterkornanteil bei 18,1 Gew.-%.

US Provisional Patent Applications No. 61/077601, eingereicht am 02. Juli 2008 und No. 61/077638, ebenfalls eingereicht am 02. Juli 2008, sind in die vorliegende Anmeldung durch Literaturhinweis eingefügt. Im Hinblick auf die oben genannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren zur Herstellung eines oxidischen geometrischen Formkörpers, umfassend das mechanische Verdichten eines in den Füllraum einer Matrize eingebrachten pulverförmigen Haufwerks, aus Bestandteilen, die wenigstens eine Metallverbindung, die durch thermische Behandlung bei einer Temperatur $\geq 100\,°C$ in ein Metalloxid überführbar ist, oder wenigstens ein Metalloxid, oder wenigstens ein Metalloxid und wenigstens eine solche Metallverbindung mit der Maßgabe umfassen, dass wenigstens ein Bestandteil des pulverförmigen Haufwerks ein Nitratsalz, ein Ammoniumsalz oder Ammoniumnitrat ist, zu einem geometrischen Vorläuferformkörper, bei dem sich der Füllraum in einer mit einer vertikalen Bohrachse B von oben nach unten durch das Matrizenmaterial hindurchgeführten Martrizenbohrung befindet und wenigstens durch

   - die Innenwand der Matrizenbohrung,
   - die obere Stirnfläche eines von unten entlang der Bohrachse B in die Matrizenbohrung hub- und senkbeweglich eingeführten unterem Stempels, auf der das in den Füllraum eingebrachte pulverförmige Haufwerk aufliegt, und
   - die längs der Bohrachse B in einem axialen Ausgangsabstand A oberhalb der oberen Stirnfläche des unteren Stempels befindliche unter Stirnfläche eines entlang der Bohrachse B hub- und senkbeweglich angebrachten oberen Stempels, dessen untere Stirnfläche das in den Füllraum eingebrachte pulverförmige Haufwerk von oben berührt, begrenzt wird,

   indem man den axialen Ausgangsabstand A der beiden Stirnflächen dadurch auf einen für die Verdichtung vorgegebenen axialen Endabstand E längs der Bohrachse B verringert, dass man den oberen Stempel absenkt und dabei die Position des unteren Stempels beibehält oder den unteren Stempel zusätzlich anhebt und nach beendeter Verdichtung der obere Stempel vom gebildeten geometrischen Vorläuferformkörper abgehoben und der geometrische Vorläuferformkörper durch Anheben des unteren Stempels aus der Matrizenbohrung entfernt wird, sowie ein sich daran anschließendes Verfahren der thermischen Behandlung des geometrischen Vorläuferformkörpers bei einer Temperatur $\geq 100\,°C$, bei dem sich wenigstens eine Teilmenge seiner Bestandteile unter Ausbildung wenigstens einer gasförmigen Verbindung zersetzt und/oder chemisch umsetzt und der oxidische geometrische Formkörper sich ausbildet, **dadurch gekennzeichnet, dass** das die Matrizenbohrung berührende Matrizenmaterial ein Hartmetall ist, das zu $\geq 80$ Gew.-% aus dem Hartstoff Wolframcarbid und zu wenigstens 5 Gew.-% aus dem metallischen Bindemittel Nickel besteht.

2. Verfahren nach Anspruch 1, **dadurch gekennzeichnet, dass** das Hartmetall neben dem Hartstoff Wolframcarbid zusätzlich wenigstens einen weiteren Hartstoff aus der Gruppe bestehend aus Metallnitriden, Metallboriden und von Wolframcarbid verschiedenen Metallcarbiden enthält.

3. Verfahren nach Anspruch 2, **dadurch gekennzeichnet, dass** der metallische Anteil des wenigstens einen weiteren Hartstoffs wenigstens ein Metall aus der Gruppe bestehend aus Ti, Zr, Hf, V, Nb, Ta, Mo, Cr und W ist.

4. Verfahren nach einem der Ansprüche 1 bis 3, **dadurch gekennzeichnet, dass** das Hartmetall als metallisches Bindemittel entweder nur Nickel oder neben Nickel noch wenigstens ein metallisches Bindemittel aus der Gruppe bestehend aus Fe, Co und Cr enthält.

5. Verfahren nach Anspruch 4, **dadurch gekennzeichnet, dass** der auf das metallische Bindemittel Ni im Hartmetall entfallende Gewichtsanteil größer ist, als der auf jedes andere im Hartmetall enthaltene metallische Bindemittel entfallende Gewichtsanteil.

6. Verfahren nach einem der Ansprüche 1 bis 5, **dadurch gekennzeichnet, dass** das Hartmetall neben dem Hartstoff Wolframcarbid zusätzlich wenigstens einen weiteren Hartstoff aus der Gruppe bestehend aus TiC, TaC, NbC, VC,

$Cr_3C_2$ und Mischmetallcarbide, die wenigstens zwei der in den vorgenannten Metallcarbiden enthaltenen Metalle enthalten, enthält.

7. Verfahren nach einem der Ansprüche 1 bis 6, **dadurch gekennzeichnet, dass** das Hartmetall zu ≥ 7 Gew.-% aus dem metallischen Bindemittel Nickel besteht.

8. Verfahren nach einem der Ansprüche 1 bis 7, **dadurch gekennzeichnet, dass** das Hartmetall zu ≥ 85 Gew.-% aus Wolframcarbid besteht.

9. Verfahren nach einem der Ansprüche 1 bis 7, **dadurch gekennzeichnet, dass** das Hartmetall zu ≥ 90 Gew.-% aus Wolframcarbid besteht.

10. Verfahren nach einem der Ansprüche 1 bis 9, **dadurch gekennzeichnet, dass** das Hartmetall zu 90 bis 95 Gew.-% aus Wolframcarbid, zu ≥ 0 bis 1 Gew.-% aus wenigstens einem Metallcarbid aus der Gruppe bestehend aus TiC, TaC, NbC, VC, $Cr_3C_2$ und Mischmetallcarbiden, die wenigstens zwei der in den vorgenannten Metallcarbiden enthaltenen Metalle enthalten, sowie zu bis zu 10 Gew.-% aus den metallischen Bindemitteln Ni, Fe, Co und/oder Cr mit der Maßgabe besteht, dass der Gewichtsanteil des Ni am Hartmetall ≥ 5 Gew.-% beträgt.

11. Verfahren nach Anspruch 10, **dadurch gekennzeichnet, dass** das Hartmetall als metallisches Bindemittel nur Nickel oder nur Nickel und Chrom enthält.

12. Verfahren nach einem der Ansprüche 1 bis 11, **dadurch gekennzeichnet, dass** die Korngröße der Hartstoffe im Hartmetall im Bereich 0,5 μm bis 2 μm liegt.

13. Verfahren nach einem der Ansprüche 1 bis 12, **dadurch gekennzeichnet, dass** die Matrize ausschließlich aus dem Hartmetall besteht.

14. Verfahren nach einem der Ansprüche 1 bis 12, **dadurch gekennzeichnet, dass** die Matrize nur auf ihrer die Matrizenbohrung berührenden Seite aus dem Hartmetall und auf ihrer von der Matrizenbohrung abgewandten Seite aus einem Werkzeugstahl der Elementzusammensetzung

| 1,50 bis 1,80 Gew.-% | C, |
|---|---|
| 0,10 bis 0,40 Gew.-% | Si, |
| 0,10 bis 0,50 Gew.-% | Mn, |
| ≥ 0 bis 0,05 Gew.-% | P, |
| ≥ 0 bis 0,05 Gew.-% | S, |
| 10 bis 13 Gew.-% | Cr, |
| 0,50 bis 0,80 Gew.-% | Mo, |
| 0,10 bis 1,10 Gew.-% | V, |
| ≥ 0 bis 0,60 Gew.-% | W, und |
| ≥ 0 bis 0,10 Gew.-% | eines oder mehrere seltene Erdmetalle, und im übrigen Fe und herstellungsbedingte Verunreinigungen, |

besteht.

15. Verfahren nach einem der Ansprüche 1 bis 14, **dadurch gekennzeichnet, dass** das pulverförmige Haufwerk wenigstens ein Nitratsalz aus der Gruppe bestehend aus Kobaltnitrat, Eisennitrat, Wismutnitrat, Nickelnitrat, Cäsiumnitrat, Kupfernitrat, Calciumnitrat, Magensiumnitrat und den Hydraten der vorgenannten Nitrate, enthält.

16. Verfahren nach einem der der Ansprüche 1 bis 15, **dadurch gekennzeichnet, dass** das pulverförmige Haufwerk wenigstens ein Ammoniumsalz aus der Gruppe bestehend aus $NH_4HCO_3$, $NH_4OH$, $(NH_4)_2CO_3$, $NH_4HSO_4$, $(NH_4)_2SO_4$, $NH_4CHO_2$, Ammoniummetavanadat, Ammoniumheptamolybdat, $NH_4CH_3CO_2$, Ammoniumoxalat und den Hydraten der vorgenannten Ammoniumverbindungen enthält.

17. Verfahren nach einem der Ansprüche 1 bis 16, **dadurch gekennzeichnet, dass** sich bei der thermischen Behandlung der geometrischen Vorläuferformkörper wenigstens eine gasförmige Verbindung aus der Gruppe bestehend aus Ammoniak, Wasserdampf, $CO_2$, CO und Stickoxiden bildet.

18. Verfahren nach einem der Ansprüche 1 bis 17, **dadurch gekennzeichnet, dass** mit der thermischen Behandlung der Vorläuferformkörper ein Gewichtsverlust einhergeht, der, auf deren Ausgangsgewicht vor deren thermischen Behandlung bezogen, 0,5 bis 40 Gew.-% beträgt.

19. Verfahren nach einem der Ansprüche 1 bis 18, **dadurch gekennzeichnet, dass** der Endabstand E 2 bis 10 mm beträgt.

20. Verfahren nach einem der Ansprüche 1 bis 18, **dadurch gekennzeichnet, dass** der Endabstand E 2 bis 8 mm beträgt.

21. Verfahren nach einem der Ansprüche 1 bis 20, **dadurch gekennzeichnet, dass** der Mittenrauhwert $R_a$ der Innenwand der Matrizenbohrung $\leq 0,1\ \mu m$ beträgt.

22. Verfahren nach einem der Ansprüche 1 bis 21, **dadurch gekennzeichnet, dass** im Endabstand E von beiden Stempeln ein Pressdruck ausgeübt wird, der im Bereich 50 bis 5000 kg/cm$^2$ liegt.

23. Verfahren nach einem der Ansprüche 1 bis 22, **dadurch gekennzeichnet, dass** im Endabstand E von beiden Stempeln ein Pressdruck ausgeübt wird, der im Bereich 500 bis 2500 kg/cm$^2$ liegt.

24. Verfahren nach einem der Ansprüche 1 bis 23, **dadurch gekennzeichnet, dass** das Verfahren der thermischen Behandlung der hergestellten Vorläuferformkörper bei einer Temperatur $\geq$ 200 °C erfolgt.

25. Verfahren nach einem der Ansprüche 1 bis 24, **dadurch gekennzeichnet, dass** das Verfahren der thermischen Behandlung der hergestellten Vorläuferformkörper bei einer Temperatur $\geq$ 300 °C erfolgt.

26. Verfahren nach einem der Ansprüche 1 bis 25, **dadurch gekennzeichnet, dass** das pulverförmige Haufwerk Graphit, Stärke, gemahlene Nussschale, feinteiliges Kunststoffgranulat, Cellulose, Stearinsäure, Malonsäure, Salz der Stearinsäure und/oder Salz der Malonsäure zugesetzt enthält.

27. Verfahren nach einem der Ansprüche 1 bis 26, **dadurch gekennzeichnet, dass** der in dem mechanisch zu verdichtenden pulverförmigen Haufwerk insgesamt enthaltene Gewichtsanteil an Nitrationen und Ammoniumionen $\geq$ 0,1 Gew.-% beträgt.

28. Verfahren nach einem der Ansprüche 1 bis 26, **dadurch gekennzeichnet, dass** der in dem mechanisch zu verdichtenden pulverförmigen Haufwerk insgesamt enthaltene Gewichtsanteil an Nitrationen und Ammoniumionen $\geq$ 0,2 Gew.-% beträgt.

29. Verfahren nach einem der Ansprüche 1 bis 28, **dadurch gekennzeichnet, dass** bei dem anschließenden Verfahren der thermischen Behandlung der hergestellten geometrischen Vorläuferformkörper in selbigen ein Multimetalloxid ausgebildet wird, das die Elemente Mo und Fe, oder die Elemente Mo, Fe und Bi, oder die Elemente Mo und V, oder die Elemente Mo, V und P, oder die Elemente V und P enthält.

30. Verfahren nach einem der Ansprüche 1 bis 29, **dadurch gekennzeichnet, dass** bei dem anschließenden Verfahren der thermischen Behandlung der hergestellten geometrischen Vorläuferformkörper in selbigen ein Multimetalloxid ausgebildet wird, in welchem das Element Mo, oder das Element V, oder das Element P dasjenige von Sauerstoff verschiedene Element ist, das molar gerechnet das numerisch am häufigsten enthaltene ist.

31. Verfahren nach einem der Ansprüche 1 bis 30, **dadurch gekennzeichnet, dass** bei dem anschließenden Verfahren der thermischen Behandlung der hergestellten geometrischen Vorläuferformkörper in selbigen ein Multimetalloxid der allgemeinen Formel XII,

$$Mo_{12}Bi_aFe_bX^1_cX^2_dX^3_eX^4_fO_n \qquad (XII),$$

mit

$X^1$ = Nickel und/oder Kobalt,
$X^2$ = Thallium, Samarium, ein Alkalimetall und/oder ein Erdalkalimetall,
$X^3$ = Zink, Phosphor, Arsen, Bor, Antimon, Zinn, Cer, Blei, Vanadium, Chrom, Niob und/oder Wolfram,
$X^4$ = Silicium, Aluminium, Titan und/oder Zirkonium,
a = 0,2 bis 5,
b = 0,01 bis 5,
c = 0 bis 10,
d = 0 bis 2,
e = 0 bis 8,
f = 0 bis 10, und
n = eine Zahl, die durch die Wertigkeit und Häufigkeit der von Sauerstoff verschiedenen Elemente in XII bestimmt wird,

ausgebildet wird.

32. Verfahren nach einem der Ansprüche 1 bis 30, **dadurch gekennzeichnet, dass** bei dem anschließenden Verfahren der thermischen Behandlung der hergestellten geometrischen Vorläuferformkörper in selbiglen ein Multimetalloxid der allgemeinen Formel XIII,

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (XIII),$$

mit

$Y^1$ = nur Wismut oder Wismut und wenigstens eines der Elemente Tellur, Antimon, Zinn und Kupfer,
$Y^2$ = Molybdän oder Wolfram, oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Vanadium, Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
$Y^8$ = Molybdän oder Wolfram, oder Molybdän und Wolfram,
a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x', y' = Zahlen, die durch die Wertigkeit und Häufigkeit der von Sauerstoff verschiedenen Elemente in XIII bestimmt werden, und
p, q = Zahlen, deren Verhältnis p/q 0,1 bis 10 beträgt,

ausgebildet wird.

33. Verfahren nach einem der Ansprüche 1 bis 30, **dadurch gekennzeichnet, dass** bei dem anschließenden Verfahren der thermischen Behandlung der hergestellten geometrischen Vorläuferformkörper in selbigen ein Multimetalloxid der allgemeinen Formel XIV,

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (XIV),$$

mit

$Z^2$ = Molybdän oder Wolfram, oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer, Vanadium, Chrom und/oder
Bi, $Z^6$ = Silicium, Aluminium, Titan und/oder Zirkonium,
$Z^7$ = Kupfer, Silber und/oder Gold,
$Z^8$ = Molybdän oder Wolfram, oder Wolfram und Molybdän
a" = 0,1 bis 1,
b" = 0,2 bis 2,
c"= 3 bis 10,
d" = 0,02 bis 2,
e" = 0,01 bis 5,
f" = 0 bis 5,
g" = 0 bis 10,
h" = 0 bis 1,
x", y" = Zahlen, die durch die Wertigkeit und Häufigkeit der von Sauerstoff verschiedenen Elemente in XIV bestimmt werden, und
p", q" = Zahlen, deren Verhältnis p" / q" 0,1 bis 5 beträgt,

ausgebildet wird.

34. Verfahren nach einem der Ansprüche 1 bis 30, **dadurch gekennzeichnet, dass** bei dem anschließenden Verfahren der thermischen Behandlung der hergestellten geometrischen Vorläuferformkörper in selbigen ein Multimetalloxid der allgemeinen Formel XV,

$$Mo_{12}P_aV_bX_c^1X_d^2X_e^3Sb_fRe_gS_hO_n \qquad (XV),$$

mit

$X^1$ = Kalium, Rubidium und/oder Cäsium,
$X^2$ = Kupfer und/oder Silber,
$X^3$ = Cer, Bor, Zirkonium, Mangan und/oder Wismut,
a = 0,5 bis 3,
b = 0,01 bis 3,
c = 0,2 bis 3,
d = 0,01 bis 2,
e = 0 bis 2,
f = 0 bis 2,
g = 0 bis 1,
h = 0 bis 0,5, und
n = eine Zahl, die durch die Wertigkeit und Häufigkeit der von Sauerstoff verschiedenen Elemente in XV bestimmt wird,

ausgebildet wird.

35. Verfahren nach einem der Ansprüche 1 bis 30, **dadurch gekennzeichnet, dass** bei dem anschließenden Verfahren der thermischen Behandlung der hergestellten geometrischen Vorläuferformkörper in selbigen ein Multimetalloxid der allgemeinen Formel XVI,

$$V_1P_bFe_cX_d^1X_e^2O_n \qquad (XVI),$$

mit

$X^1$ = Mo, Bi, Co, Ni, Si, Zn, Hf, Zr, Ti, Cr, Mn, Cu, B, Sn und/oder Nb,
$X^2$ = Li, K, Na, Rb, Cs und/oder Tl,

b = 0,9 bis 1,5,

c = 0 bis 0,1,

d = 0 bis 0,1,

e = 0 bis 0,1, und

n = eine Zahl, die durch die Wertigkeit und Häufigkeit der von Sauerstoff verschiedenen Elemente in XVI,

ausgebildet wird.

36. Verfahren nach einem der Ansprüche 1 bis 28, **dadurch gekennzeichnet, dass** bei dem anschließenden Verfahren der thermischen Behandlung der hergestellten geometrischen Vorläuferformkörper in selbigen ein bei Normalbedingungen festes Oxid ausgebildet wird, in welchem kein Übergangsmetall der 5. bis 11. Nebengruppe und auch nicht Phosphor dasjenige von Sauerstoff verschiedene Element ist, das molar gerechnet das numerisch am häufigsten enthaltene ist.

37. Verfahren nach einem der Ansprüche 1 bis 28, **dadurch gekennzeichnet, dass** das pulverförmige Haufwerk wenigstens ein Metalloxid aus der Gruppe bestehend aus Aluminiumoxid, Wolframoxid, Antimonoxid, Zirkoniumoxid, Wismutoxid, Molybdänoxid, Siliciumoxid, Magnesiumoxid und Mischoxiden, die wenigstens zwei der in den vorgenannten Metalloxiden enthaltenen Metallelemente enthalten, enthält.

38. Verfahren der heterogen katalysierten partiellen Gasphasenoxidation wenigstens einer organischen Verbindung an einem Katalysatorfestbett, **dadurch gekennzeichnet, dass** man nach einem Verfahren gemäß einem der Ansprüche 1 bis 37 einen geometrischen oxidischen Formkörper herstellt und mit diesem das Verfahren der heterogen katalysierten partiellen Gasphasenoxidation wenigstens einer organischen Verbindung im Katalysatorfestbett katalysiert.

39. Verfahren nach Anspruch 38, **dadurch gekennzeichnet, dass** die heterogen katalysierte partielle Gasphasenoxidation diejenige

a) von Propylen zu Acrolein und/oder Acrylsäure

oder

b) von Acrolein zu Acrylsäure,

oder

c) von Methacrolein zu Methacrylsäure,

oder

d) von iso-Buten zu Methacrolein und/oder Methacrylsäure,

oder

e) von Propan zu Acrolein und/oder Acrylsäure,

oder

f) von iso-Butan zu Methacrolein und/oder Methacrylsäure,

oder

g) von wenigstens einem $C_4$-Kohlenwasserstoff und/oder Benzol zu Maleinsäureanhydrid,

oder

h) von Methanol zu Formaldehyd

oder

i) die Oxichlorierung von Ethylen zu 1,2-Dichlorethan

ist.

## Claims

1. A process for producing an oxidic geometric shaped body comprising the mechanical compaction of a pulverulent aggregate which has been introduced into the fill chamber of a die and is composed of constituents which comprise at least one metal compound which can be converted to a metal oxide by thermal treatment at a temperature of ≥ 100°C, or at least one metal oxide, or at least one metal oxide and at least one such metal compound, with the proviso that at least one constituent of the pulverulent aggregate is a nitrate salt, an ammonium salt or ammonium nitrate, to give a geometric shaped precursor body, in which the fill chamber is disposed in a die bore conducted through the die material from the top downward with a vertical bore axis B and is delimited at least by

- the inner wall of the die bore,
- the upper end face of a lower punch introduced from below along the bore axis B into the die bore so as to be liftable and lowerable, on which the pulverulent aggregate introduced into the fill chamber rests, and
- the lower end face, disposed along the bore axis B at an axial starting distance A above the upper end face of the lower punch, of an upper punch mounted so as to be liftable and lowerable along the bore axis B, whose lower end face is in contact with the pulverulent aggregate introduced into the fill chamber from above,

by reducing the axial starting distance A of the two end faces along the bore axis B to an axial end distance E predefined for the compaction by lowering the upper punch while maintaining the position of the lower punch or additionally lifting the lower punch, and on completion of compaction, by lifting the upper punch from the geometric shaped precursor body formed and removing the geometric shaped precursor body from the die bore by lifting the lower punch,

and a subsequent process for thermal treatment of the geometric shaped precursor body at a temperature of $\geq$ 100°C, in which at least a portion of its constituents is decomposed and/or chemically converted to form at least one gaseous compound and the oxidic geometric shaped body forms,

wherein the die material in contact with the die bore is a hard metal which consists to an extent of $\geq 80\%$ by weight of the hard material tungsten carbide and to an extent of at least 5% by weight of the metallic binder nickel.

2. The process according to claim 1, wherein the hard metal, in addition to the hard substance tungsten carbide, additionally comprises at least one further hard substance from the group consisting of metal nitrides, metal borides and metal carbides other than tungsten carbide.

3. The process according to claim 2, wherein the metallic component of the at least one further hard substance is at least one metal from the group consisting of Ti, Zr, Hf, V, Nb, Ta, Mo, Cr and W.

4. The process according to any one of claims 1 to 3, wherein the hard metal, as the metallic binder, comprises either only nickel or, in addition to nickel, also at least one metallic binder from the group consisting of Fe, Co and Cr.

5. The process according to claim 4, wherein the proportion by weight accounted for by the metallic binder Ni in the hard metal is greater than the proportion by weight accounted for by any other metallic binder present in the hard metal.

6. The process according to any one of claims 1 to 5, wherein the hard metal, in addition to the hard substance tungsten carbide, additionally comprises at least one further hard substance from the group consisting of TiC, TaC, NbC, VC, $Cr_3C_2$ and mixed metal carbides which comprise at least two of the metals present in the aforementioned metal carbides.

7. The process according to any one of claims 1 to 6, wherein the hard metal consists to an extent of $\geq 7\%$ by weight of the metallic binder nickel.

8. The process according to any one of claims 1 to 7, wherein the hard metal consists to an extent of $\geq 85\%$ by weight of tungsten carbide.

9. The process according to any one of claims 1 to 7, wherein the hard metal consists to an extent of $\geq 90\%$ by weight of tungsten carbide.

10. The process according to any one of claims 1 to 9, wherein the hard metal consists to an extent of from 90 to 95% by weight of tungsten carbide, to an extent of from $\geq 0$ to 1% by weight of at least one metal carbide from the group consisting of TiC, TaC, NbC, VC, $Cr_3C_2$ and mixed metal carbides which comprise at least two of the metals present in the aforementioned metal carbides, and up to 10% by weight of the metallic binders Ni, Fe, Co and/or Cr, with the proviso that the proportion by weight of the Ni in the hard metal is $\geq 5\%$ by weight.

11. The process according to claim 10, wherein the hard metal comprises, as the metallic binder, only nickel or only nickel and chromium.

12. The process according to any one of claims 1 to 11, wherein the particle size of the hard substances in the hard metal is in the range from 0.5 $\mu$m to 2 $\mu$m.

13. The process according to any one of claims 1 to 12, wherein the die consists exclusively of the hard metal.

14. The process according to any one of claims 1 to 12, wherein the die consists of the hard metal only on its side in contact with the die bore and of a tool steel on its side facing away from the die bore, said tool steel having the following element composition

 1.50 to 1.80% by wt. of C,
 0.10 to 0.40% by wt. of Si,
 0.10 to 0.50% by wt. of Mn,
 $\geq$ 0 to 0.05% by wt. of P,
 $\geq$ 0 to 0.05% by wt. of S,
 10 to 13% by wt. of Cr,
 0.50 to 0.80% by wt. of Mo,
 0.10 to 1.10% by wt. of V,
 $\geq$ 0 to 0.60% by wt. of W, and
 $\geq$ 0 to 0.10% by wt. of one or more rare earth metals, and apart from these Fe and impurities resulting from the production.

15. The process according to any one of claims 1 to 14, wherein the pulverulent aggregate comprises at least one nitrate salt from the group consisting of cobalt nitrate, iron nitrate, bismuth nitrate, nickel nitrate, cesium nitrate, copper nitrate, calcium nitrate, magnesium nitrate and the hydrates of the aforementioned nitrates.

16. The process according to any one of claims 1 to 15, wherein the pulverulent aggregate comprises at least one ammonium salt from the group consisting of $NH_4HCO_3$, $NH_4OH$, $(NH_4)_2CO_3$, $NH_4HSO_4$, $(NH_4)_2SO_4$, $NH_4CHO_2$, ammonium metavanadate, ammonium heptamolybdate, $NH_4CH_3CO_2$, ammonium oxalate and the hydrates of the aforementioned ammonium compounds.

17. The process according to any one of claims 1 to 16, wherein at least one gaseous compound from the group consisting of ammonia, water vapor, $CO_2$, CO and nitrogen oxides forms in the course of the thermal treatment of the geometric shaped precursor bodies.

18. The process according to any one of claims 1 to 17, wherein the thermal treatment of the shaped precursor bodies is accompanied by a weight loss which, based on the starting weight thereof before the thermal treatment thereof, is from 0.5 to 40% by weight.

19. The process according to any one of claims 1 to 18, wherein the end distance E is from 2 to 10 mm.

20. The process according to any one of claims 1 to 18, wherein the end distance E is from 2 to 8 mm.

21. The process according to any one of claims 1 to 20, wherein the mean roughness $R_a$ of the inner wall of the die bore is $\leq$ 0.1 $\mu$m.

22. The process according to any one of claims 1 to 21, wherein, at the end distance E of the two punches, a pressure in the range from 50 to 5000 $kg/cm^2$ is exerted.

23. The process according to any one of claims 1 to 22, wherein, at the end distance E of the two punches, a pressure in the range from 500 to 2500 $kg/cm^2$ is exerted.

24. The process according to any one of claims 1 to 23, wherein the process for thermal treatment of the shaped precursor bodies produced is effected at a temperature of $\geq$ 200°C.

25. The process according to any one of claims 1 to 24, wherein the process for thermal treatment of the shaped precursor bodies produced is effected at a temperature of $\geq$ 300°C.

26. The process according to any one of claims 1 to 25, wherein the pulverulent aggregate comprises added graphite, starch, ground nutshell, fine polymer granule, cellulose, stearic acid, malonic acid, salt of stearic acid and/or salt of malonic acid.

27. The process according to any one of claims 1 to 26, wherein the total proportion by weight of nitrate ions and ammonium ions present in the pulverulent aggregate to be mechanically compacted is $\geq$ 0.1% by weight.

28. The process according to any one of claims 1 to 26, wherein the total proportion by weight of nitrate ions and ammonium ions present in the pulverulent aggregate to be mechanically compacted is $\geq 0.2\%$ by weight.

29. The process according to any one of claims 1 to 28, wherein the subsequent process for thermal treatment of the geometric shaped precursor bodies produced forms a multimetal oxide which comprises the elements Mo and Fe, or the elements Mo, Fe and Bi, or the elements Mo and V, or the elements Mo, V and P, or the elements V and P.

30. The process according to any one of claims 1 to 29, wherein the subsequent process for thermal treatment of the geometric shaped precursor bodies produced forms a multimetal oxide in which the element Mo, or the element V, or the element P is that element other than oxygen which, calculated in molar terms, is numerically the most common.

31. The process according to any one of claims 1 to 30, wherein the subsequent process for thermal treatment of the geometric shaped precursor bodies produced forms a multimetal oxide of the general formula XII

$$Mo_{12}Bi_aFe_bX^1_cX^2_dX^3_eX^4_fO_n \qquad\qquad (XII)$$

where

$X^1$ = nickel and/or cobalt,
$X^2$ = thallium, samarium, an alkali metal and/or an alkaline earth metal,
$X^3$ = zinc, phosphorus, arsenic, boron, antimony, tin, cerium, lead, vanadium, chromium, niobium and/or tungsten,
$X^4$ = silicon, aluminum, titanium and/or zirconium,
$a$ = 0.2 to 5,
$b$ = 0.01 to 5,
$c$ = 0 to 10,
$d$ = 0 to 2,
$e$ = 0 to 8,
$f$ = 0 to 10, and
$n$ = a number which is determined by the valency and frequency of the elements in XII other than oxygen.

32. The process according to any one of claims 1 to 30, wherein the subsequent process for thermal treatment of the geometric shaped precursor bodies produced forms a multimetal oxide of the general formula XIII

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad\qquad (XIII)$$

where

$Y^1$ = only bismuth or bismuth and at least one of the elements tellurium, antimony, tin and copper,
$Y^2$ = molybdenum or tungsten, or molybdenum and tungsten,
$y^3$ = an alkali metal, thallium and/or samarium,
$Y^4$ = an alkaline earth metal, nickel, cobalt, copper, manganese, zinc, tin, cadmium and/or mercury,
$Y^5$ = iron or iron and at least one of the elements vanadium, chromium and cerium,
$Y^6$ = phosphorus, arsenic, boron and/or antimony,
$Y^7$ = a rare earth metal, titanium, zirconium, niobium, tantalum, rhenium, ruthenium, rhodium, silver, gold, aluminum, gallium, indium, silicon, germanium, lead, thorium and/or uranium,
$Y^8$ = molybdenum or tungsten, or molybdenum and tungsten,
$a'$ = 0.01 to 8,
$b'$ = 0.1 to 30,
$c'$ = 0 to 4,
$d'$ = 0 to 20,
$e'$ > 0 to 20,
$f'$ = 0 to 6,
$g'$ = 0 to 15,
$h'$ = 8 to 16,
$x', y'$ = numbers which are determined by the valency and frequency of the elements in XIII other than oxygen, and
$p, q$ = numbers whose p/q ratio is from 0.1 to 10.

**33.** The process according to any one of claims 1 to 30, wherein the subsequent process for thermal treatment of the geometric shaped precursor bodies produced forms a multimetal oxide of the general formula XIV

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (XIV)$$

where

$Z^2$ = molybdenum or tungsten, or molybdenum and tungsten,
$Z^3$ = nickel and/or cobalt,
$Z^4$ = thallium, an alkali metal and/or an alkaline earth metal,
$Z^5$ = phosphorus, arsenic, boron, antimony, tin, cerium, vanadium, chromium and/or Bi,
$Z^6$ = silicon, aluminum, titanium and/or zirconium,
$Z^7$ = copper, silver and/or gold,
$Z^8$ = molybdenum or tungsten, or molybdenum and tungsten,
a" = 0.1 to 1,
b" = 0.2 to 2,
c" = 3 to 10,
d" = 0.02 to 2,
e" = 0.01 to 5,
f" = 0 to 5,
g" = 0 to 10,
h" = 0 to 1,
x", y" = numbers which are determined by the valency and frequency of the elements in XIV other than oxygen, and
p", q" = numbers whose p" / q" ratio is from 0.1 to 5.

**34.** The process according to any one of claims 1 to 30, wherein the subsequent process for thermal treatment of the geometric shaped precursor bodies produced forms a multimetal oxide of the general formula XV

$$Mo_{12}P_aV_bX^1_cX^2_dX^3_eSb_fRe_gS_hO_n \qquad (XV)$$

where

$X^1$ = potassium, rubidium and/or cesium,
$X^2$ = copper and/or silver,
$X^3$ = cerium, boron, zirconium, manganese and/or bismuth,
a = 0.5 to 3,
b = 0.01 to 3,
c = 0.2 to 3,
d = 0.01 to 2,
e = 0 to 2,
f = 0 to 2,
g = 0 to 1,
h = 0 to 0.5, and
n = a number which is determined by the valency and frequency of the elements in XV other than oxygen.

**35.** The process according to any one of claims 1 to 30, wherein the subsequent process for thermal treatment of the geometric shaped precursor bodies produced forms a multimetal oxide of the general formula XVI

$$V_1P_bFe_cX^1_dX^2_eO_n \qquad (XVI)$$

where

$X^1$ = Mo, Bi, Co, Ni, Si, Zn, Hf, Zr, Ti, Cr, Mn, Cu, B, Sn and/or Nb,
$X^2$ = Li, K, Na, Rb, Cs and/or Tl,
b = 0.9 to 1.5,
c = 0 to 0.1,
d = 0 to 0.1,
e = 0 to 0.1, and

n = a number which by the valency and frequency of the elements in XVI other than oxygen.

36. The process according to any one of claims 1 to 28, wherein the subsequent process for thermal treatment of the geometric shaped precursor bodies produced forms an oxide which is solid under standard conditions and in which no transition metal of transition group 5 to 11 nor phosphorus is that element other than oxygen which, calculated in molar terms, is numerically the most common.

37. The process according to any one of claims 1 to 28, wherein the pulverulent aggregate comprises at least one metal oxide from the group consisting of aluminum oxide, tungsten oxide, antimony oxide, zirconium oxide, bismuth oxide, molybdenum oxide, silicon oxide, magnesium oxide and mixed oxides which comprise at least two of the metal elements present in the aforementioned metal oxides.

38. A process for heterogeneously catalyzed partial gas phase oxidation of at least one organic compound over a fixed catalyst bed, wherein a geometric oxidic shaped body is produced by a process according to any of claims 1 to 37 and is used to catalyze the process for heterogeneously catalyzed partial gas phase oxidation of at least one organic compound in the fixed catalyst bed.

39. The process according to claim 38, wherein the heterogeneously catalyzed partial gas phase oxidation is that

a) of propylene to acrolein and/or acrylic acid
or
b) of acrolein to acrylic acid,
or
c) of methacrolein to methacrylic acid,
or
d) of isobutene to methacrolein and/or methacrylic acid,
or
e) of propane to acrolein and/or acrylic acid,
or
f) of isobutane to methacrolein and/or methacrylic acid,
or
g) of at least one $C_4$ hydrocarbon and/or benzene to maleic anhydride,
or
h) of methanol to formaldehyde
or
i) the oxychlorination of ethylene to 1,2-dichloroethane.

**Revendications**

1. Procédé de fabrication d'un corps moulé géométrique oxydique, comprenant le compactage mécanique d'un agrégat en poudre introduit dans la chambre de remplissage d'une matrice, constitué par des constituants qui comprennent au moins un composé métallique qui peut être transformé par traitement thermique à une température ≥ 100 °C en un oxyde métallique ou au moins un oxyde métallique ou au moins un oxyde métallique et au moins un tel composé métallique, à condition qu'au moins un constituant de l'agrégat en poudre soit un sel de nitrate, un sel d'ammonium ou un nitrate d'ammonium, afin d'obtenir un précurseur de corps moulé géométrique, selon lequel la chambre de remplissage se trouve dans un alésage de matrice ayant un axe d'alésage vertical B, passant du haut vers le bas au travers du matériau de matrice, et est délimitée au moins par :

- la paroi intérieure de l'alésage de matrice,
- la surface frontale supérieure d'un piston inférieur introduit depuis le bas le long de l'axe d'alésage B dans l'alésage de matrice par un mouvement de soulèvement et d'abaissement, sur laquelle l'agrégat en poudre introduit dans la chambre de remplissage se trouve, et
- la surface frontale inférieure, qui se trouve le long de l'axe d'alésage B à un écart axial initial A au-dessus de la surface frontale supérieure du piston inférieur, d'un piston supérieur introduit par un mouvement de soulèvement et d'abaissement le long de l'axe d'alésage B, dont la surface frontale inférieure entre en contact par le haut avec l'agrégat en poudre introduit dans la chambre de remplissage,

EP 2 307 191 B1

selon lequel l'écart axial initial A entre les deux surfaces frontales est réduit à un écart axial final E le long de l'axe d'alésage B prescrit pour le compactage,

par abaissement du piston supérieur et maintien de la position du piston inférieur ou en outre soulèvement du piston inférieur, et soulèvement du piston supérieur du précurseur de corps moulé géométrique formé après la fin du compactage, et extraction du précurseur de corps moulé géométrique de l'alésage de matrice par soulèvement du piston inférieur,

ainsi qu'un procédé ultérieur de traitement thermique du précurseur de corps moulé géométrique à une température $\geq 100$ °C, selon lequel au moins une partie de ses constituants est décomposée et/ou transformée chimiquement avec formation d'au moins un composé gazeux, et le corps moulé géométrique oxydique est formé, **caractérisé en ce que** le matériau de matrice en contact avec l'alésage de matrice est un métal dur, qui est constitué par $\geq 80$ % en poids de la matière dure carbure de tungstène et moins de 5 % en poids du liant métallique nickel.

2. Procédé selon la revendication 1, **caractérisé en ce que** le métal dur contient en plus de la matière dure carbure de tungstène en outre au moins une autre matière dure du groupe constitué par les nitrures métalliques, les borures métalliques et les carbures métalliques différents du carbure de tungstène.

3. Procédé selon la revendication 2, **caractérisé en ce que** la proportion métallique de ladite au moins une matière dure supplémentaire est au moins un métal du groupe constitué par Ti, Zr, Hf, V, Nb, Ta, Mo, Cr et W.

4. Procédé selon l'une quelconque des revendications 1 à 3, **caractérisé en ce que** le métal dur contient en tant que liant métallique soit uniquement du nickel, soit en plus du nickel également au moins un liant métallique du groupe constitué par Fe, Co et Cr.

5. Procédé selon la revendication 4, **caractérisé en ce que** la proportion en poids de liant métallique Ni dans le métal dur est supérieure à la proportion en poids de chacun des autres liants métalliques contenus dans le métal dur.

6. Procédé selon l'une quelconque des revendications 1 à 5, **caractérisé en ce que** le métal dur contient en plus de la matière dure carbure de tungstène en outre au moins une autre matière dure du groupe constitué par TiC, TaC, NbC, VC, $Cr_3C_2$ et les carbures métalliques mixtes qui contiennent au moins deux des métaux contenus dans les carbures métalliques susmentionnés.

7. Procédé selon l'une quelconque des revendications 1 à 6, **caractérisé en ce que** le métal dur est constitué par $\geq 7$ % en poids du liant métallique nickel.

8. Procédé selon l'une quelconque des revendications 1 à 7, **caractérisé en ce que** le métal dur est constitué par $\geq 85$ % en poids de carbure de tungstène.

9. Procédé selon l'une quelconque des revendications 1 à 8, **caractérisé en ce que** le métal dur est constitué par $\geq 90$ % en poids de carbure de tungstène.

10. Procédé selon l'une quelconque des revendications 1 à 9, **caractérisé en ce que** le métal dur est constitué par 90 à 95 % en poids de carbure de tungstène, $\geq 0$ à 1 % en poids d'au moins un carbure métallique du groupe constitué par TiC, TaC, NbC, VC, $Cr_3C_2$ et les carbures métalliques mixtes qui contiennent au moins deux des métaux contenus dans les carbures métalliques susmentionnés, ainsi que jusqu'à 10 % en poids des liants métalliques Ni, Fe, Co et/ou Cr, à condition que la proportion en poids de Ni dans le métal dur soit $\geq 5$ % en poids.

11. Procédé selon la revendication 10, **caractérisé en ce que** le métal dur contient en tant que liant métallique uniquement du nickel ou uniquement du nickel et du chrome.

12. Procédé selon l'une quelconque des revendications 1 à 11, **caractérisé en ce que** la taille de particule des matières dures dans le métal dur se situe dans la plage allant de 0,5 $\mu$m à 2 $\mu$m.

13. Procédé selon l'une quelconque des revendications 1 à 12, **caractérisé en ce que** la matrice est exclusivement constituée par le métal dur.

14. Procédé selon l'une quelconque des revendications 1 à 12, **caractérisé en ce que** la matrice est constituée uniquement sur son côté en contact avec l'alésage de matrice par le métal dur et sur son côté opposé à l'alésage de matrice par un acier à outils ayant la composition élémentaire suivante :

1,50 à 1,80 % en poids de C,
0,10 à 0,40 % en poids de Si,
0,10 à 0,50 % en poids de Mn,
$\geq 0$ à 0,05 % en poids de P,
$\geq 0$ à 0,05 % en poids de S,
10 à 13 % en poids de Cr,
0,50 à 0,80 % en poids de Mo,
0,10 à 1,10 % en poids de V,
$\geq 0$ à 0,60 % en poids de W, et
$\geq 0$ à 0,10 % en poids d'un ou de plusieurs métaux de terres rares, le reste étant Fe et des impuretés liées à la fabrication.

**15.** Procédé selon l'une quelconque des revendications 1 à 14, **caractérisé en ce que** l'agrégat en poudre contient au moins un sel de nitrate du groupe constitué par le nitrate de cobalt, le nitrate de fer, le nitrate de bismuth, le nitrate de nickel, le nitrate de césium, le nitrate de cuivre, le nitrate de calcium, le nitrate de magnésium et les hydrates des nitrates susmentionnés.

**16.** Procédé selon l'une quelconque des revendications 1 à 15, **caractérisé en ce que** l'agrégat en poudre contient au moins un sel d'ammonium du groupe constitué par $NH_4HCO_3$, $NH_4OH$, $(NH_4)_2CO_3$, $NH_4HSO_4$, $(NH_4)_2SO_4$, $NH_4CHO_2$, le métavanadate d'ammonium, l'heptamolybdate d'ammonium, $NH_4CH_3CO_2$, l'oxalate d'ammonium et les hydrates des composés d'ammonium susmentionnés.

**17.** Procédé selon l'une quelconque des revendications 1 à 16, **caractérisé en ce que**, lors du traitement thermique du précurseur de corps moulé géométrique, au moins un composé gazeux du groupe constitué par l'ammoniac, la vapeur d'eau, $CO_2$, CO et les oxydes d'azote est formé.

**18.** Procédé selon l'une quelconque des revendications 1 à 17, **caractérisé en ce que** le traitement thermique du précurseur de corps moulé s'accompagne d'une perte de poids, qui est de 0,5 à 40 % en poids par rapport à son poids initial avant le traitement thermique.

**19.** Procédé selon l'une quelconque des revendications 1 à 18, **caractérisé en ce que** l'écart final E est de 2 à 10 mm.

**20.** Procédé selon l'une quelconque des revendications 1 à 18, **caractérisé en ce que** l'écart final E est de 2 à 8 mm.

**21.** Procédé selon l'une quelconque des revendications 1 à 20, **caractérisé en ce que** la valeur de rugosité moyenne $R_a$ de la paroi intérieure de l'alésage de matrice est $\leq 0,1$ $\mu$m.

**22.** Procédé selon l'une quelconque des revendications 1 à 21, **caractérisé en ce qu'**une pression de compression qui se situe dans la plage allant de 50 à 5 000 kg/cm$^2$ est exercée à l'écart final E par les deux pistons.

**23.** Procédé selon l'une quelconque des revendications 1 à 22, **caractérisé en ce qu'**une pression de compression qui se situe dans la plage allant de 500 à 2 500 kg/cm$^2$ est exercée à l'écart final E par les deux pistons.

**24.** Procédé selon l'une quelconque des revendications 1 à 23, **caractérisé en ce que** le procédé de traitement thermique du précurseur de corps moulé fabriqué a lieu à une température $\geq 200$ °C.

**25.** Procédé selon l'une quelconque des revendications 1 à 24, **caractérisé en ce que** le procédé de traitement thermique du précurseur de corps moulé fabriqué a lieu à une température $\geq 300$ °C.

**26.** Procédé selon l'une quelconque des revendications 1 à 24, **caractérisé en ce que** l'agrégat en poudre contient du graphite, de l'amidon, des coquilles de noix broyées, un granulat de plastique finement divisé, de la cellulose, de l'acide stéarique, de l'acide malonique, un sel de l'acide stéarique et/ou un sel de l'acide malonique ajoutés.

**27.** Procédé selon l'une quelconque des revendications 1 à 26, **caractérisé en ce que** la proportion en poids totale d'ions nitrate et d'ions ammonium contenue dans l'agrégat en poudre à compacter mécaniquement est $\geq 0,1$ % en poids.

**28.** Procédé selon l'une quelconque des revendications 1 à 26, **caractérisé en ce que** la proportion en poids totale d'ions nitrate et d'ions ammonium contenue dans l'agrégat en poudre à compacter mécaniquement est $\geq 0,2$ % en poids.

29. Procédé selon l'une quelconque des revendications 1 à 28, **caractérisé en ce que**, lors du procédé ultérieur de traitement thermique du précurseur de corps moulé géométrique fabriqué, un oxyde de plusieurs métaux est formé dans celui-ci, qui contient les éléments Mo et Fe, ou les éléments Mo, Fe et Bi, ou les éléments Mo et V, ou les éléments Mo, V et P, ou les éléments V et P.

30. Procédé selon l'une quelconque des revendications 1 à 29, **caractérisé en ce que**, lors du procédé ultérieur de traitement thermique du précurseur de corps moulé géométrique fabriqué, un oxyde de plusieurs métaux est formé dans celui-ci, dans lequel l'élément Mo ou l'élément V ou l'élément P est l'élément différent de l'oxygène qui est numériquement contenu le plus fréquemment, calculé en moles.

31. Procédé selon l'une quelconque des revendications 1 à 30, **caractérisé en ce que**, lors du procédé ultérieur de traitement thermique du précurseur de corps moulé géométrique fabriqué, un oxyde de plusieurs métaux de formule générale XII est formé dans celui-ci :

$$Mo_{12}Bi_aFe_bX^1_cX^2_dX^3_eX^4_fO_n \qquad (XII)$$

avec

$X^1$ = nickel et/ou cobalt,
$X^2$ = thallium, samarium, un métal alcalin et/ou un métal alcalino-terreux,
$X^3$ = zinc, phosphore, arsenic, bore, antimoine, étain, cérium, plomb, vanadium, chrome, niobium et/ou tungstène, $X^4$ = silicium, aluminium, titane et/ou zirconium,
a = 0, 2 à 5,
b = 0,01 à 5,
c = 0 à 10,
d = 0 à 2,
e = 0 à 8,
f = 0 à 10, et
n = un nombre déterminé par la valence et la fréquence des éléments différents de l'oxygène dans XII.

32. Procédé selon l'une quelconque des revendications 1 à 30, **caractérisé en ce que**, lors du procédé ultérieur de traitement thermique du précurseur de corps moulé géométrique fabriqué, un oxyde de plusieurs métaux de formule générale XIII est formé dans celui-ci :

$$[Y^1_{a'}Y^2_{b'}O_{x'}]_p[Y^3_{c'}Y^4_{d'}Y^5_{e'}Y^6_{f'}Y^7_{g'}Y^8_{h'}O_{y'}]_q \qquad (XIII)$$

avec

$Y^1$ = uniquement le bismuth ou le bismuth et au moins un des éléments tellure, antimoine, étain et cuivre,
$Y^2$ = molybdène ou tungstène, ou molybdène et tungstène,
$Y^3$ = un métal alcalin, thallium et/ou samarium,
$Y^4$ = un métal alcalino-terreux, nickel, cobalt, cuivre, manganèse, zinc, étain, cadmium et/ou mercure,
$Y^5$ = fer ou fer et au moins un des éléments vanadium, chrome et cérium,
$Y^6$ = phosphore, arsenic, bore et/ou antimoine,
$Y^7$ = un métal de terres rares, titane, zirconium, niobium, tantale, rhénium, ruthénium, rhodium, argent, or, aluminium, gallium, indium, silicium, germanium, plomb, thorium et/ou uranium,
$Y^8$ = molybdène ou tungstène, ou molybdène et tungstène,
a' = 0,01 à 8,
b' = 0,1 à 30,
c' = 0 à 4,
d' = 0 à 20,
e' > 0 à 20,
f' = 0 à 6,
g' = 0 à 15,
h' = 8 à 16,

x', y' = nombres déterminés par la valence et la fréquence des éléments différents de l'oxygène dans XIII, et
p, q = nombres dont le rapport p/q est de 0,1 à 10.

33. Procédé selon l'une quelconque des revendications 1 à 30, **caractérisé en ce que**, lors du procédé ultérieur de traitement thermique du précurseur de corps moulé géométrique fabriqué, un oxyde de plusieurs métaux de formule générale XIV est formé dans celui-ci :

$$[Bi_{a''}Z^2_{b''}O_{x''}]_{p''}[Z^8_{12}Z^3_{c''}Z^4_{d''}Fe_{e''}Z^5_{f''}Z^6_{g''}Z^7_{h''}O_{y''}]_{q''} \qquad (XIV)$$

avec

$Z^2$ = molybdène ou tungstène, ou molybdène et tungstène,
$Z^3$ = nickel et/ou cobalt,
$Z^4$ = thallium, un métal alcalin et/ou un métal alcalino-terreux,
$Z^5$ = phosphore, arsenic, bore, antimoine, étain, cérium, vanadium, chrome et/ou Bi,
$Z^6$ = silicium, aluminium, titane et/ou zirconium,
$Z^7$ = cuivre, argent et/ou or,
$Z^8$ = molybdène ou tungstène, ou tungstène et molybdène,
a" = 0,1 à 1,
b" = 0,2 à 2,
c" = 3 à 10,
d" = 0,02 à 2,
e" = 0,01 à 5,
f" = 0 à 5,
g" = 0 à 10,
h" = 0 à 1,
x", y" = nombre déterminés par la valence et la fréquence des éléments différents de l'oxygène dans XIV, et
p", q" = nombres dont le rapport p"/q" est de 0,1 à 5.

34. Procédé selon l'une quelconque des revendications 1 à 30, **caractérisé en ce que**, lors du procédé ultérieur de traitement thermique du précurseur de corps moulé géométrique fabriqué, un oxyde de plusieurs métaux de formule générale XV est formé dans celui-ci :

$$Mo_{12}P_aV_bX^1_cX^2_dX^3_ESb_fRe_gS_hO_n \qquad (XV)$$

avec

$X^1$ = potassium, rubidium et/ou césium,
$X^2$ = cuivre et/ou argent,
$X^3$ = cérium, bore, zirconium, manganèse et/ou bismuth,
a = 0,5 à 3,
b = 0,01 à 3,
c = 0,2 à 3,
d = 0,01 à 2,
e = 0 à 2,
f = 0 à 2,
g = 0 à 1,
h = 0 à 0,5 et
n = un nombre qui est déterminé par la valence et la fréquence des éléments différents de l'oxygène dans XV.

35. Procédé selon l'une quelconque des revendications 1 à 30, **caractérisé en ce que**, lors du procédé ultérieur de traitement thermique du précurseur de corps moulé géométrique fabriqué, un oxyde de plusieurs métaux de formule générale XVI est formé dans celui-ci :

$$V_1P_bFe_cX^1_dX^2_eO_n \qquad (XVI)$$

avec

$X^1$ = Mo, Bi, Co, Ni, Si, Zn, Hf, Zr, Ti, Cr, Mn, Cu, B, Sn et/ou Nb,
$X^2$ = Li, K, Na, Rb, Cs et/ou Tl,
b = 0,9 à 1,5,
c = 0 à 0,1,
d = 0 à 0,1,
e = 0 à 0,1 et
n = un nombre qui est déterminé par la valence et la fréquence des éléments différents de l'oxygène dans XVI.

36. Procédé selon l'une quelconque des revendications 1 à 28, **caractérisé en ce que**, lors du procédé ultérieur de traitement thermique du précurseur de corps moulé géométrique fabriqué, un oxyde solide en conditions normales est formé dans celui-ci, dans lequel ni un métal de transition des groupes de transition 5 à 11, ni le phosphore n'est l'élément différent de l'oxygène qui est numériquement contenu le plus fréquemment, calculé en moles.

37. Procédé selon l'une quelconque des revendications 1 à 28, **caractérisé en ce que** l'agrégat en poudre contient au moins un oxyde métallique du groupe constitué par l'oxyde d'aluminium, l'oxyde de tungstène, l'oxyde d'antimoine, l'oxyde de zirconium, l'oxyde de bismuth, l'oxyde de molybdène, l'oxyde de silicium, l'oxyde de magnésium et les oxydes mixtes qui contiennent au moins deux des éléments métalliques contenus dans les oxydes métalliques susmentionnés.

38. Procédé d'oxydation partielle en phase gazeuse sous catalyse hétérogène d'au moins un composé organique sur un lit catalytique fixe, **caractérisé en ce qu'**un corps moulé oxydique géométrique est fabriqué par un procédé selon l'une quelconque des revendications 1 à 37, et le procédé d'oxydation partielle en phase gazeuse sous catalyse hétérogène d'au moins un composé organique est catalysé avec celui-ci dans le lit catalytique fixe.

39. Procédé selon la revendication 38, **caractérisé en ce que** l'oxydation partielle en phase gazeuse sous catalyse hétérogène est celle :

a) de propylène en acroléine et/ou acide acrylique
ou
b) d'acroléine en acide acrylique
ou
c) de méthacroléine en acide méthacrylique
ou
d) d'isobutène en méthacroléine et/ou en acide méthacrylique
ou
e) de propane en acroléine et/ou en acide acrylique ou
f) d'isobutane en méthacroléine et/ou en acide méthacrylique
ou
g) d'au moins un hydrocarbure en $C_4$ et/ou de benzène en anhydride de l'acide maléique
ou
h) de méthanol en formaldéhyde
ou est
i) l'oxychloration d'éthylène en 1,2-dichloroéthane.

Fig. 1

Fig. 2a

Fig. 2b

Fig. 3a

Fig. 3b

Fig. 4a

14

4

34

13

35

Fig. 4b

Fig. 4c

Fig. 4d

Fig. 4e

Fig. 4f

Fig. 5a

Fig. 5b

Fig. 5c

Fig. 5d

Fig. 5e

Fig. 6

Fig. 7

Fig. 8

Figur 9

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 184790 A
- US 20050263926 A
- JP 10029097 A
- US 7147011 B
- US 102007028332 A
- DE 102005037678 A
- DE 102007003778
- GB 1350634 A
- BE 894920
- EP 1364732 A
- DE 19714430 A
- DE 102007028332
- DE 102007017080
- DE 2624853 A
- DE 19733969 A
- DE 2435777 A
- WO 2005115733 A
- DE 102007025869
- WO 2005030393 A
- EP 467144 A
- EP 1060792 A
- DE 19855913 A
- WO 0168245 A
- EP 2005497012 A
- DE 102005035978 A
- WO 0378059 A
- WO 03078310 A
- DE 19922113 A
- WO 0224620 A
- WO 02062737 A
- US 20050131253 A
- DE 102007003778 A
- DE 102007028332 A
- DE 10046957 A
- DE 4407020 A
- EP 575897 A
- DE 3338380 C
- DE 10325487 A
- WO 0053557 A
- WO 0053558 A
- DE 19910506 A
- EP 1106598 A
- WO 0136364 A
- DE 19927624 A

- DE 19948248 A
- DE 19948523 A
- DE 19948241 A
- EP 700714 A
- DE 10313213 A
- DE 10313209 A
- DE 10232748 A
- DE 10313208 A
- WO 03039744 A
- EP 279374 A
- DE 3338380 A
- DE 3300044 A
- DE 102004003212 A
- DE 102005013039 A
- DE 102005009891 A
- DE 102005010111 A
- DE 102005009885 A
- DE 19746210 A
- EP 015565 A
- EP 962253 A
- DE 10122027 A
- EP 608838 A
- DE 19835247 A
- EP 895809 A
- EP 1254709 A
- EP 1192987 A
- EP 1262235 A
- EP 1193240 A
- JP 11343261 A
- JP 11343262 A
- EP 1090684 A
- EP 1301457 A
- EP 1254707 A
- EP 1335793 A
- DE 10046672 A
- DE 10034825 A
- EP 1556337 A
- DE 10033121 A
- WO 0198246 A
- EP 1558569 A
- WO 9948606 A
- EP 2008050341 W
- US 61077601 B
- US 61077638 B

**In der Beschreibung aufgeführte Nicht-Patentliteratur**

- Tabellenbuch Metall. Verlag Europa Lehrmittel, 1999, D-42781

- Tabellenbuch Metall. Verlag Europa Lehrmittel, 1999, D-42781